(19) Europäisches Patentamt
European Patent Office
Office européen des brevets

(11) Veröffentlichungsnummer: **0 581 095 A2**

# (12) EUROPÄISCHE PATENTANMELDUNG

(21) Anmeldenummer: **93111103.3**

(22) Anmeldetag: **12.07.93**

(51) Int. Cl.5: **C07C 69/00**, C07D 263/32, C07C 69/736

(30) Priorität: **24.07.92 DE 4224457**

(43) Veröffentlichungstag der Anmeldung: **02.02.94 Patentblatt 94/05**

(84) Benannte Vertragsstaaten: **AT BE CH DE DK ES FR GB GR IE IT LI NL PT SE**

(71) Anmelder: **BASF Aktiengesellschaft**
**Carl-Bosch-Strasse 38**
**D-67063 Ludwigshafen(DE)**

(72) Erfinder: **Mueller, Bernd, Dr.**
**Jean-Ganss-Strasse 21**
**D-6710 Frankenthal(DE)**
Erfinder: **Roehl, Franz, Dr.**
**Sebastian-Kneipp-Strasse 17**
**D-6707 Schifferstadt(DE)**
Erfinder: **Koenig, Hartmann, Dr.**
**Albert-Einstein-Allee 16**
**D-6703 Limburgerhof(DE)**
Erfinder: **Sauter, Hubert, Dr.**
**Neckarpromenade 20**
**D-6800 Mannheim 1(DE)**
Erfinder: **Lorenz, Gisela, Dr.**
**Erlenweg 13**
**D-6730 Neustadt(DE)**
Erfinder: **Ammermann, Eberhard, Dr.**
**Von-Gagern-Strasse 2**
**D-6148 Heppenheim(DE)**

## (54) Substituierte Acrylsäureester und diese enthaltende Pflanzenschutzmittel.

(57) Substituierte Acrylsäureester der Formel I

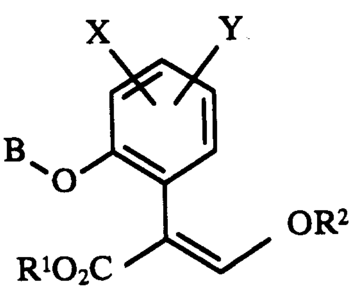

I

in der die Substituenten die folgende Bedeutung besitzen:
B bedeutet
Alkyl, das substituiert ist, Alkenyl, das substituiert ist, Alkinyl, das substituiert ist, Cycloalkyl, das substituiert ist, Cycloalkenyl, das substituiert ist, Cycloalkinyl, das substituiert ist oder Heterocyclyl, das substituiert ist.
X und Y bedeuten unabhängig voneinander
Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl,

EP 0 581 095 A2

Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy.

$R^1$ und $R^2$ Alkyl, Alkenyl, Alkinyl, Cycloalkyl oder Cycloalkenyl und die Säureadditionsprodukte und die Basenadditionsprodukte dieser Verbindungen und Fungizide, die diese Verbindungen enthalten.

Die vorliegende Erfingung betrifft substituierte Acrylsäureester und ihre Verwendung als Pflanzenschutzmittel, insbesondere zur Bekämpfung von Pilzen, Insekten, Nematoden und Spinnmilben.

Es ist bekannt Acrylsäureester, wie z. B. den α-(2-Hydroxyphenyl)-β-methoxy-acrylsäuremethylester (EP 251 082, EP 178 826), als Fungizide zu verwenden. Ihre fungizide Wirkung ist jedoch unbefriedigend.

Es wurde nun überraschend gefunden, daß substituierte Acrylsäureester der Formel I

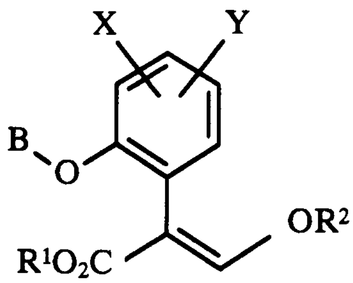

I

in der die Substituenten die folgende Bedeutung besitzen:
B bedeutet
Alkyl, das mit 1 - 4 gleichen oder verschiedenen Substituenten $R^3$ substituiert ist, Alkenyl, das mit 1 - 4 gleichen oder verschiedenen Substituenten $R^4$ substituiert ist, Alkinyl, das mit 1 - 4 gleichen oder verschiedenen Substituenten $R^5$ substituiert ist, Cycloalkyl, das mit 1 -4 gleichen oder verschiedenen Substituenten $R^6$ substituiert ist, Cycloalkenyl, das mit 1 - 4 gleichen oder verschiedenen Substituenten $R^7$ substituiert ist, Cycloalkinyl, das mit 1 - 4 gleichen oder verschiedenen Substituenten $R^8$ substituiert ist oder Heterocyclyl, das mit 1 - 4 gleichen oder verschiedenen Substituenten $R^9$ substituiert ist,
X und Y bedeuten unabhängig voneinander
Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyl oximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,
Stehen die Substituenten X und Y an benachbarten Kohlenstoffatomen, dann können sie zu einem ggf. substituierten aromatischen oder heteroaromatischen, alicyclischen oder heterocyclischen, partiell oder vollständig hydrierten Ring kondensiert sein,
$R^1$ und $R^2$ können ggf. substituiert sein, und bedeuten unabhängig voneinander Alkyl, Alkenyl, Alkinyl, Cycloalkyl oder Cycloalkenyl, $R^3$ und $R^4$ können ggf. substituiert sein mit 1 - 4 gleichen oder verschiedenen Substituenten $R^{10}$, und $R^3$ und $R^4$ bedeuten Nitro, Alkoxy, Haloalkoxy, Alkinyl, Cycloalkyl, Hetaryl, Heterocyclyl, Cycloalkenyl, Cycloalkinyl, Alkenyloxy, Alkinyloxy, Cycloalkyloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Cycloalkyloxycarbonyl,

Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Hetarylalkyloxy, Heterocyclylalkyloxy, Alkinylalkenyl, Cycloalkylalkenyl, Cycloalkenylalkenyl, Cycloalkinylalkenyl, Hetarylalkenyl, Heterocyclylalkenyl, Alkoximinoalkyl, Alkenyloximinoalkyl, Alkinyloximinoalkyl, Cycloalkyloximinoalkyl, Cycloalkenyloximinoalkyl, Cycloalkinyloximinoalkyl, Aryloximinoalkyl, Hetaryloximinoalkyl, Heterocyclyloximinoalkyl, Alkoximinoalkenyl, Alkenyloximinoalkenyl, Alkinyloximinoalkenyl, Cycloalkyloximinoalkenyl, Cycloalkenyloximinoalkenyl, Cycloalkinyloximinoalkenyl, Aryloximinoalkenyl, Hetaryloximinoalkenyl oder Heterocyclyloximinoalkenyl,

$R^5$, $R^6$, $R^7$ und $R^8$ können ggf. substituiert sein mit 1 - 4 gleichen oder verschiedenen Substituenten $R^{10}$, und $R^5$, $R^6$, $R^7$ und $R^8$ bedeuten Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino oder Heterocyclylcarbonylamino,

$R^9$ kann ggf. substituiert sein mit 1 - 4 gleichen oder verschiedenen Substituenten $R^{10}$ und $R^9$ bedeutet Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,

$R^{10}$ kann ggf. substituiert sein mit 1 - 4 gleichen oder verschiedenen Substituenten $R^{11}$ und $R^{10}$ bedeutet Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,

$R^{11}$ kann ggf. substituiert sein und bedeutet Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy und die Säureadditionsprodukte und die Basenadditionsprodukte dieser Verbindungen,

und ihre pflanzenverträglichen Säureadditionsprodukte und Basenadditionsprodukte neben einer hohen

fungitoxischen, insektiziden, nematiziden und akariziden Wirkung auch eine sehr gute Pflanzenvertraglichkeit besitzen.

Säuren für Säureadditionsprodukte sind z. B. Mineralsäuren, wie beispielsweise Chlorwasserstoffsäure, Bromwasserstoffsäure, Phosphorsäure, Schwefelsäure, Salpetersäure, oder aber Carbonsäuren, wie Ameisensäure, Essigsäure, Oxalsäure, Malonsäure, Milchsäure, Apfelsäure, Bernsteinsäure, Weinsäure, Zitronensäure, Salicylsäure, p-Toluolsulfonsäure, Dodecylbenzolsulfonsäure, aber auch allgemein Protonenacide Verbindungen, z. B. Saccharin.

Basen für Basenadditionsprodukte sind z. B. Kalium-, Natrium-, -hydroxid, -carbonat, Ammoniumhydroxid.

Die neuen Verbindungen der Formel I können bei der Herstellung als Gemische von Stereoisomeren (E/Z-Isomere, Diastereomere, Enantiomere) anfallen, die in üblicher Weise, z. B. durch Kristallisation oder Chromatographie, in die Einzelkomponenten getrennt werden können. Sowohl die einzelnen Isomeren als auch ihre Gemische können als Fungizide, Akarizide, Namatizide oder Insektizide verwendet werden und werden von der Erfindung erfaßt.

Die genannten Alkylreste besitzen bevorzugt 1 - 10 Kohlenstoffatome, sind ggf. substituiert und bedeuten beispielsweise
Methyl, Ethyl, Propyl, n-Propyl, i-Propyl, Butyl, n-Butyl, i-Butyl, t-Butyl, s-Butyl, Pentyl, Pentyl-1, Pentyl-2, Pentyl-3, 2-Methylbutyl-1, 2-Methylbutyl-2, 2-Methylbutyl-3, 3-Methylbutyl-1, 2,2-Dimethylpropyl-1, Hexyl, Hexyl-1, Hexyl-2, Hexyl-3, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Di-methylbutyl, 1-Ethylbutyl, 2-Ethylbutyl, 1,1,2-Trimethyl-propyl, 1,2,2-Trimethylpropyl, 1-Ethyl-1-methylpropyl, 1-Ethyl-2-methylpropyl, Heptyl, Heptyl-1, Heptyl-2, Heptyl-3, Heptyl-4, 1-Methylhexyl, 2-Methylhexyl, 3-Methylhexyl, 4-Methylhexyl, 5-Methylhexyl, 1-Ethylpentyl, 2-Ethylpentyl, 3-Ethylpentyl, 1-Propylbutyl, 1-Isopropylbutyl, Octyl, Octyl-1, Octyl-2, Octyl-3, Octyl-4, 1-Methylheptyl, 2-Methylheptyl, 3-Methylheptyl, 4-Methylheptyl, 5-Methylheptyl, 6-Methylheptyl, 1-Ethylhexyl, 2-Ethylhexyl, 3-Ethylhexyl, 4-Ethylhexyl, 1-Propylpentyl, 2-Propylpentyl, Nonyl, 1-Nonyl, 2-Nonyl, 3-Nonyl, 4-Nonyl, 5-Nonyl, 1-Methyloctyl, 2-Methyloctyl, 3-Methyloctyl, 4-Methyloctyl, 5-Methyloctyl, 6-Methyloctyl, 7-Methyloctyl, 4-Methyl-2-propylpentyl, Decyl, 1-Decyl, 2-Decyl, 3-Decyl, 4-Decyl, 5-Decyl, 1-Ethyloctyl, 2-Ethyloctyl, 3-Ethyloctyl, 4-Ethyloctyl, 5-Ethyloctyl, 6-Ethyloctyl oder 2-Propylheptyl.

Die genannten Alkenylreste besitzen bevorzugt 2 - 10 Kohlenstoffatome, sind ggf. substituiert und bedeuten beispielsweise
Ethenyl,Propenyl, 1-Propenyl, 2-Propenyl, Butenyl, 1-Butenyl, 2-Butenyl, 3-Butenyl, 1-Methyl-2-propenyl, 2-Methyl-2-propenyl, Pentenyl, 1-Pentenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, Hexenyl, 1-Hexenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl, Heptenyl, Octenyl, Nonenyl oder Decenyl.

Die genannten Alkinylreste besitzen bevorzugt 2 - 10 Kohlenstoffatome, sind ggf. substituiert und bedeuten beispielsweise
Ethinyl, Propinyl, 1-Propinyl, 2-propinyl, Butinyl, 1-Butinyl, 2-Butinyl, 3-Butinyl, 1-Methyl-2-propinyl, Pentinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-methyl-2-pentinyl, Hexinyl, 1-Methyl-3-pentinyl, 1-Methyl-4-pentinyl, 1-Methyl-3-pentinyl, 2-Methyl-4-pentinyl, 3-Methyl-4-pentinyl, 4-Methyl-2-pentinyl, 1,1-dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, Heptinyl, Octinyl, Noninyl oder Decinyl.

Die genannten Halogene bedeuten Fluor, Chlor, Brom oder Jod.

Die genannten Cycloalkylreste besitzen bevorzugt 3 - 10 Kohlenstoffatome, sind ggf. substituiert und bedeuten beispielsweise
Cyclopropyl, Cyclobutyl, Cyclopentyl, Cyclohexyl, Cycloheptyl, Cyclooctyl, Cyclononyl, Cyclodecyl, Bornanyl, Norbonanyl, Dicyclohexyl, Bicyclo[3,3,0]octyl, Bicyclo[3,2,1]octyl, Bicyclo[2,2,2]octyl oder Bicyclo-[3,3,1]nonyl.

Die genannten Cycloalkenylreste besitzen bevorzugt 3 - 10 Kohlenstoffatome, sind ggf. substituiert und bedeuten beispielsweise Cyclopropenyl, Cyclobutenyl, Cyclopentenyl, Cyclohexenyl, Cycloheptenyl, Cyclooctenyl, Cyclononenyl, Cyclodecenyl, Bornenyl, Norbonenyl, Bicyclo [3,3,0]octenyl, Bicyclo[3,2,1]octenyl, Bicyclo[2,2,2]octenyl oder Bicyclo[3,3,1]nonenyl. Die genannten Cycloalkinylreste besitzen bevorzugt 6 - 10 Kohlenstoffatome, sind ggf. substituiert und bedeuten beispielsweise Cyclohexin, Cycloheptin, Cyclooctin, Cyclononin, Cyclodecin, Cycloundecin oder Cyclodedecin.

Die genannten Haloalkylreste besitzen bevorzugt 1 - 4 Kohlenstoffatome, sind ggf. substituiert und bedeuten beispielsweise
Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-di-fluorethyl, 2,2-Dichlor-2-fluorethyl, 2,2,2-Trichlorethyl oder Pentafluorethyl.

Die genannten Arylreste besitzen bevorzugt 6, 10 oder 14 Kohlenstoffatome, sind ggf. substituiert und bedeuten beispielsweise Phenyl, Naphthyl, 1-Naphthyl, 2-Napthyl, Anthracenyl, 1-Anthracenyl, 2-Anthracenyl oder 9-Anthracenyl.

Die genannten Hetarylreste besitzen bevorzugt 5 - 14 Ringatome, davon 1 - 4 Heteroatome aus der Gruppe N, O, S, sind ggf. substituiert und bedeuten beispielsweise
Furyl, 2-Furyl, 3-Furyl, Thienyl, 2-Thienyl, 3-Thienyl, Pyrrolyl, 1-Pyrrolyl, 2-Pyrrolyl, 3-Pyrrolyl, Isoxazolyl, 3-Isoxazolyl, 4-Isoxazolyl, 5-Isoxazolyl, Isothiazolyl, 3-Isothiazolyl, 4-Isothiazolyl, 5-Isothiazolyl, Pyrazolyl, 1-Pyrazolyl, 3-Pyrazolyl, 4-Pyrazolyl, 5-Pyrazolyl, Oxazolyl, 2-Oxazolyl, 4-Oxazolyl, 5-Oxazolyl, Thiazolyl, 2-Thiazolyl, 4-Thiazolyl, 5-Thiazolyl, Imidazolyl, 1-Imidazolyl, 2-Imidazolyl, 4-Imidazolyl, 5-Imidazolyl, 1,2,3-Thiadiazolyl, 1,2,4-Thiadiazolyl, 1,2,5-Thiazolyl, 1,3,4-Thiadiazolyl, Tetrazolyl, 1,2,3,4-Thiatriazolyl, 1,2,3,4-Oxatriazolyl, Pyridyl, 2-Pyridyl, 3-Pyridyl, 4-Pyridyl, Pyridazinyl, 3-Pyridazinyl, 4-Pyridazinyl, Pyrimidinyl, 2-Pyrimidinyl, 4-Pyrimidinyl, 5-Pyrimidinyl, Pyrazinyl, 2-Pyrazinyl, 3-Pyra-zinyl, 1,2,4-Triazinyl, 1,3,5-Triazinyl oder 1,2,4,5-Tetrazinyl,

Dabei können benachbarte Substituenten des Heteroaromaten kondensiert sein zu einem aromatischen oder heteroaromatischen Ring, so daß Hetaryl auch kondensierte Ringsysteme umfaßt wie z. B. Benzofuranyl, Isobenzofuranyl, 1-Benzothienyl, 2-Benzothienyl, Indolyl, Isoindolyl, Benzisoxazolyl, Benzoxazol, Benzisothiazolyl, Benzthiazolyl, 2-Benzthiazolyl, 4-Benzthiazolyl, 5-Benzthiazolyl, 6-Benzthiazolyl, 7-Benzthiazolyl, Indazolyl, Benzimidazolyl, Benzthiazolyl, Benzofurazanyl, Dibenzofuranyl, Dibenzothienyl, Acridinyl, Phenanthridinyl, Carbazolyl, Chinolinyl, Isochinolinyl, Phthalazinyl, Chinazolinyl, Chinoxalinyl, Cinnolinyl, 1,5-Napthyridinyl, 1,6-Naphthyridinyl, 1,7-Naphthyridinyl, 1,8-Naphthyridinyl, Pteridinyl, Pyrrolopyridinyl, Pyrrolopyridazinyl, Pyrrolopyrimidinyl, Pyrrolopyrazinyl, Pyrrolotriazinyl, Furopyridinyl, Furopyridazinyl, Furopyrimidinyl, Furopyrazinyl, Furotriazinyl, Thienopyridinyl, Thienopyridazinyl, Thienopyrmidinyl, Thienopyrazinyl, Thienotriazinyl, Imidazopyridinyl, Imidazopyridazinyl, Imidazopyrimidinyl, Imidazopyrazinyl, Pyrazolopyridinyl, Pyrazolopyridazinyl, Pyrazolopyrimidinyl, Pyrazolopyrazinyl, Isoxazolopyridinyl, Isoxazolopyridazinyl, Isoxazolopyrimidinyl, Isoxazolopyrazinyl, Oxazolopyridinyl, Oxazolopyridazinyl, Oxazolopyrimidinyl, Oxazolopyrazinyl, Thiazolopyridinyl, Thiazolopyridazinyl, Thiazolopyrimidinyl, Thiazolopyrazinyl, Isothiazolopyridinyl, Isothiazolopyridazinyl, Isothiazolopyrimidinyl, Isothiazolopyrazinyl, Triazolopyridinyl, Triazolopyridazinyl, Triazolopyrimidinyl oder Triazolopyrazinyl.

Die genannten Heterocyclylreste besitzen bevorzugt 3 - 15 Ringatome, davon 1 - 4 Heteroatome aus der Gruppe N, O, S, sind gesättigt oder parallel ungesättigt und ggf. substituiert und bedeuten beispielsweise
2-Tetrahydrofuranyl, Oxiranyl, 3-Tetrahydrofuranyl, 2-Tetrahydrothienyl, 3-Tetrahydrothinyl, 2-Pyrrolidinyl, 3-Pyrrolidinyl, 3-Isoxazolidinyl, 4-Isoxazolidinyl, 4-isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,5-Dihydrofur-2-yl, 2,5-Dihydrofur-3-yl, 2,3-Dihydrofur-3-yl, 2,3-Dihydrothien-2-yl, 2,3-Dihydrothien-3-yl, 2,5-Dihydrothien-2-yl, 2,5-Di-hydrothien-2-yl, 2,4-Pyrrolin-2-yl, 2,3-Pyrrolin-3-yl, 2,5-Pyrrolin-2-yl, 2,5-Pyrrolin-3-yl, 2,3-Isoxazolin-3-yl, 3,4-Isoxazolin-3-yl, 4,5-Isoxazolin-2-yl, 2,3-Isoxazolin-4-yl, 3,4-Isoxazolin-4-yl, 4,5-Isoxazolin-5-yl, 2,3-isothia-zolin-3-yl, 3,4-Isothiazolin-3-yl, 4,5-Isothiazolin-3-yl, 2,3-Isothiazolin-4-yl, 3,4-Isothiazolin-4-yl, 4,5-isothiazolin-4-yl, 2,3-Isothiazolin-5-yl, 3,4-Isothiazolin 5-yl, 4,5-Isothiazolin-5-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-2-yl, 3,4-Dihydropyrazol-4-yl, 3,4-dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-piperidinyl, 4-Piperidinyl, 3-Tetrahydropyridazinyl, 4-Tetrahydropyridazinyl, 2-Tetrahydropyrimidinyl, 4-Tetrahydropyrimidnyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl,

Oxazol-2-in-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothin-2-yl, 2H-1,4-Benzothiazin-3-yl, 2H-1,4-Benzoxazin-3-yl, 1,3-Dihydrooxazin-2-yl, 1,3-Dithian-2-yl, N-Morpholinyl oder Dihydrochinazolinyl.

Ausdrücklich eingeschlossen sind solche heterocyclischen Reste, die noch weitere funktionelle Gruppen wie beispielsweise Oxo- oder Thioxogruppen tragen, so daß die Heterocyclylreste auch cyclische Ester, Thioester oder Amide bzw. deren Thio- Analoge umfassen wie beispielsweise Pyrrolidonyl, Piperidonyl, 1-Azacycloheptan-2-onyl, Imidazolidinonyl, Diketopiperazinyl oder Thiopyrrolidonyl.

Die neuen Verbindungen können beispielsweise nach folgenden Verfahren hergestellt werden:

So kann z. B. das Phenol 1 (EP 251 082) unter alkalischen Bedingungen (z. B. Alkalicarbonate in dipolar aprotischen Lösungsmitteln wie Dimethylformamid, N-Methylpyrrolidon, Tetramethylharnstoff, Dimethylpropylenharnstoff, Dimethylsulfoxid, Sulfolan usw.) mit den entsprechenden Alkylierungsmitteln zu den erfindungsgemäßen Verbindungen 2 umgesetzt werden (Schema 1)

Schema 1

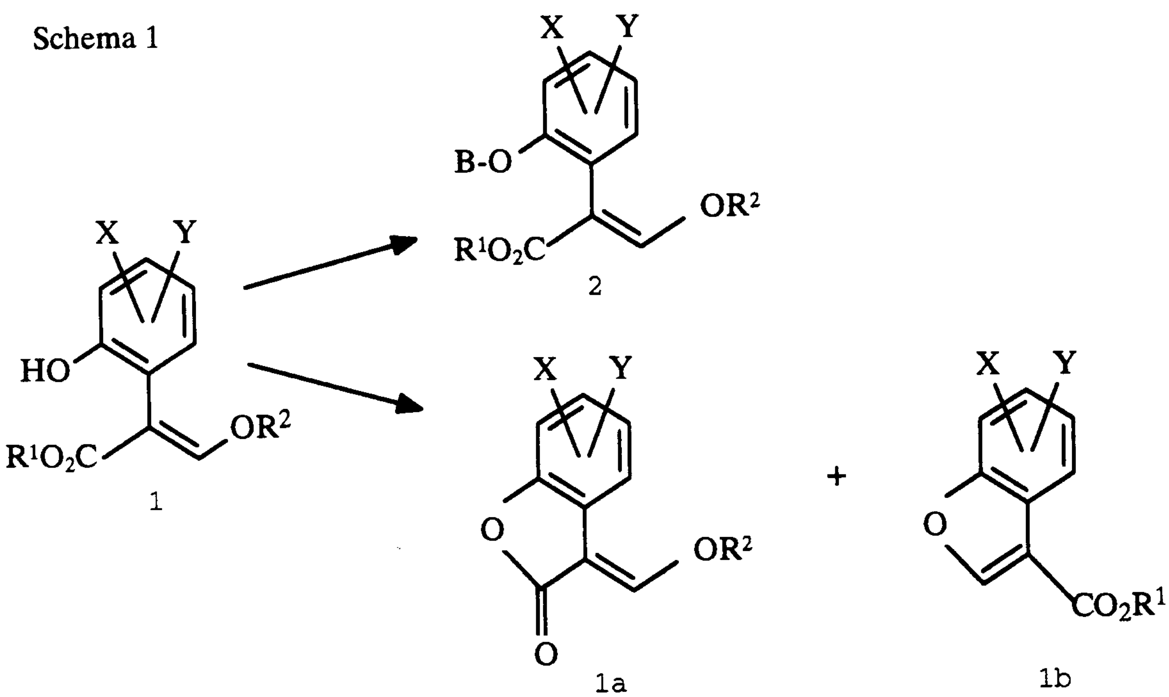

1, 2, 1a, 1b

Die Hetero-Arylierung des Phenols 1 ist zwar bekannt (EP 242 081), die Alkylierung von 1 insbesondere mit reaktionstragen Alkylierungsmitteln zu den Ethern 2 ist jedoch problematisch, da hierbei die Cyclisierung von 1 zu 1a bzw. 1b zum Teil als einzig feststellbare Reaktion auftritt (Schema 1). Diese Nebenproduktbildung kann zurückgedrängt werden, wenn man zur Erhöhung der Reaktionsgeschwindigkeit der bimolekularen Reaktion zwischen dem Phenol 1 und dem Alkylierungsmittel Hal-B (Hal = Cl, Br, J) zu den Wirkstoffen 2 im relativ hochkonzentrierten Lösungen und zur Aktivierung reaktionsträger Alkylierungsmittel (Hal = Cl, Br) in Gegenwart katalytischer Mengen von Iodsalzen (z.B. NaJ, KJ) in z.B. den oben genannten dipolar-aprotischen Lösungsmitteln arbeitet.

Analog erhält man die Wirkstoffe 2 durch Umsetzung des Phenols 1 mit Alkoholen unter den Bedingungen der Mitsunobu-Reaktion (Synthesis 1981, 1).

Außerdem können die Phenylessigester 3 unter alkalischen Bedingungen zu den formylierten Produkten 4 umgesetzt werden, die zu den erfindungsgemäßen Verbindungen 3 alkyliert werden können (Schema 2).

Schema 2

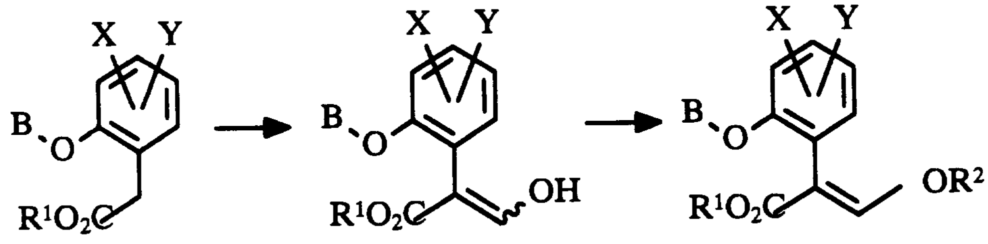

3 4 2

Außerdem können die Ketoester 5 in einer Wittig-Reaktion mit $(C_6H_5)_3P^+$-$CH_2$-O-$R^2$-Cl zu den erfindungsgemäßen Verbindungen 2 umgesetzt werden (Schema 3).

Schema 3

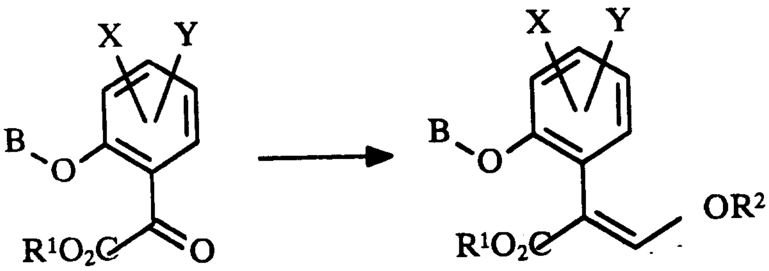

5 2

In der chemischen Literatur sind zahlreiche Methoden zur Synthese der α-Ketoester 5 beschrieben (siehe z. B.: L. Weinstock et al., Synthetic Communications 11, 943 - 946 (1981); J. March, Advanced Organic Chemistry, 3. Auflage (1985), J. Wiley & Sons; R. Larock, Comprehensive Organic Transformations, 1. Auflage 1989, VCH Publishers; M. Fieser, Reagents for Organic Synthesis, J. Wiley & Sons), beispielhaft erwähnt sei hier nur die Synthese der Ketoester 5 ausgehend von Benzaldehyden 6 über einen Cyanhydrinweg (EP 422 597), ausgehend von ortho-Halogenaromaten 7 (Hal = Cl, Br, J) über einen Grignard-Weg (EP 253 213), ausgehend von Benzoesäureestern 8 über eine Pummerer-Umlagerung (JACS 1966, 5498; Synthesis 1982, 41), ausgehend von Acetophenonen 9 über eine Oxidation / Veresterung (Synthetic Communications 21, 2045 (1991); J. Prakt. Chem 45, 377 (1892)) oder ausgehend von Benzoylcyaniden 10 (Tetrahedron Letters 1980, 3539; Schema 4).

Schema 4

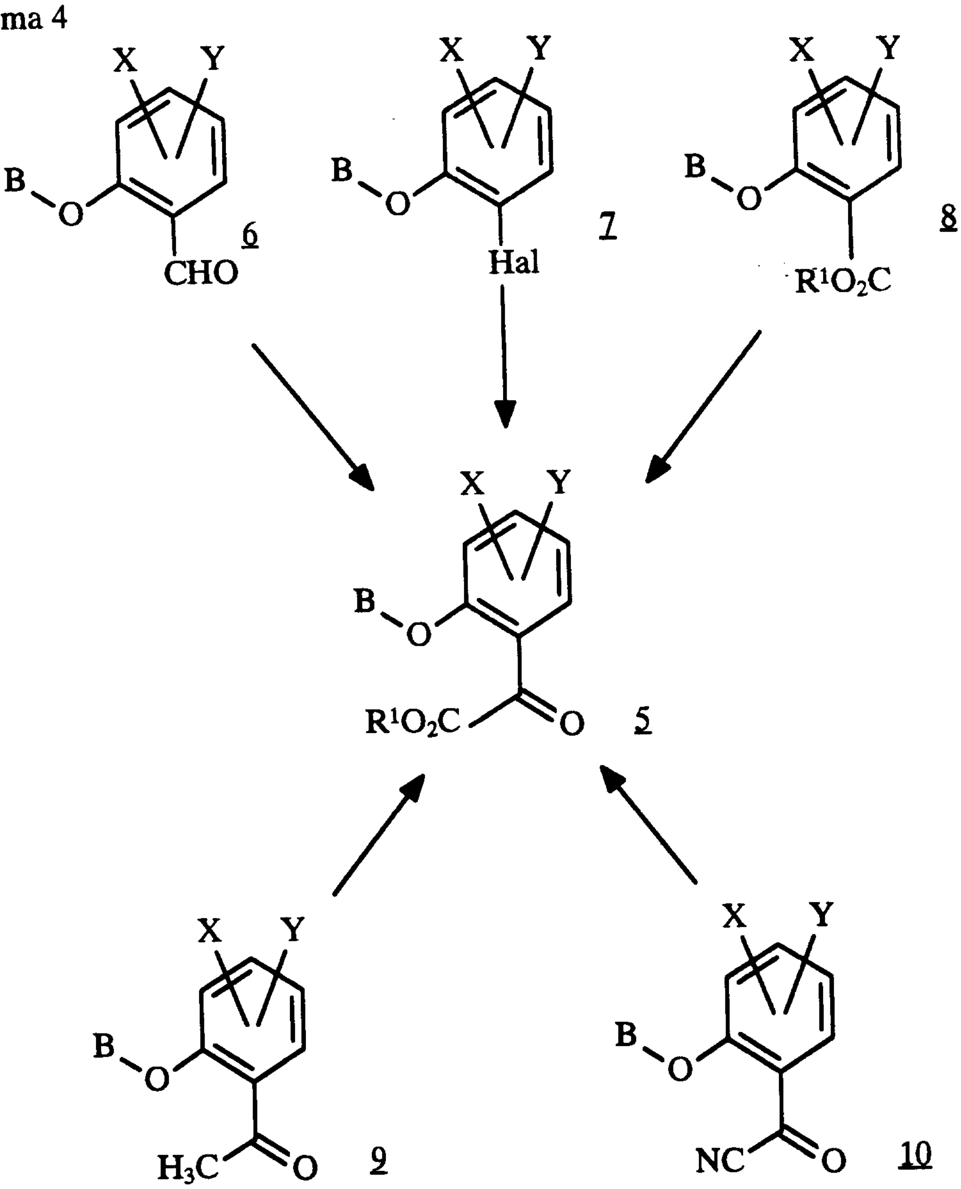

6 7 8

5

9 10

Außerdem sind die erfindungsgemäßen Verbindungen 2 aus den Phenylessigestern 3 über eine Enaminbildung zu 11, Hydrolyse des Enamins zum Formylderivat 4 (siehe Schema 2) und Alkylierung des Formylderivats (analog DE 40 25 892) zugänglich (Schema 5).

Schema 5

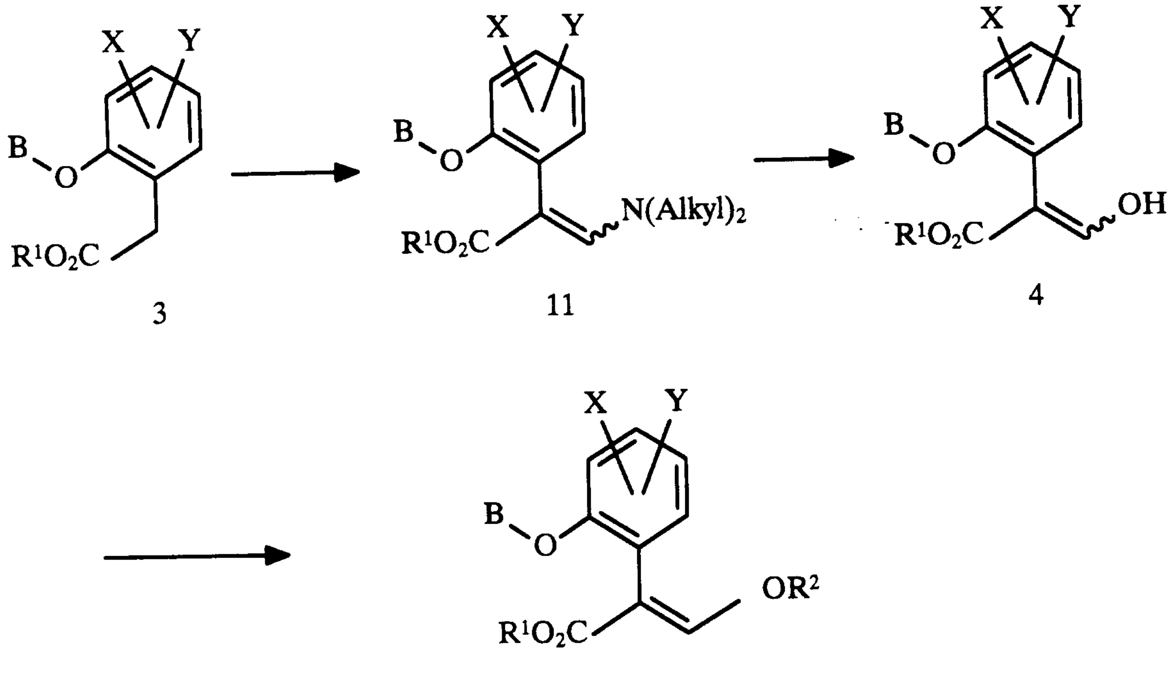

3 11 4

2

Außerdem können die Enolether 2 durch Eliminierung eines Alkohols $R^2$-OH aus den Acetalen 12 (J. Chem. Soc. Chem. Commun. 1980, 838; Chem. Lett. *1976*, 796) sowie aus den Acrylestern 13 durch aufeinanderfolgende Reaktion mit Brom, einem Alkoholat $R^2$-O-M (M = Li, Na, K, Mg) und einer Protonensaure wie z. B. $NaHSO_4$ (J. Chem. Soc. 1958, 153) hergestellt werden (analog EP 431 328; Schema 6).

Schema 6

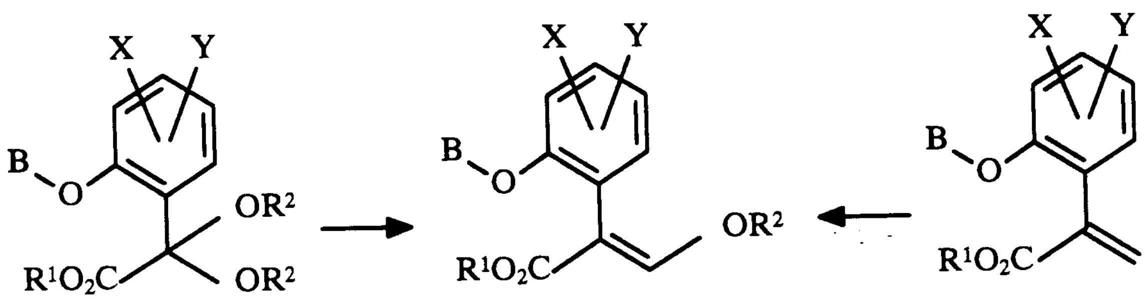

12 2 13

Außerdem ist der Propargylether 14 ein wertvolles Zwischenprodukt zur Synthese der erfindungsgemäßen Verbindungen 15 und 16 . So liefert die Umsetzung von 14 mit Aldehydoximen $R^{10}$-CH=NOH in Gegenwart von NaOCl-Lösung die Isoxazole 15 bzw. die Umsetzung von 14 mit Aryl- oder Hetaryl-halogeniden (Hal = J, Br) in Gegenwart eines Pd-Katalysators die Acetylene 16 (Schema 7).

Schema 7

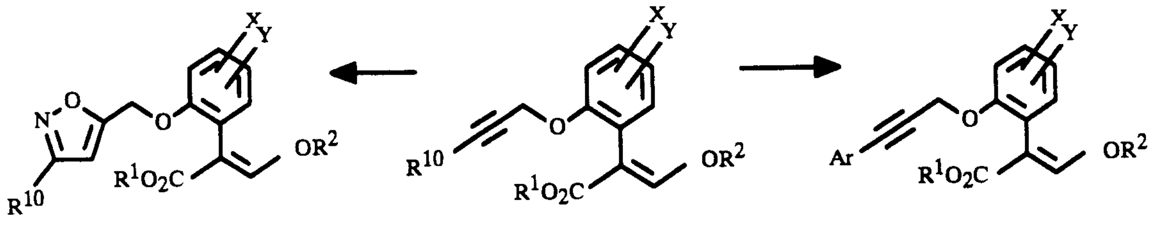

15 14 16

Die folgenden Beispiele erläutern die Herstellung der neuen Verbindungen:

Beispiel 1

α-(2-(Benzoylmethyloxy)-phenyl)-β-methoxy-acrylsäuremethylester (Tabelle 3, Nr. 18)

Eine Mischung von 2,1 g (10 mmol) α-(2-Hydroxyphenyl)-β-methoxyacrylsäuremethylester (EP 251 082), 2,0 g (10 mmol) Phenacylbromid und 1,5 g (11 mmol) $K_2CO_3$ in 5 ml Dimethylformamid wird über Nacht bei Raumtemperatur gerührt. Anschließend gibt man zusätzlich 0,6 g Phenacylbromid und 0,5 g $K_2CO_3$ hinzu.

Man rührt 6 Stunden bei Raumtemperatur (20 °C), verdünnt die Reaktionsmischung mit Wasser und extrahiert die wässrige Phase dreimal mit Methyl-t-butylether. Die organische Phase wird über $MgSO_4$ getrocknet und eingeengt. Der Rückstand wird säulenchromatographisch mit Hexan/Essigester-Gemischen gereinigt. Man erhält 1,7 g (52 %) der Titelverbindung als farblosen Festkörper (Fp = 76 °C).

$^1H$-NMR ($CDCl_3$; δ in ppm):

7,95 (d, breit, 2 H, Aromat); 7,5 (m, 4 H, 3 x Aromat, 1 x Vinyl); 7,25 (m, 2 H, Aromat); 7,0 (t, 1 H, J = 8 Hz, Aromat); 6,9 (d, 1 H, J = 8 Hz, Aromat); 5,2 (s, 2 H, O-$CH_2$); 3,8 (s, 3 H, O-$CH_3$); 3,65 (s, 3 H, O-$CH_3$)

Beispiel 2

α-(2-((N-Phenylpyrrolidon-3-yl)-oxy)-phenyl)-β-methoxy-acrylsäuremethylester (Tabelle 3, Nr. 16)

a) 2-((N-Phenylpyrrolidon-3-yl)-oxy)-benzaldehyd

14,8 g (0,12 mol) Salicylaldehyd in 100 ml Dimethylformamid wird unter leichter Kühlung mit 3,2 g (0,13 mol) Natriumhydrid versetzt. Nach Abklingen der Gasentwicklung gibt man 29 g (0,12 mol) 3-Brom-N-phenylpyrrolidon (hergestellt analog J. Med. Chem. 30, 1995 (1987)) hinzu und rührt über Nacht bei Raumtemperatur. Anschließend verdünnt man die Reaktionsmischung mit Wasser und extrahiert die wässrige Phase dreimal mit Methyl-t-butylether. Die vereinigten organischen Phasen werden über $MgSO_4$ getrocknet und eingeengt. Der Rückstand wird säulenchromatographisch gereinigt. Man erhält 12,8 g (39 %) der Titelverbindung als hellgelben Festkörper.

$^1H$-NMR ($CDCl_3$; δ in ppm):

10,5 (s, 1 H, CHO); 7,0 - 8,0 (m, 9 H, Aromat); 5,15 (t, 1 H, J = 8 Hz, O-CH); 3,9 (m, 2 H, N-$CH_2$); 2,7 (m, 1 H, $\underline{C}H_AH_B$); 2,4 (m, 1 H, $CH_A\underline{H}_B$)

b) 2-((N-Phenylpyrrolidon-3-yl)-oxy)-phenylglyoxylsäuremethylester

Eine Mischung von 12,8 g (45 mmol) des Aldehyds aus Beispiel 2 a in 100 ml Methylenchlorid und 5 g (100 mmol) Natriumcyanid und 5 g (94 mmol) Ammoniumchlorid in 100 ml Wasser wird 3 Stunden bei Raumtemperatur gerührt. Dann wird die organische Phase abgetrennt und die wässrige Phase noch zweimal mit Methylenchlorid extrahiert. Die vereinigten organischen Phasen werden über $MgSO_4$ getrocknet und eingeengt. Der Rückstand wird in 100 ml Methanol aufgenommen und mit 20 ml 3 n Salzsäure in Ether versetzt. Man rührt über Nacht bei Raumtemperatur und dampft anschließend die Reaktionsmischung i. Vak. ein.

Der Rückstand wird in 100 ml Wasser aufgenommen und 30 Minuten auf 100 °C erhitzt, wobei sich ein viskoses Öl abscheidet. Das Wasser wird abdekantiert und der Rückstand wird in Methylenchlorid aufgenommen. Die organisch Phase wird über $MgSO_4$ getrocknet und eingeengt.

Der Rückstand wird in 100 ml Methylenchlorid aufgenommen und mit einer Lösung von 0,3 g KBr, 0,7 g $NaH_2PO_4 \cdot H_2O$ und 0,9 g $Na_2HPO_4 \cdot 2\ H_2O$ in 100 ml Wasser versetzt. Dann gibt man 0,2 Tetramethylpiperidin-N-oxyl und anschließend unter starkem Rühren 40 ml Chlorbleichlauge (enthält 12,5 % aktives $Cl_2$) hinzu. Man rührt 1 Stunde bei Raumtemperatur, trennt die wässrige Phase ab, gibt wiederum 40 ml Chlorbleichlauge hinzu und rührt nochmals 1 Stunde. Dann wird die organische Phase abgetrennt, einmal mit Wasser extrahiert, über $MgSO_4$ getrocknet und eingeengt. Der Rückstand wird säulenchromatographisch gereinigt. Man erhält 6,4 g (42 %) der Titelverbindung als beigen Festkörper (Fp = 110 °C).
$^1$H-NMR ($CDCl_3$; $\delta$ in ppm):
7,9 (d, breit, 1 H, Aromat); 7,7 (d, 2 H, J = 8 Hz, Aromat); 7,6 (t, breit, 1 H, Aromat); 7,4 (t, 2 H, J = 8 Hz, Aromat); 7,3 (d, 1 H, J = 8 Hz, Aromat); 7 - 7,25 (m, 2 H, Aromat); 5,15 (t, 1 H, J = 8 Hz, O-CH); 4,0 (m, 1 H, N- $C\underline{H}_AH_B$); 3,85 (m, 1 H, N-$CH_A\underline{H}_B$); 3,85 (s, 3 H, -O-$CH_3$); 2,65 (m, 1 H, -$C\underline{H}_AH_B$); 2,3 (m, 1 H, -$CH_A\underline{H}_B$)

c) $\alpha$-(2-((N-Phenylpyrrolidon-3-yl)-oxy)-phenyl)-$\beta$-methoxy-acrylsäuremethylester (Tabelle 3, Nr. 16)

4,0 g (11 mmol) Methoxymethyl-triphenylphosphoniumchorid und 1 g (9 mmol) Kalium-t-butanolat in 50 ml Tetrahydrofuran werden 30 Minuten bei Raumtemperatur gerührt. Dann kühlt man auf - 20 °C, gibt 2 g (5,9 mmol) des Ketoesters aus Beispiel 2 b hinzu und läßt langsam auf Raumtemperatur erwärmen. Man rührt 2 Stunden bei Raumtemperatur, verdünnt die Reaktionsmischung mit Wasser und extrahiert die wässrige Phase dreimal mit Methyl-t-butylether. Die vereinigten organischen Phasen werden über $MgSO_4$ getrocknet und eingeengt. Der Rückstand wird säulenchromatographisch mit Hexan/Essigester-Gemischen gereinigt. Man erhält 1,4 g (65 %) des trans-Isomeren der Titelverbindung als hellgelbes Öl.
$^1$H-NMR ($CDCl_3$; $\delta$ in ppm):
7,7 (d, breit, 1 H, Aromat); 7,5 (s, 1 H, Vinyl); 6,9 - 7,45 (m, 8 H, Aromat); 4,95 (t, 1 H, J = 8 Hz, O-CH); 3,7 - 4,0 (m, 2 H, N-$CH_2$); 3,8 (s, 3 H, O-$CH_3$); 3,7 (s, 3 H, O-$CH_3$); 2,45 (m, 1 H, -$C\underline{H}_AH_B$); 2,25 (m, 1 H, -$CH_A\underline{H}_B$)

Beispiel 3

$\alpha$-(2-(3-(meta-Methylphenyl)-prop-2-inyloxy)-phenyl)-$\beta$-methoxy-acrylsäure-methylester (Tabelle 3, Nr. 6)

a) 2-(Prop-2-inyloxy)-phenylessigsäuremthylester

75 g (0,45 mol) ortho-Hydroxyphenylessigsäuremethylester in 500 ml Dimethylformamid werden portionsweise mit 13 g (0,54 mol) Natriumhydrid versetzt. Dabei wird leicht gekühlt. Nach 30 Minuten bei Raumtemperatur ist die Gasentwicklung abgeklungen und man gibt 65 g (0,54 mol) Propargylbromid hinzu. Man rührt über Nacht bei Raumtemperatur, verdünnt anschließend die Reaktionsmischung mit 1 l Wasser und extrahiert die wässrige Phase dreimal mit Methyl-t-butylether. Die vereinigten organischen Phasen werden über $MgSO_4$ getrocknet und eingeengt. Der Rückstand wird destilliert. Man erhält 71,6 g (78 %) der Titelverbindung als hellgelbe Flüssigkeit (Kp (0,3 mbar) = 98 - 100 °C).
$^1$H-NMR ($CDCl_3$; $\delta$ in ppm):
7,25 (m, 2 H, Aromat); 7,0 (m, 2 H, Aromat); 4,7 (d, 1 H, J = ca. 1 Hz, O-$CH_2$); 3,7 (s, 1 H, O-$CH_3$), 3,65 (s, 2 H, $CH_2$); 2,5 (t, 1 H, J = ca. 1 Hz, =CH)

b) $\alpha$-(2-Propinoxyphenyl)-$\beta$-methoxy-acrylsäuremethylester

Eine Mischung von 20 g (0,1 mol) des Phenylessigesters aus Beispiel 3 a, 20 ml Ameisensäuremethylester und 2,7 g (0,11 mol) Natriumhydrid in 100 ml Tetrahydrofuran wird bei Raumtemperatur gerührt. Dabei tritt eine zuerst langsame und dann heftige Gasentwicklung ein. Ist die Gasentwicklung abgeklungen verdünnt man die Reaktionsmischung mit Wasser und extrahiert die wässrige Phase zweimal mit Methyl-t-butylether. Diese organischen Phasen werden verworfen. Dann wird die wässrige Phase angesäuert und dreimal mit Methyl-t-butylether extrahiert. Die vereinigten Phasen werden über Kieselgel abgesaugt und eingeengt.

Der Rückstand wird in 200 ml Aceton aufgenommen und mit 14 g (0,1 mol) $K_2CO_3$ und 11 g (0,09 mol) Dimethylsulfat versetzt. Man rührt 4 Stunden bei Raumtemperatur und engt anschließend die Reaktionsmischung ein. Der Rückstand wird in Methylenchlorid aufgenommen. Die organische Phase wird mit verdünntem Ammoniak und Wasser gewaschen, über $MgSO_4$ getrocknet und eingeengt. Der Rückstand wird säulenchromatographisch mit Hexan/Essigester-Gemsichen gereinigt. Man erhält 11 g (45 %) des trans-Isomeren der Titelverbindung als hellgelben Festkörper.
$^1$H-NMR ($CDCl_3$; $\delta$ in ppm):
7,5 (s, 1 H, Vinyl); 7,25 (m, 2 H, Aromat); 7,05 (m, 2 H, Aromat); 4,65 (d, 2 H, J = ca. 1 Hz, O-$CH_2$); 3,8 (s, 3 H, O-$CH_3$); 3,7 (s, 3 H, O-$CH_3$); 2,5 (t, 1 H, J = ca. 1 Hz, =CH)

c) $\alpha$-(2-(3-(meta-Methylphenyl)-prop-2-inyloxy)-phenyl)-$\beta$-methoxyacrylsäuremethylester (Tabelle 3, Nr. 6)

Eine Mischung von 2 g (8,1 mmol) des Propargylethers aus Beispiel 3 b, 1,8 g (8,2 mmol) meta-Jodtoluol, 2 g (20 mmol) Triethylamin, 0,3 g Triphenylphosphin, 20 mg CuJ und 60 mg Palladium-II-acetat in 20 ml Dimethylformamid wird eine Stunde bei 70 °C gerührt. Dann wird die Reaktionsmischung mit Wasser verdünnt und die wässrige Phase wird dreimal mit Methyl-t-butylether extrahiert. Die vereinigten organischen Phasen werden über $MgSO_4$ getrocknet und eingeengt. Der Rückstand wird säulenchromatographisch mit Hexan/Essigester-Gemischen gereinigt. Man erhält 0,9 g (33 %) der Titelverbindung als kristallinen Festkörper (Fp = 76 °C).
$^1$H-NMR ($CDCl_3$; $\delta$ in ppm):
7,5 (s, 1 H, Vinyl); 6,9 - 7,4 (m, 8 H, Aromat); 4,9 (s, 2 H, O-$CH_2$); 3,8 (s, 3 H, O-$CH_3$); 3,7 (s, 3 H, O-$CH_3$); 2,5 (s, 3 H, $CH_3$)

Beispiel 4

$\alpha$-(2-((3-para-Methylphenylisoxazol-5-yl)-methyloxy)-phenyl)-$\beta$-methoxy-acrylsäuremethylester (Tabelle 3, Nr. 13)

Eine Lösung von 2 g (8,1 mmol) des Propargylethers aus Beispiel 3 b und 1,7 g (12,5 mmol) p-Methylbenzaldehydoxim in 30 ml Methylenchlorid wird mit 50 ml Chlorbleichlauge (enthält 12,5 % aktives $Cl_2$) versetzt. Man rührt 30 Minuten bei Raumtemperatur, trennt die Phasen und extrahiert die wässrige noch dreimal mit Methylenchlorid. Die vereinigten organischen Phasen werden einmal mit Wasser extrahiert, über $MgSO_4$ getrocknet und eingeengt. Der Rückstand kristallisiert und wird mit Methyl-t-butylether/Hexan ausgerührt. man erhält 1,9 g (62 %) des trans-isomeren der Titelverbindung als hellgelben Festkörper (Fp = 82 °C).
$^1$H-NMR ($CDCl_3$; $\delta$ in ppm):
7,7 (d, 2 H, J = 8 Hz, Aromat); 7,55 (s, 1 H, Vinyl); 7,25 (m, 4 H, Aromat); 7,05 (m, 2 H, Aromat); 6,6 (s, 1 H, Isoxazol); 5,2 (s, 2 H, O-$CH_2$); 3,85 (s, 3 H, O-$CH_3$); 3,7 (s, 3 H, O-$CH_3$); 2,4 (s, 3 H, $CH_3$)
In entsprechender Weise könnn die in den folgenden Tabellen genannten Verbindungen hergestellt werden.

Tabelle 1

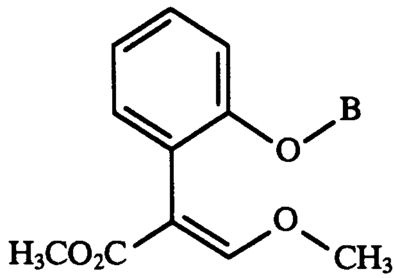

In Tabelle 1 kann die Gruppe B folgende Bedeutungen annehmen:

I: 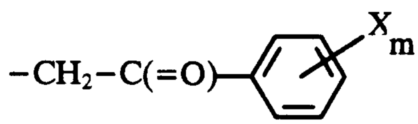

II: 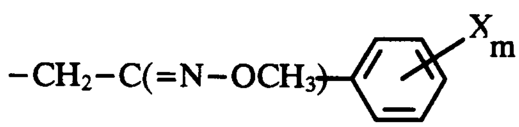

III: 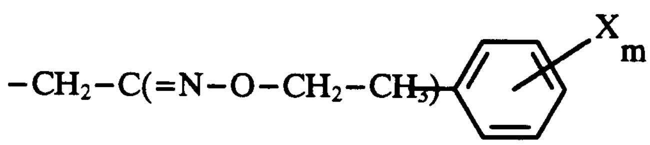

IV: 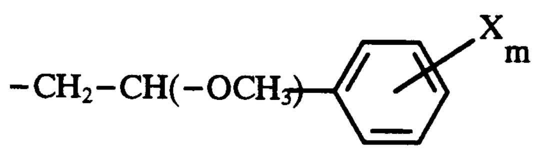

V: 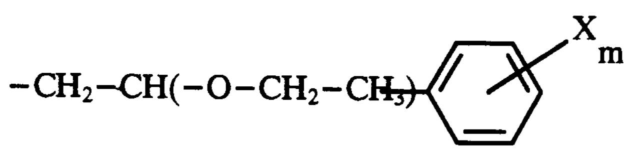

VI: 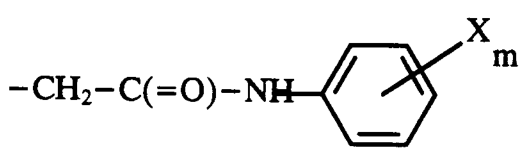

VII: 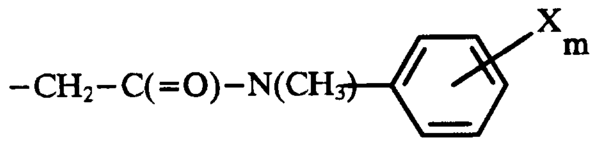

VIII: 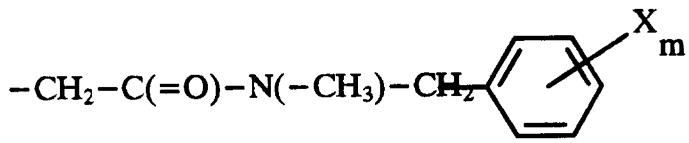

IX: 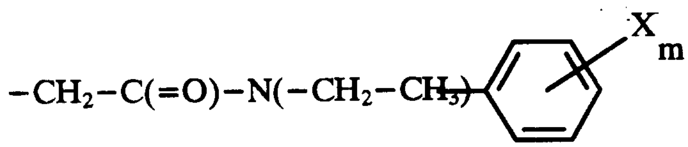

X: 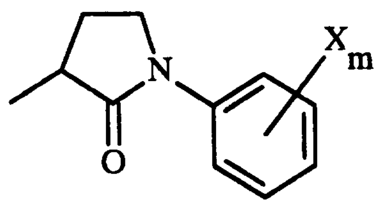

XI: 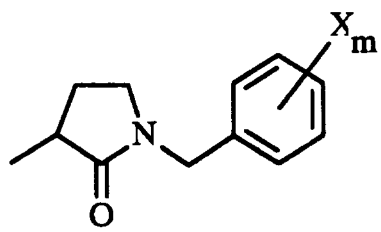

XII: 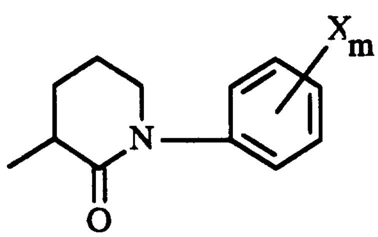

XIII: 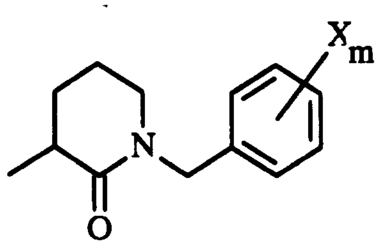

XIV:

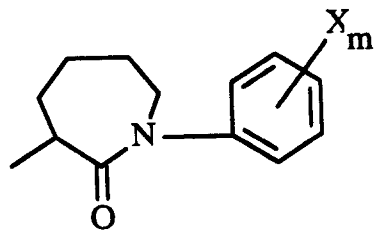

XV:

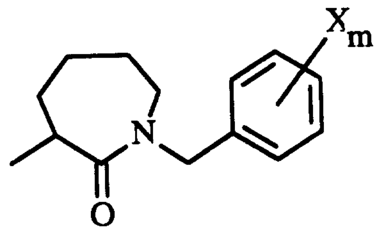

XVI:

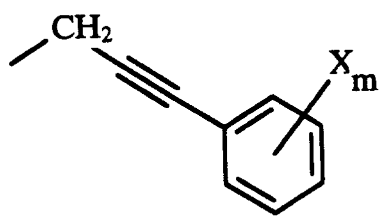

XVII:

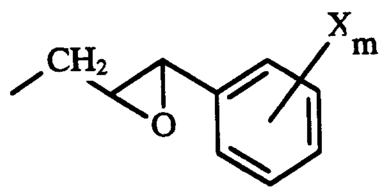

XVIII:

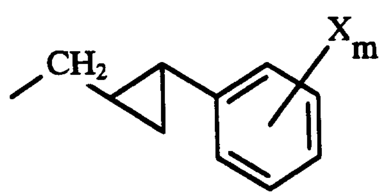

XIX:

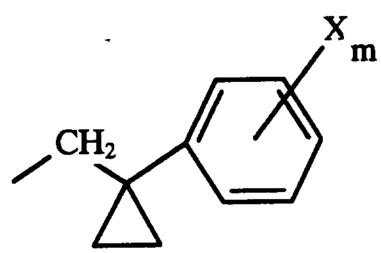

XX:

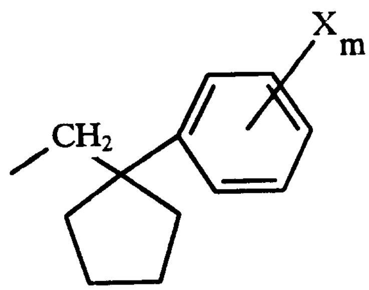

XXI:

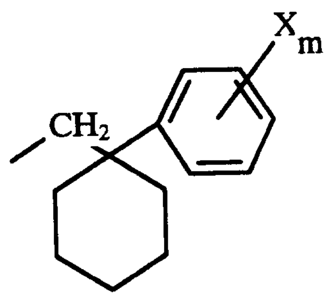

XXII:

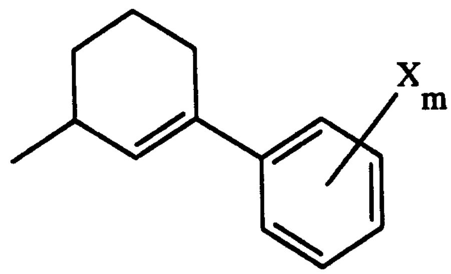

XXIII:

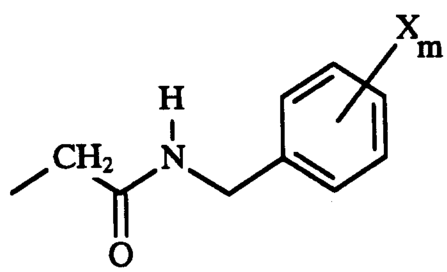

In Tabelle 1 bedeuten beispielsweise die Verbindungen

I/1.2:

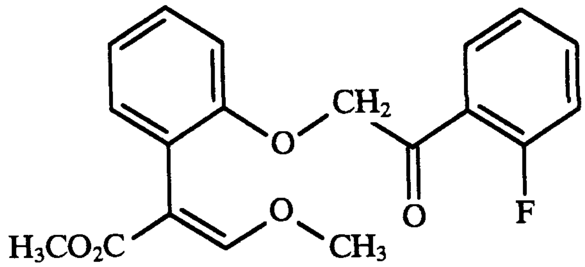

II/1.11:

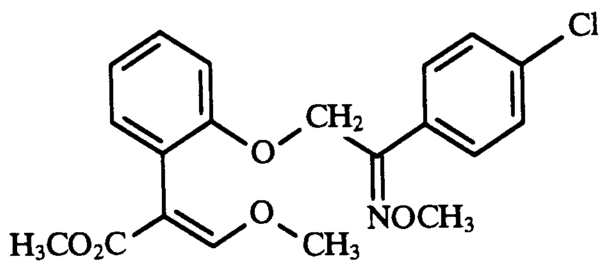

VII/1.66:

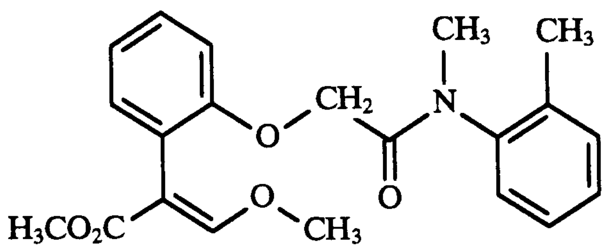

X/1.160:

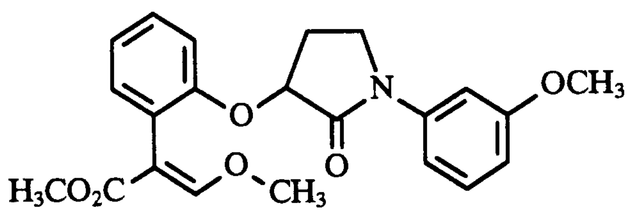

und

XXII/1.66:

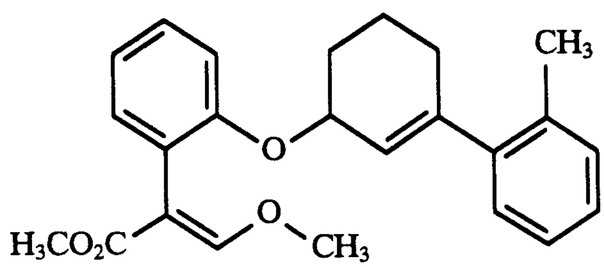

Für jede der Gruppen B (I bis XXIII) kann $X_m$ folgende Bedeutung besitzen

Tabelle 1

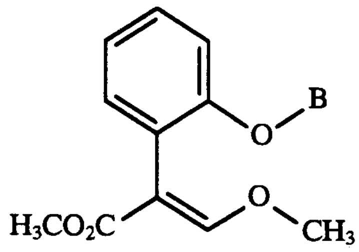

| Nummer | $X_m$ |
|---|---|
| 1 | 4-C(=O)-$C_6H_5$ |
| 2 | 2-F |
| 3 | 3-F |
| 4 | 4-F |
| 5 | 2,4-$F_2$ |
| 6 | 2,4,6-$F_3$ |
| 7 | 2,3,4,5,6-$F_5$ |
| 8 | 2,3-$F_2$ |
| 9 | 2-Cl |
| 10 | 3-Cl |
| 11 | 4-Cl |
| 12 | 2,3-$Cl_2$ |
| 13 | 2,4-$Cl_2$ |
| 14 | 2,5-$Cl_2$ |
| 15 | 2,6-$Cl_2$ |
| 16 | 3,4-$Cl_2$ |
| 17 | 3,5-$Cl_2$ |
| 18 | 2,3,4-$Cl_3$ |
| 19 | 2,3,5-$Cl_3$ |
| 20 | 2,3,6-$Cl_3$ |
| 21 | 2,4,5-$Cl_3$ |
| 22 | 2,4,6-$Cl_3$ |
| 23 | 3,4,5-$Cl_3$ |

| Nummer | $X_m$ |
|---|---|
| 24 | 2,3,4,6-$Cl_4$ |
| 25 | 2,3,5,6-$Cl_4$ |
| 26 | 2,3,4,5,6-$Cl_5$ |
| 27 | 2-Br |
| 28 | 3-Br |
| 29 | 4-Br |
| 30 | 2,4-$Br_2$ |
| 31 | 2,5-$Br_2$ |
| 32 | 2,6-$Br_2$ |
| 33 | 2,4,6-$Br_3$ |
| 34 | 2,3,4,5,6-$Br_5$ |
| 35 | 2-J |
| 36 | 3-J |
| 37 | 4-J |
| 38 | 2,4-$J_2$ |
| 39 | 2-Cl, 3-F |
| 40 | 2-Cl, 4-F |
| 41 | 2-Cl, 5-F |
| 42 | 2-Cl, 6-F |
| 43 | 2-Cl, 3-Br |
| 44 | 2-Cl, 4-Br |
| 45 | 2-Cl, 5-Br |
| 46 | 2-Cl, 6-Br |
| 47 | 2-Br, 3-Cl |
| 48 | 2-Br, 4-Cl |
| 49 | 2-Br, 5-Cl |
| 50 | 2-Br, 3-F |
| 51 | 2-Br, 4-F |
| 52 | 2-Br, 5-F |
| 53 | 2-Br, 6-F |
| 54 | 2-F, 3-Cl |
| 55 | 2-F, 4-Cl |
| 56 | 2-F, 5-Cl |
| 57 | 3-Cl, 4-F |
| 58 | 3-Cl, 5-F |
| 59 | 3-Cl, 4-Br |

| Nummer | $X_m$ |
|---|---|
| 60 | 3-Cl, 5-Br |
| 61 | 3-F, 4-Cl |
| 62 | 3-F, 4-Br |
| 63 | 3-Br, 4-Cl |
| 64 | 3-Br, 4-F |
| 65 | 2,6-$Cl_2$, 4-Br |
| 66 | 2-$CH_3$ |
| 67 | 3-$CH_3$ |
| 68 | 4-$CH_3$ |
| 69 | 2,3-$(CH_3)_2$ |
| 70 | 2,4-$(CH_3)_2$ |
| 71 | 2,5-$(CH_3)_2$ |
| 72 | 2,6-$(CH_3)_2$ |
| 73 | 3,4-$(CH_3)_2$ |
| 74 | 3,5-$(CH_3)_2$ |
| 75 | 2,3,5-$(CH_3)_3$ |
| 76 | 2,3,4-$(CH_3)_3$ |
| 77 | 2,3,6-$(CH_3)_3$ |
| 78 | 2,4,5-$(CH_3)_3$ |
| 79 | 2,4,6-$(CH_3)_3$ |
| 80 | 3,4,5-$(CH_3)_3$ |
| 81 | 2,3,4,6-$(CH_3)_4$ |
| 82 | 2,3,5,6-$(CH_3)_4$ |
| 83 | 2,3,4,5,6-$(CH_3)_5$ |
| 84 | 2-$C_2H_5$ |
| 85 | 3-$C_2H_5$ |
| 86 | 4-$C_2H_5$ |
| 87 | 2,4-$(C_2H_5)_2$ |
| 88 | 2,6-$(C_2H_5)_2$ |
| 89 | 3,5-$(C_2H_5)_2$ |
| 90 | 2,4,6-$(C_2H_5)_3$ |
| 91 | 2-n-$C_3H_7$ |
| 92 | 3-n-$C_3H_7$ |
| 93 | 4-n-$C_3H_7$ |
| 94 | 2-i-$C_3H_7$ |
| 95 | 3-i-$C_3H_7$ |

| Nummer | $X_m$ |
|---|---|
| 96 | 4-i-$C_3H_7$ |
| 97 | 2,4-(i-$C_3H_7$)$_2$ |
| 98 | 2,6-(i-$C_3H_7$)$_2$ |
| 99 | 3,5-(i-$C_3H_7$)$_2$ |
| 100 | 2,4,6-(i-$C_3H_7$)$_3$ |
| 101 | 2-s-$C_4H_9$ |
| 102 | 3-s-$C_4H_9$ |
| 103 | 4-s-$C_4H_9$ |
| 104 | 2-t-$C_4H_9$ |
| 105 | 3-t-$C_4H_9$ |
| 106 | 4-t-$C_4H_9$ |
| 107 | 2,3-(t-$C_4H_9$)$_2$ |
| 108 | 2,4-(t-$C_4H_9$)$_2$ |
| 109 | 2,5-(t-$C_4H_9$)$_2$ |
| 110 | 2,6-(t-$C_4H_9$)$_2$ |
| 111 | 3,4-(t-$C_4H_9$)$_2$ |
| 112 | 2,4,6-(t-$C_4H_9$)$_3$ |
| 113 | 4-n-$C_9H_{19}$ |
| 114 | 4-n-$C_{12}H_{25}$ |
| 115 | 4-n-$C_{15}H_{31}$ |
| 116 | 4-(1,1,3,3-Tetramethylbutyl) |
| 117 | 4-(2,4,4-Trimethylpropyl) |
| 118 | 2-t-$C_4H_9$, 4-$CH_3$ |
| 119 | 2-t-$C_4H_9$, 5-$CH_3$ |
| 120 | 2,6-(t-$C_4H_9$)$_2$, 4-$CH_3$ |
| 121 | 2-$CH_3$, 4-t-$C_4H_9$ |
| 122 | 2-$CH_3$, 6-t-$C_4H_9$ |
| 123 | 2-$CH_3$, 4-i-$C_3H_7$ |
| 124 | 2-$CH_3$, 5-i-$C_3H_7$ |
| 125 | 3-$CH_3$, 4-i-$C_3H_7$ |
| 126 | 2-i-$C_3H_7$, 5-$CH_3$ |
| 127 | 2,4-(t-$C_4H_9$)$_2$, 6-i-$C_3H_7$ |
| 128 | 2-Allyl |
| 129 | 3-Allyl |
| 130 | 4-Allyl |
| 131 | 2-Allyl, 6-$CH_3$ |

| Nummer | $X_m$ |
|---|---|
| 132 | 2-cyclo-$C_6H_{11}$ |
| 133 | 3-cyclo-$C_6H_{11}$ |
| 134 | 4-cyclo-$C_6H_{11}$ |
| 135 | 2,4-(cyclo-$C_6H_{11}$)2, 6-$CH_3$ |
| 136 | 2-$CH_3$, 4-cyclo-$C_6H_{11}$ |
| 137 | 2-$CH_2$-$C_6H_5$ |
| 138 | 3-$CH_2$-$C_6H_5$ |
| 139 | 4-$CH_2$-$C_6H_5$ |
| 140 | 2-$CH_2$-$C_6H_5$, 4-$CH_3$ |
| 141 | 2-$CH_3$, 4-$CH_2$-$C_6H_5$ |
| 142 | 2-$C_6H_5$ |
| 143 | 3-$C_6H_5$ |
| 144 | 4-$C_6H_5$ |
| 145 | 4-(2-i-$C_3H_7$-$C_6H_4$) |
| 146 | 4-$C_6H_5$, 2,6-$(CH_3)_2$ |
| 147 | 2-Cl, 4-$C_6H_5$ |
| 148 | 2-Br, 4-$C_6H_5$ |
| 149 | 2-$C_6H_5$, 4-Cl |
| 150 | 2-$C_6H_5$, 4-Br |
| 151 | 2-$CH_2C_6H_5$, 4-Cl |
| 152 | 2-$CH_2C_6H_5$, 4-Br |
| 153 | 2-Cl, 4-$CH_2C_6H_5$ |
| 154 | 2-Br, 4-$CH_2C_6H_5$ |
| 155 | 2-cyclo-$C_6H_{11}$, 4-Cl |
| 156 | 2-cyclo-$C_6H_{11}$, 4-Br |
| 157 | 2-Cl, 4-cyclo-$C_6H_{11}$ |
| 158 | 2-Br, 4-cyclo-$C_6H_{11}$ |
| 159 | 2-$OCH_3$ |
| 160 | 3-$OCH_3$ |
| 161 | 4-$OCH_3$ |
| 162 | 2-$OC_2H_5$ |
| 163 | 3-O-$C_2H_5$ |
| 164 | 4-O-$C_2H_5$ |
| 165 | 2-O-n-$C_3H_7$ |
| 166 | 3-O-n-$C_3H_7$ |
| 167 | 4-O-n-$C_3H_7$ |

| Nummer | $X_m$ |
|---|---|
| 168 | $2-O-i-C_3H_7$ |
| 169 | $3-O-i-C_3H_7$ |
| 170 | $4-O-i-C_3H_7$ |
| 171 | $2-O-n-C_6H_{13}$ |
| 172 | $3-O-n-C_6H_{13}$ |
| 173 | $4-O-n-C_6H_{13}$ |
| 174 | $2-O-n-C_8H_{17}$ |
| 175 | $3-O-n-C_8H_{17}$ |
| 176 | $4-O-n-C_8H_{17}$ |
| 177 | $2-O-CH_2C_6H_5$ |
| 178 | $3-O-CH_2C_6H_5$ |
| 179 | $4-O-CH_2C_6H_5$ |
| 180 | $2-O-(CH_2)_3C_6H_5$ |
| 181 | $3-O-(CH_2)_3C_6H_5$ |
| 182 | $4-O-(CH_2)_3C_6H_5$ |
| 183 | $2,4-(OCH_3)_2$ |
| 184 | $2-CF_3$ |
| 185 | $3-CF_3$ |
| 186 | $4-CF_3$ |
| 187 | $2-OCF_3$ |
| 188 | $3-OCF_3$ |
| 189 | $4-OCF_3$ |
| 190 | $3-OCH_2CHF_2$ |
| 191 | $2-NO_2$ |
| 192 | $3-NO_2$ |
| 193 | $4-NO_2$ |
| 194 | 2-CN |
| 195 | 3-CN |
| 196 | 4-CN |
| 197 | $2-CH_3$, 3-Cl |
| 198 | $2-CH_3$, 4-Cl |
| 199 | $2-CH_3$, 5-Cl |
| 200 | $2-CH_3$, 6-Cl |
| 201 | $2-CH_3$, 3-F |
| 202 | $2-CH_3$, 4-F |
| 203 | $2-CH_3$, 5-F |

| Nummer | $X_m$ |
|---|---|
| 204 | 2-$CH_3$, 6-F |
| 205 | 2-$CH_3$, 3-Br |
| 206 | 2-$CH_3$, 4-Br |
| 207 | 2-$CH_3$, 5-Br |
| 208 | 2-$CH_3$, 6-Br |
| 209 | 2-Cl, 3-$CH_3$ |
| 210 | 2-Cl, 4-$CH_3$ |
| 211 | 2-Cl, 5-$CH_3$ |
| 212 | 2-F, 3-$CH_3$ |
| 213 | 2-F, 4-$CH_3$ |
| 214 | 2-F, 5-$CH_3$ |
| 215 | 2-Br, 3-$CH_3$ |
| 216 | 2-Br, 4-$CH_3$ |
| 217 | 2-Br, 5-$CH_3$ |
| 218 | 3-$CH_3$, 4-Cl |
| 219 | 3-$CH_3$, 5-Cl |
| 220 | 3-$CH_3$, 4-F |
| 221 | 3-$CH_3$, 5-F |
| 222 | 3-$CH_3$, 4-Br |
| 223 | 3-$CH_3$, 5-Br |
| 224 | 3-F, 4-$CH_3$ |
| 225 | 3-Cl, 4-$CH_3$ |
| 226 | 3-Br, 4-$CH_3$ |
| 227 | 2-Cl, 4,5-$(CH_3)_2$ |
| 228 | 2-Br, 4,5-$(CH_3)_2$ |
| 229 | 2-Cl, 3,5-$(CH_3)_2$ |
| 230 | 2- Br, 3,5-$(CH_3)_2$ |
| 231 | 2,6-$Cl_2$, 4-$CH_3$ |
| 32 | 2,6-$F_2$, 4-$CH_3$ |
| 233 | 2,6-$Br_2$, 4-$CH_3$ |
| 234 | 2,4-$Br_2$, 6-$CH_3$ |
| 235 | 2,4-$F_2$, 6-$CH_3$ |
| 236 | 2,4-$Br_2$, 6-$CH_3$ |
| 237 | 2,6-$(CH_3)_2$, 4-F |
| 238 | 2,6-$(CH_3)_2$, 4-Cl |
| 239 | 2,6-$(CH_3)_2$, 4-Br |

| Nummer | $X_m$ |
|---|---|
| 240 | 3,5-$(CH_3)_2$, 4-F |
| 241 | 3,5-$(CH_3)_2$, 4-Cl |
| 242 | 3,5-$(CH_3)_2$, 4-Br |
| 243 | 2,3,6-$(CH_3)_3$, 4-F |
| 244 | 2,3,6-$(CH_3)_3$, 4-Cl |
| 245 | 2,3,6-$(CH_3)_3$, 4-Br |
| 246 | 2,4-$(CH_3)_2$, 6-F |
| 247 | 2,4-$(CH_3)_2$, 6-Cl |
| 248 | 2,4-$(CH_3)_2$, 6-Br |
| 249 | 2-i-$C_3H_7$, 4-Cl, 5-$CH_3$ |
| 250 | 2-Cl, 4-$NO_2$ |
| 251 | 2-$NO_2$, 4-Cl |
| 252 | 2-$OCH_3$, 5-$NO_2$ |
| 253 | 2,4-$Cl_2$, 5-$NO_2$ |
| 254 | 2,4-$Cl_2$, 6-$NO_2$ |
| 255 | 2,6-$Cl_2$, 4-$NO_2$ |
| 256 | 2,6-$Br_2$, 4-$NO_2$ |
| 257 | 2,6-$J_2$, 4-$NO_2$ |
| 258 | 2-$CH_3$, 5-i-$C_3H_7$, 4-Cl |
| 259 | 2-$CO_2CH_3$ |
| 260 | 3-$CO_2CH_3$ |
| 261 | 4-$CO_2CH_3$ |
| 262 | 2-$CO_2(C_2H_5)$ |
| 263 | 3-$CO_2(C_2H_5)$ |
| 264 | 4-$CO_2(C_2H_5)$ |
| 265 | 2-$CO_2$(n-$C_3H_7$) |
| 266 | 3-$CO_2$(n-$C_3H_7$) |
| 267 | 4-$CO_2$(n-$C_3H_7$) |
| 268 | 2-$CO_2$(i-$C_3H_7$) |
| 269 | 3-$CO_2$(i-$C_3H_7$) |
| 270 | 4-$CO_2$(i-$C_3H_7$) |
| 271 | 2-$CO_2$(n-$C_6H_{13}$) |
| 272 | 3-$CO_2$(n-$C_6H_{13}$) |
| 273 | 4-$CO_2$(n-$C_6H_{13}$) |
| 274 | 2-$CH_2$-$OCH_3$ |
| 275 | 3-$CH_2$-$OCH_3$ |

| Nummer | $X_m$ |
|---|---|
| 276 | 4-$CH_2$-$OCH_3$ |
| 277 | 2-$CH_2O(C_2H_5)$ |
| 278 | 3-$CH_2O(C_2H_5)$ |
| 279 | 4-$CH_2O(C_2H_5)$ |
| 280 | 2-$CH_2O(n-C_3H_7)$ |
| 281 | 3-$CH_2O(n-C_3H_7)$ |
| 282 | 4-$CH_2O(n-C_3H_7)$ |
| 283 | 2-$CH_2O(i-C_3H_7)$ |
| 284 | 3-$CH_2O(i-C_3H_7)$ |
| 285 | 4-$CH_2O(i-C_3H_7)$ |
| 286 | 2-CHO |
| 287 | 3-CHO |
| 288 | 4-CHO |
| 289 | 2-CO-$CH_3$ |
| 290 | 3-CO-$CH_3$ |
| 291 | 4-CO-$CH_3$ |
| 292 | 2-CO-$CH_2$-$CH_3$ |
| 293 | 3-CO-$CH_2$-$CH_3$ |
| 294 | 4-CO-$CH_2$-$CH_3$ |
| 295 | 2-CO-$CH_2$-$CH_2$-$CH_3$ |
| 296 | 3-CO-$CH_2$-$CH_2$-$CH_3$ |
| 297 | 4-CO-$CH_2$-$CH_2$-$CH_3$ |
| 298 | 2-CO-$CH(CH_3)$-$CH_3$ |
| 299 | 3-CO-$CH(CH_3)$-$CH_3$ |
| 300 | 4-CO-$CH(CH_3)$-$CH_3$ |
| 301 | 2-Me-4-CHO |
| 302 | 2-Me-4-$CH_3$-CO |
| 303 | 2-Me-4-$CH_3$-$CH_2$-CO |
| 304 | 2-Me-4-$CH_3$-$CH_2$-$CH_2$-CO |
| 305 | 2-Me-4-$CH_3$-$CH(CH_3)$-CO |
| 306 | 2,5-$Me_2$-4-CHO |
| 307 | 2,5-$Me_2$-4-$CH_3$-CO |
| 308 | 2,5-$Me_2$-4-$CH_3$-$CH_2$-CO |
| 309 | 2,5-$Me_2$-4-$CH_3$-$CH_2$-$CH_2$-CO |
| 310 | 2,5-$Me_2$-4-$CH_3$-$CH(CH_3)$-CO |
| 311 | 2-Cl-4-CHO |

| Nummer | $X_m$ |
|---|---|
| 312 | 2-Cl-4-$CH_3$-CO |
| 313 | 2-Cl-4-$CH_3$-$CH_2$-CO |
| 314 | 2-Cl-4-$CH_3$-CH($CH_3$)-CO |
| 315 | 2,5-$Cl_2$-4-CHO |
| 316 | 2,5-$Cl_2$-4-$CH_3$-CO |
| 317 | 2,5-$Cl_2$-4-$CH_3$-$CH_2$-CO |
| 318 | 2,5-$Cl_2$-4-$CH_3$-$CH_2$-$CH_2$-CO |
| 319 | 2,5-$Cl_2$-4-$CH_3$--CH($CH_3$)-CO |
| 320 | 2-C(=$NOCH_3$)-$CH_3$ |
| 321 | 3-C(=$NOCH_3$)-$CH_3$ |
| 322 | 4-C(=$NOCH_3$)-$CH_3$ |
| 323 | 2-C(=$NOC_2H_5$)-$CH_3$ |
| 324 | 3-C(=$NOC_2H_5$)-$CH_3$ |
| 325 | 4-C(=$NOC_2H_5$)-$CH_3$ |
| 326 | 2-C(=NO-n-$C_3H_7$)-$CH_3$ |
| 327 | 3-C(=NO-n-$C_3H_7$)-$CH_3$ |
| 328 | 4-C(=NO-n-$C_3H_7$)-$CH_3$ |
| 329 | 2-C(=NO-i-$C_3H_7$)-$CH_3$ |
| 330 | 3-C(=NO-i-$C_3H_7$)-$CH_3$ |
| 331 | 4-C(=NO-i-$C_3H_7$)-$CH_3$ |
| 332 | 2-C(=NO-Allyl)-$CH_3$ |
| 333 | 3-C(=NO-Allyl)-$CH_3$ |
| 334 | 4-C(=NO-Allyl)-$CH_3$ |
| 335 | 2-C(=NO-trans-Chlorallyl)-$CH_3$ |
| 336 | 3-C(=NO-trans-Chlorallyl)-$CH_3$ |
| 337 | 4-C(=NO-trans-Chlorallyl)-$CH_3$ |
| 338 | 2-C(=NO-Propargyl)-$CH_3$ |
| 339 | 3-C(=NO-Propargyl)-$CH_3$ |
| 340 | 4-C(=NO-Propargyl)-$CH_3$ |
| 341 | 2-C(=NO-n-$C_4H_9$)-$CH_3$ |
| 342 | 3-C(=NO-n-$C_4H_9$)-$CH_3$ |
| 343 | 4-C(=NO-n-$C_4H_9$)-$CH_3$ |
| 344 | 2-C(=NO-$CH_2$-$C_6H_5$)-$CH_3$ |
| 345 | 3-C(=NO-$CH_2$-$C_6H_5$)-$CH_3$ |
| 346 | 4-C(=NO-$CH_2$-$C_6H_5$)-$CH_3$ |
| 347 | 2-$CH_3$-4-CH=$NOCH_3$ |

| Nummer | $X_m$ |
|---|---|
| 348 | $2-CH_3-4-CH=NOC_2H_5$ |
| 349 | $2-CH_3-4-CH=NO-n-C_3H_7$ |
| 350 | $2-CH_3-4-CH=NO-i-C_3H_7$ |
| 351 | $2-CH_3-4-CH=NO-$Allyl |
| 352 | 2-CH3-4-CH=NO-(trans-Chlorallyl) |
| 353 | $2-CH_3-4-CH=NO-$Propargyl |
| 354 | $2-CH_3-4-CH=NO-n-C_4H_9$ |
| 355 | $2-CH_3-4-CH=NO-CH_2-C_6H_5$ |
| 356 | $2-CH_3-4-(CH_3-C=NOCH_3)$ |
| 357 | $2-CH_3-4-(CH_3-C=NOC_2H_5)$ |
| 358 | $2-CH_3-4-(CH_3-C=NO-n-C_3H_7)$ |
| 359 | $2-CH_3-4-(CH_3-C=NO-i-C_3H_7)$ |
| 360 | $2-CH_3-4-(CH_3-C=NO-$Allyl) |
| 361 | $2-CH_3-4-(CH_3-C=NO-$trans-Chlorallyl) |
| 362 | $2-CH_3-4-(CH_3-C=NO-$Propargyl) |
| 363 | $2-CH_3-4-(CH_3-C=NO-n-C_4H_9)$ |
| 364 | $2-CH_3-4-(CH_3-C=NO-CH_2-C_6H_5)$ |
| 365 | $2-CH_3-4-(C_2H_5-C=NO-CH_3)$ |
| 366 | $2-CH_3-4-(C_2H_5-C=NO-C_2H_5)$ |
| 367 | $2-CH_3-4-(C_2H_5-C=NO-n-C_3H_7)$ |
| 368 | $2-CH_3-4-(C_2H_5-C=NO-i-C_3H_7$ |
| 369 | $2-CH_3-4-(C_2H_5-C=NO-$Allyl) |
| 370 | $2-CH_3-4-(C_2H_5-C=NO-$trans-Chlorallyl) |
| 371 | $2-CH_3-4-(C_2H_5-C=NO-$Propargyl) |
| 372 | $2-CH_3-4-(C_2H_5-C=NO-n-C_4H_9)$ |
| 373 | $2-CH_3-4-(C_2H_5-C=NO-CH_2-C_6H_5)$ |
| 374 | $2,5-(CH_3)_2-4-(CH_3-C=NOCH_3)$ |
| 375 | $2,5-(CH_3)_2-4-(CH_3-C=NOC_2H_5)$ |
| 376 | $2,5-(CH_3)_2-4-(CH_3-C=NO-n-C_3H_7)$ |
| 377 | $2,5-(CH_3)_2-4-(CH_3-C=NO-i-C_3H_7)$ |
| 378 | $2,5-(CH_3)_2-4-(CH_3-C=NO-$Allyl) |
| 379 | $2,5-(CH_3)_2-4-(CH_3-C=NO-$trans-Chlorallyl) |
| 380 | $2,5-(CH_3)_2-4-(CH_3-C=NO-$Proparyl) |
| 381 | $2,5-(CH_3)_2-4-(CH_3-C=NO-n-C_4H_9)$ |
| 382 | $2,5-(CH_3)_2-4-(CH_3-C=NO-CH_2-C_6H_5)$ |
| 383 | $2-C_6H_5$ |

| Nummer | $X_m$ |
|---|---|
| 384 | 3-$C_6H_5$ |
| 385 | 4-$C_6H_5$ |
| 386 | 2-(2'-F-C6H4) |
| 387 | 2-(3'-F-$C_6H_4$) |
| 388 | 2-(4'-F-$C_6H_4$) |
| 389 | 3-(2'-F-$C_6H_4$) |
| 390 | 3-(3'-F-$C_6H_4$) |
| 391 | 3-(4'-F-$C_6H_4$) |
| 392 | 4-(2'-F-$C_6H_4$) |
| 393 | 4-(3'-F-$C_6H_4$) |
| 394 | 4-(4'-F-$C_6H_4$) |
| 395 | 2-(2'-Cl-$C_6H_4$) |
| 396 | 2-(3'-Cl-$C_6H_4$) |
| 397 | 2-(4'-Cl-$C_6H_4$) |
| 398 | 3-(2'-Cl-$C_6H_4$) |
| 399 | 3-(3'-Cl-$C_6H_4$) |
| 400 | 3-(4'-Cl-$C_6H_4$) |
| 401 | 4-(2'-Cl-$C_6H_4$) |
| 402 | 4-(3'-Cl-$C_6H_4$) |
| 403 | 4-(4'-Cl-$C_6H_4$) |
| 405 | 2-(2'-$CH_3$-$C_6H_4$) |
| 406 | 2-(3'-$CH_3$-$C_6H_4$) |
| 407 | 2-(4'-$CH_3$-$C_6H_4$) |
| 408 | 3-(2'-$CH_3$-$C_6H_4$) |
| 409 | 3-(3'-$CH_3$-$C_6H_4$) |
| 410 | 3-(4'-$CH_3$-$C_6H_4$) |
| 411 | 4-(2'-$CH_3$-$C_6H_4$) |
| 412 | 4-(3'-$CH_3$-$C_6H_4$) |
| 413 | 4-(4'-$CH_3$-$C_6H_4$) |
| 414 | 2-(2'-$CH_3$-CO-$C_6H_4$) |
| 415 | 2-(3'-$CH_3$-CO-$C_6H_4$) |
| 416 | 2-(4'-$CH_3$-CO-$C_6H_4$) |
| 417 | 3-(2'-$CH_3$-CO-$C_6H_4$) |
| 418 | 3-(3'-$CH_3$-CO-$C_6H_4$) |
| 419 | 3-(4'-$CH_3$-CO-$C_6H_4$) |
| 420 | 4-(2'-$CH_3$-CO-$C_6H_4$) |

| Nummer | $X_m$ |
|---|---|
| 421 | 4-(3'-$CH_3$-CO-$C_6H_4$) |
| 422 | 4-(4'-$CH_3$-CO-$C_6H_4$) |
| 423 | 2-(2'-($CH_3$-C(=NOAllyl))-$C_6H_4$) |
| 424 | 2-(3'-($CH_3$-C(=NOAllyl))-$C_6H_4$) |
| 425 | 2-(4'-($CH_3$-C(=NOAllyl))-$C_6H_4$) |
| 426 | 3-(2'-($CH_3$-C(=NOAllyl))-$C_6H_4$) |
| 427 | 3-(3'-($CH_3$-C(=NOAllyl))-$C_6H_4$) |
| 428 | 3-(4'-($CH_3$-C(=NOAllyl))-$C_6H_4$) |
| 429 | 4-(2'-($CH_3$-C(=NOAllyl))-$C_6H_4$) |
| 430 | 4-(3'-($CH_3$-C(=NOAllyl))-$C_6H_4$) |
| 431 | 4-(4'-($CH_3$-C(=NOAllyl))-$C_6H_4$) |
| 432 | 2-(2'-$CH_3O_2C$-$C_6H_4$) |
| 433 | 2-(3'-$CH_3O_2C$-$C_6H_4$) |
| 434 | 2-(4'-$CH_3O_2C$-$C_6H_4$) |
| 435 | 3-(2'-$CH_3O_2C$-$C_6H_4$) |
| 436 | 3-(3'-$CH_3O_2C$-$C_6H_4$) |
| 437 | 3-(4'-$CH_3O_2C$-$C_6H_4$) |
| 438 | 4-(2'-$CH_3O_2C$-$C_6H_4$) |
| 439 | 4-(3'-$CH_3O_2C$-$C_6H_4$) |
| 440 | 4-(4'-$CH_3O_2C$-$C_6H_4$) |
| 441 | 2-(2'-$CH_3O$-$C_6H_4$) |
| 442 | 2-(3'-$CH_3O$-$C_6H_4$) |
| 443 | 2-(4'-$CH_3O$-$C_6H_4$) |
| 444 | 3-(2'-$CH_3O$-$C_6H_4$) |
| 445 | 3-(3'-$CH_3O$-$C_6H_4$) |
| 446 | 3-(4'-$CH_3O$-$C_6H_4$) |
| 447 | 4-(2'-$CH_3O$-$C_6H_4$) |
| 448 | 4-(3'-$CH_3O$-$C_6H_4$) |
| 449 | 4-(4'-$CH_3O$-$C_6H_4$) |
| 450 | 2-(2'-$O_2N$-$C_6H_4$) |
| 451 | 2-(3'-$O_2N$-$C_6H_4$) |
| 452 | 2-(4'-$O_2N$-$C_6H_4$) |
| 453 | 3-(2'-$O_2N$-$C_6H_4$) |
| 454 | 3-(3'-$O_2N$-$C_6H_4$) |
| 455 | 3-(4'-$O_2N$-$C_6H_4$) |
| 456 | 4-(2'-$O_2N$-$C_6H_4$) |

| Nummer | $X_m$ |
|---|---|
| 457 | 4-(3'-$O_2N$-$C_6H_4$) |
| 458 | 4-(4'-$O_2N$-$C_6H_4$) |
| 459 | 2-(2'-NC-$C_6H_4$) |
| 460 | 2-(3'-NC-$C_6H_4$) |
| 461 | 2-(4'-NC-$C_6H_4$) |
| 462 | 3-(2'-NC-$C_6H_4$) |
| 463 | 3-(3'-NC-$C_6H_4$) |
| 464 | 3-(4'-NC-$C_6H_4$) |
| 465 | 4-(2'-NC-$C_6H_4$) |
| 466 | 4-(3'-NC-$C_6H_4$) |
| 467 | 4-(4'-NC-$C_6H_4$) |
| 468 | 2-(2'-$CF_3$-$C_6H_4$) |
| 469 | 2-(3'-$CF_3$-$C_6H_4$) |
| 470 | 2-(4'-$CF_3$-$C_6H_4$) |
| 471 | 3-(2'-$CF_3$-$C_6H_4$) |
| 472 | 3-(3'-$CF_3$-$C_6H_4$) |
| 473 | 3-(4'-$CF_3$-$C_6H_4$) |
| 474 | 4-(2'-$CF_3$-$C_6H_4$) |
| 475 | 4-(3'-$CF_3$-$C_6H_4$) |
| 476 | 4-(4'-$CF_3$-$C_6H_4$) |
| 477 | 2-O-$C_6H_5$ |
| 475 | 3-O-$C_6H_5$ |
| 476 | 4-O-$C_6H_5$ |
| 478 | 2-O-(2'-F-C6H4) |
| 479 | 2-O-(3'-F-$C_6H_4$) |
| 480 | 2-O-(4'-F-$C_6H_4$) |
| 481 | 3-O-(2'-F-$C_6H_4$) |
| 482 | 3-O-(3'-F-$C_6H_4$) |
| 483 | 3-O-(4'-F-$C_6H_4$) |
| 484 | 4-O-(2'-F-$C_6H_4$) |
| 485 | 4-O-(3'-F-$C_6H_4$) |
| 486 | 4-O-(4'-F-$C_6H_4$) |
| 487 | 2-O-(2'-Cl-$C_6H_4$) |
| 488 | 2-O-(3'-Cl-$C_6H_4$) |
| 489 | 2-O-(4'-Cl-$C_6H_4$) |
| 490 | 3-O-(2'-Cl-$C_6H_4$) |

| Nummer | $X_m$ |
|---|---|
| 491 | 3-O-(3'-Cl-$C_6H_4$) |
| 492 | 3-O-(4'-Cl-$C_6H_4$) |
| 493 | 3-O-(4'-Cl-$C_6H_4$) |
| 494 | 4-O-(2'-Cl-$C_6H_4$) |
| 495 | 4-O-(3'-Cl-$C_6H_4$) |
| 496 | 4-O-(4'-Cl-$C_6H_4$) |
| 497 | 2-O-(2'-$CH_3$-$C_6H_4$) |
| 498 | 2-O-(3'-$CH_3$-$C_6H_4$) |
| 499 | 2-O-(4'-$CH_3$-$C_6H_4$) |
| 500 | 3-O-(2'-$CH_3$-$C_6H_4$) |
| 501 | 3-O-(3'-$CH_3$-$C_6H_4$) |
| 502 | 3-O-(4'-$CH_3$-$C_6H_4$) |
| 503 | 4-O-(2'-$CH_3$-$C_6H_4$) |
| 504 | 4-O-(3'-$CH_3$-$C_6H_4$) |
| 505 | 4-O-(4'-$CH_3$-$C_6H_4$) |
| 506 | 2-O-(2'-$CH_3$-CO-$C_6H_4$) |
| 507 | 2-O-(3'-$CH_3$-CO-$C_6H_4$) |
| 508 | 2-O-(4'-$CH_3$-CO-$C_6H_4$) |
| 509 | 3-O-(2'-$CH_3$-CO-$C_6H_4$) |
| 510 | 3-O-(3'-$CH_3$-CO-$C_6H_4$) |
| 511 | 3-O-(4'-$CH_3$-CO-$C_6H_4$) |
| 512 | 4-O-(2'-$CH_3$-CO-$C_6H_4$) |
| 513 | 4-O-(3'-$CH_3$-CO-$C_6H_4$) |
| 514 | 4-O-(4'-$CH_3$-CO-$C_6H_4$) |
| 515 | 2-O-(2'-($CH_3$-C(=NOAllyl))-$C_6H_4$) |
| 516 | 2-O-(3'-($CH_3$-C(=NOAllyl))-$C_6H_4$) |
| 517 | 2-O-(4'-($CH_3$-C(=NOAllyl))-$C_6H_4$) |
| 518 | 3-O-(2'-($CH_3$-C(=NOAllyl))-$C_6H_4$) |
| 519 | 3-O-(3'-($CH_3$-C(=NOAllyl))-$C_6H_4$) |
| 520 | 3-O-(4'-($CH_3$-C(=NOAllyl))-$C_6H_4$) |
| 521 | 4-O-(2'-($CH_3$-C(=NOAllyl))-$C_6H_4$) |
| 522 | 4-O-(3'-($CH_3$-C(=NOAllyl))-$C_6H_4$) |
| 523 | 4-O-(4'-($CH_3$-C(=NOAllyl))-$C_6H_4$) |
| 524 | 2-O-(2'-$CH_3O_2C$-$C_6H_4$) |
| 525 | 2-O-(3'-$CH_3O_2C$-$C_6H_4$) |
| 526 | 2-O-(4'-$CH_3O_2C$-$C_6H_4$) |

| Nummer | $X_m$ |
|---|---|
| 527 | 3-O-(2'-$CH_3O_2C$-$C_6H_4$) |
| 528 | 3-O-(3'-$CH_3O_2C$-$C_6H_4$) |
| 529 | 3-O-(4'-$CH_3O_2C$-$C_6H_4$) |
| 530 | 4-O-(2'-$CH_3O_2C$-$C_6H_4$) |
| 531 | 4-O-(3'-$CH_3O_2C$-$C_6H_4$) |
| 532 | 4-O-(4'-$CH_3O_2C$-$C_6H_4$) |
| 533 | 2-O-(2'-$CH_3O$-$C_6H_4$) |
| 534 | 2-O-(3'-$CH_3O$-$C_6H_4$) |
| 535 | 2-O-(4'-$CH_3O$-$C_6H_4$) |
| 536 | 3-O-(2'-$CH_3O$-$C_6H_4$) |
| 537 | 3-O-(3'-$CH_3O$-$C_6H_4$) |
| 538 | 3-O-(4'-$CH_3O$-$C_6H_4$) |
| 539 | 4-O-(2'-$CH_3O$-$C_6H_4$) |
| 540 | 4-O-(3'-$CH_3O$-$C_6H_4$) |
| 541 | 4-O-(4'-$CH_3O$-$C_6H_4$) |
| 542 | 2-O-(2'-$O_2N$-$C_6H_4$) |
| 543 | 2-O-(3'-$O_2N$-$C_6H_4$) |
| 544 | 2-O-(4'-$O_2N$-$C_6H_4$) |
| 545 | 3-O-(2'-$O_2N$-$C_6H_4$) |
| 546 | 3-O-(3'-$O_2N$-$C_6H_4$) |
| 547 | 3-O-(4'-$O_2N$-$C_6H_4$) |
| 548 | 4-O-(2'-$O_2N$-$C_6H_4$) |
| 549 | 4-O-(3'-$O_2N$-$C_6H_4$) |
| 550 | 4-O-(4'-$O_2N$-$C_6H_4$) |
| 551 | 2-O-(2'-NC-$C_6H_4$) |
| 552 | 2-O-(3'-NC-$C_6H_4$) |
| 553 | 2-O-(4'-NC-$C_6H_4$) |
| 554 | 3-O-(2'-NC-$C_6H_4$) |
| 555 | 3-O-(3'-NC-$C_6H_4$) |
| 556 | 3-O-(4'-NC-$C_6H_4$) |
| 557 | 4-O-(2'-NC-$C_6H_4$) |
| 558 | 4-O-(3'-NC-$C_6H_4$) |
| 559 | 4-O-(4'-NC-$C_6H_4$) |
| 560 | 2-O-(2'-$CF_3$-$C_6H_4$) |
| 561 | 2-O-(3'-$CF_3$-$C_6H_4$) |
| 562 | 2-O-(4'-$CF_3$-$C_6H_4$) |

| Nummer | $X_m$ |
|---|---|
| 563 | 3-O-(2'-$CF_3$-$C_6H_4$) |
| 564 | 3-O-(3'-$CF_3$-$C_6H_4$) |
| 565 | 3-O-(4'-$CF_3$-$C_6H_4$) |
| 566 | 4-O-(2'-$CF_3$-$C_6H_4$) |
| 567 | 4-O-(3'-$CF_3$-$C_6H_4$) |
| 568 | 4-O-(4'-$CF_3$-$C_6H_4$) |
| 569 | 2-Pyridinyl-2' |
| 570 | 2-Pyridinyl-3' |
| 571 | 2-Pyridinyl-4' |
| 572 | 3-Pyridinyl-2' |
| 573 | 3-Pyridinyl-3' |
| 574 | 3-Pyridinyl-4' |
| 575 | 4-Pyridinyl-2' |
| 576 | 4-Pyridinyl-3' |
| 577 | 4-Pyridinyl-4' |
| 578 | 2-Pyrimidinyl-2' |
| 579 | 2-Pyrimidinyl-3' |
| 580 | 2-Pyrimidinyl-4' |
| 581 | 3-Pyrimidinyl-2' |
| 582 | 3-Pyrimidinyl-3' |
| 583 | 3-Pyrimidinyl-4' |
| 584 | 4-Pyrimidinyl-2' |
| 585 | 4-Pyrimidinyl-3' |
| 586 | 4-Pyrimidinyl-4' |
| 587 | 2-Pyrazolyl-1' |
| 588 | 2-Pyrazolyl-3' |
| 589 | 2-Pyrazolyl-4' |
| 590 | 3-Pyrazolyl-1' |
| 591 | 3-Pyrazolyl-3' |
| 592 | 3-Pyrazolyl-4' |
| 593 | 4-Pyrazolyl-1' |
| 594 | 4-Pyrazolyl-3' |
| 595 | 4-Pyrazolyl-4' |
| 596 | 2-Isoxazolyl-3' |
| 597 | 2-Isoxazolyl-4' |
| 598 | 2-Isoxazolyl-5' |

| Nummer | $X_m$ |
|---|---|
| 599 | 3-Isoxazolyl-3' |
| 600 | 3-Isoxazolyl-4' |
| 601 | 3-Isoxazolyl-5' |
| 602 | 4-Isoxazolyl-3' |
| 603 | 4-Isoxazolyl-4' |
| 604 | 4-Isoxazolyl-5' |
| 605 | 2-Isothiazolyl-3' |
| 606 | 2-Isothiazolyl-4' |
| 607 | 2-Isothiazolyl-5' |
| 608 | 3-Isothiazolyl-3' |
| 609 | 3-Isothiazolyl-4' |
| 610 | 3-Isothiazolyl-5' |
| 611 | 4-Isothiazolyl-3' |
| 612 | 4-Isothiazolyl-4' |
| 613 | 4-Isothiazolyl-5' |
| 614 | 2-Imidazolyl-1' |
| 615 | 2-Imidazolyl-2' |
| 616 | 2-Imidazolyl-4' |
| 617 | 3-Imidazolyl-1' |
| 618 | 3-Imidazolyl-2' |
| 619 | 3-Imidazolyl-4' |
| 620 | 4-Imidazolyl-1' |
| 621 | 4-Imidazolyl-2' |
| 622 | 4-Imidazolyl-4' |
| 623 | 2-Oxazolyl-2' |
| 624 | 2-Oxazolyl-4' |
| 625 | 2-Oxazolyl-5' |
| 626 | 3-Oxazolyl-2' |
| 627 | 3-Oxazolyl-4' |
| 628 | 3-Oxazolyl-5' |
| 629 | 4-Oxazolyl-2' |
| 630 | 4-Oxazolyl-4' |
| 631 | 4-Oxazolyl-5' |
| 632 | 2-Thiazolyl-2' |
| 633 | 2-Thiazolyl-4' |
| 634 | 2-Thiazolyl-5' |

| Nummer | $X_m$ |
|---|---|
| 635 | 3-Thiazolyl-2' |
| 636 | 3-Thiazolyl-4' |
| 637 | 3-Thiazolyl-5' |
| 638 | 4-Thiazolyl-2' |
| 639 | 4-Thiazolyl-4' |
| 640 | 4-Thiazolyl-5' |

Tabelle 2

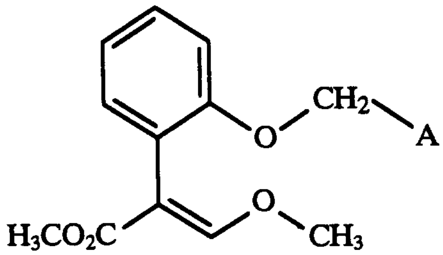

| Nummer | A |
|---|---|
| 1 | Pyrrolyl-3 |
| 2 | N-$CH_3$-Pyrrolyl-3 |
| 3 | N-$C_6H_5$-Pyrrolyl-3 |
| 4 | N-(4'-$CH_3$-$C_6H_4$)-Pyrrolyl-3 |
| 5 | N-(3'-$CH_3$-$C_6H_4$)-Pyrrolyl-3 |
| 6 | N-(2'-$CH_3$-$C_6H_4$)-Pyrrolyl-3 |
| 7 | N-(4'-$CH_3O$-$C_6H_4$)-Pyrrolyl-3 |
| 8 | N-(3'-$CH_3O$-$C_6H_4$)-Pyrrolyl-3 |
| 9 | N-(2'-$CH_3O$-$C_6H_4$)-Pyrrolyl-3 |
| 10 | N-(4'-$NO_2$-$C_6H_4$)-Pyrrolyl-3 |
| 11 | N-(3'-$NO_2$-$C_6H_4$)-Pyrrolyl-3 |
| 12 | N-(2'-$NO_2$-$C_6H_4$)-Pyrrolyl-3 |
| 13 | N-(4'-CN-$C_6H_4$)-Pyrrolyl-3 |
| 14 | N-(3'-CN-$C_6H_4$)-Pyrrolyl-3 |
| 15 | N-(2'-CN-$C_6H_4$)-Pyrrolyl-3 |
| 16 | N-(4'-Cl-$C_6H_4$)-Pyrrolyl-3 |
| 17 | N-(3'-Cl-$C_6H_4$)-Pyrrolyl-3 |
| 18 | N-(2'-Cl-$C_6H_4$)-Pyrrolyl-3 |
| 19 | Pyrrolyl-2 |
| 20 | N-$CH_3$-Pyrrolyl-2 |
| 21 | N-$C_6H_5$-Pyrrolyl-2 |
| 22 | N-(4'-$CH_3$-$C_6H_4$)-Pyrrolyl-2 |
| 23 | N-(3'-$CH_3$-$C_6H_4$)-Pyrrolyl-2 |
| 24 | N-(2'-$CH_3$-$C_6H_4$)-Pyrrolyl-2 |
| 25 | N-(4'-$CH_3O$-$C_6H_4$)-Pyrrolyl-2 |

| Nummer | A |
|---|---|
| 26 | N-(3'-$CH_3O$-$C_6H_4$)-Pyrrolyl-2 |
| 27 | N-(2'-$CH_3O$-$C_6H_4$)-Pyrrolyl-2 |
| 28 | N-(4'-$NO_2$-$C_6H_4$)-Pyrrolyl-2 |
| 29 | N-(3'-$NO_2$-$C_6H_4$)-Pyrrolyl-2 |
| 30 | N-(2'-$NO_2$-$C_6H_4$)-Pyrrolyl-2 |
| 31 | N-(4'-CN-$C_6H_4$)-Pyrrolyl-2 |
| 32 | N-(3'-CN-$C_6H_4$)-Pyrrolyl-2 |
| 33 | N-(2'-CN-$C_6H_4$)-Pyrrolyl-2 |
| 34 | N-(4'-Cl-$C_6H_4$)-Pyrrolyl-2 |
| 35 | N-(3'-Cl-$C_6H_4$)-Pyrrolyl-2 |
| 36 | N-(2'-Cl-$C_6H_4$)-Pyrrolyl-2 |
| 37 | Furyl-2 |
| 38 | 5-$CH_3$-Furyl-2 |
| 39 | 5-$C_6H_5$-Furyl-2 |
| 40 | 5-(4'-$CH_3$-$C_6H_4$)-Furyl-2 |
| 41 | 5-(3'-$CH_3$-$C_6H_4$)-Furyl-2 |
| 42 | 5-(2'-$CH_3$-$C_6H_4$)-Furyl-2 |
| 43 | 5-(4'-$CH_3O$-$C_6H_4$)-Furyl-2 |
| 44 | 5-(3'-$CH_3O$-$C_6H_4$)-Furyl-2 |
| 45 | 5-(2'-$CH_3O$-$C_6H_4$)-Furyl-2 |
| 46 | 5-(4'-$NO_2$-$C_6H_4$)-Furyl-2 |
| 47 | 5-(3'-$NO_2$-$C_6H_4$)-Furyl-2 |
| 48 | 5-(2'-$NO_2$-$C_6H_4$)-Furyl-2 |
| 49 | 5-(4'-CN-$C_6H_4$)-Furyl-2 |
| 50 | 5-(3'-CN-$C_6H_4$)-Furyl-2 |
| 51 | 5-(2'-CN-$C_6H_4$)-Furyl-2 |
| 52 | 5-(4'-Cl-$C_6H_4$)-Furyl-2 |
| 53 | 5-(3'-Cl-$C_6H_4$)-Furyl-2 |
| 54 | 5-(2'-Cl-$C_6H_4$)-Furyl-2 |
| 55 | 4-$CH_3$-Furyl-2 |
| 56 | 4-$C_6H_5$-Furyl-2 |
| 57 | 4-(4'-$CH_3$-$C_6H_4$)-Furyl-2 |
| 58 | 4-(3'-$CH_3$-$C_6H_4$)-Furyl-2 |
| 59 | 4-(2'-$CH_3$-$C_6H_4$)-Furyl-2 |
| 60 | 4-(4'-$CH_3O$-$C_6H_4$)-Furyl-2 |
| 61 | 4-(3'-$CH_3O$-$C_6H_4$)-Furyl-2 |

| Nummer | A |
|---|---|
| 62 | 4-(2'-$CH_3O$-$C_6H_4$)-Furyl-2 |
| 63 | 4-(4'-$NO_2$-$C_6H_4$)-Furyl-2 |
| 64 | 4-(3'-$NO_2$-$C_6H_4$)-Furyl-2 |
| 65 | 4-(2'-$NO_2$-$C_6H_4$)-Furyl-2 |
| 66 | 4-(4'-CN-$C_6H_4$)-Furyl-2 |
| 67 | 4-(3'-CN-$C_6H_4$)-Furyl-2 |
| 68 | 4-(2'-CN-$C_6H_4$)-Furyl-2 |
| 69 | 4-(4'-Cl-$C_6H_4$)-Furyl-2 |
| 70 | 4-(3'-Cl-$C_6H_4$)-Furyl-2 |
| 71 | 4-(2'-Cl-$C_6H_4$)-Furyl-2 |
| 72 | Thienyl-2 |
| 73 | 5-$CH_3$-Thienyl-2 |
| 74 | 5-$C_6H_5$-Thienyl-2 |
| 75 | 5-(4'-$CH_3$-$C_6H_4$)-Thienyl-2 |
| 76 | 5-(3'-$CH_3$-$C_6H_4$)-Thienyl-2 |
| 77 | 5-(2'-$CH_3$-$C_6H_4$)-Thienyl-2 |
| 78 | 5-(4'-$CH_3O$-$C_6H_4$)-Thienyl-2 |
| 79 | 5-(3'-$CH_3O$-$C_6H_4$)-Thienyl-2 |
| 80 | 5-(2'-$CH_3O$-$C_6H_4$)-Thienyl-2 |
| 81 | 5-(4'-$NO_2$-$C_6H_4$)-Thienyl-2 |
| 82 | 5-(3'-$NO_2$-$C_6H_4$)-Thienyl-2 |
| 83 | 5-(2'-$NO_2$-$C_6H_4$)-Thienyl-2 |
| 84 | 5-(4'-CN-$C_6H_4$)-Thienyl-2 |
| 85 | 5-(3'-CN-$C_6H_4$)-Thienyl-2 |
| 86 | 5-(2'-CN-$C_6H_4$)-Thienyl-2 |
| 87 | 5-(4'-Cl-$C_6H_4$)-Thienyl-2 |
| 88 | 5-(3'-Cl-$C_6H_4$)-Thienyl-2 |
| 89 | 5-(2'-Cl-$C_6H_4$)-Thienyl-2 |
| 90 | 4-$CH_3$-Thienyl-2 |
| 91 | 4-$C_6H_5$-Thienyl-2 |
| 92 | 4-(4'-$CH_3$-$C_6H_4$)-Thienyl-2 |
| 93 | 4-(3'-$CH_3$-$C_6H_4$)-Thienyl-2 |
| 94 | 4-(2'-$CH_3$-$C_6H_4$)-Thienyl-2 |
| 95 | 4-(4'-$CH_3O$-$C_6H_4$)-Thienyl-2 |
| 96 | 4-(3'-$CH_3O$-$C_6H_4$)-Thienyl-2 |
| 97 | 4-(2'-$CH_3O$-$C_6H_4$)-Thienyl-2 |

| Nummer | A |
|---|---|
| 98 | 4-(4'-$NO_2$-$C_6H_4$)-Thienyl-2 |
| 99 | 4-(3'-$NO_2$-$C_6H_4$)-Thienyl-2 |
| 100 | 4-(2'-$NO_2$-$C_6H_4$)-Thienyl-2 |
| 101 | 4-(4'-CN-$C_6H_4$)-Thienyl-2 |
| 102 | 4-(3'-CN-$C_6H_4$)-Thienyl-2 |
| 103 | 4-(2'-CN-$C_6H_4$)-Thienyl-2 |
| 104 | 4-(4'-Cl-$C_6H_4$)-Thienyl-2 |
| 105 | 4-(3'-Cl-$C_6H_4$)-Thienyl-2 |
| 106 | 4-(2'-Cl-$C_6H_4$)-Thienyl-2 |
| 107 | Thienyl-3 |
| 108 | 5-$CH_3$-Thienyl-3 |
| 109 | 5-$C_6H_5$-Thienyl-3 |
| 110 | 5-(4'-$CH_3$-$C_6H_4$)-Thienyl-3 |
| 111 | 5-(3'-$CH_3$-$C_6H_4$)-Thienyl-3 |
| 112 | 5-(2'-$CH_3$-$C_6H_4$)-Thienyl-3 |
| 113 | 5-(4'-$CH_3O$-$C_6H_4$)-Thienyl-3 |
| 114 | 5-(3'-$CH_3O$-$C_6H_4$)-Thienyl-3 |
| 115 | 5-(2'-$CH_3O$-$C_6H_4$)-Thienyl-3 |
| 116 | 5-(4'-$NO_2$-$C_6H_4$)-Thienyl-3 |
| 117 | 5-(3'-$NO_2$-$C_6H_4$)-Thienyl-3 |
| 118 | 5-(2'-$NO_2$-$C_6H_4$)-Thienyl-3 |
| 119 | 5-(4'-CN-$C_6H_4$)-Thienyl-3 |
| 120 | 5-(3'-CN-$C_6H_4$)-Thienyl-3 |
| 121 | 5-(2'-CN-$C_6H_4$)-Thienyl-3 |
| 122 | 5-(4'-Cl-$C_6H_4$)-Thienyl-3 |
| 123 | 5-(3'-Cl-$C_6H_4$)-Thienyl-3 |
| 124 | 5-(2'-Cl-$C_6H_4$)-Thienyl-3 |
| 125 | Pyrazolyl-4 |
| 126 | N-$CH_3$-Pyrazolyl-4 |
| 127 | N-$C_6H_5$-Pyrazolyl-4 |
| 128 | N-(4'-$CH_3$-$C_6H_4$)-Pyrazolyl-4 |
| 129 | N-(3'-$CH_3$-$C_6H_4$)-Pyrazolyl-4 |
| 130 | N-(2'-$CH_3$-$C_6H_4$)-Pyrazolyl-4 |
| 131 | N-(4'-$CH_3O$-$C_6H_4$)-Pyrazolyl-4 |
| 132 | N-(3'-$CH_3O$-$C_6H_4$)-Pyrazolyl-4 |
| 133 | N-(2'-$CH_3O$-$C_6H_4$)-Pyrazolyl-4 |

| Nummer | A |
|---|---|
| 134 | N-(4'-$NO_2$-$C_6H_4$)-Pyrazolyl-4 |
| 135 | N-(3'-$NO_2$-$C_6H_4$)-Pyrazolyl-4 |
| 136 | N-(2'-$NO_2$-$C_6H_4$)-Pyrazolyl-4 |
| 137 | N-(4'-CN-$C_6H_4$)-Pyrazolyl-4 |
| 138 | N-(3'-CN-$C_6H_4$)-Pyrazolyl-4 |
| 139 | N-(2'-CN-$C_6H_4$)-Pyrazolyl-4 |
| 140 | N-(4'-Cl-$C_6H_4$)-Pyrazolyl-4 |
| 141 | N-(3'-Cl-$C_6H_4$)-Pyrazolyl-4 |
| 142 | N-(2'-Cl-$C_6H_4$)-Pyrazolyl-4 |
| 143 | 3-$CH_3$-N-Methylpyrazolyl-4 |
| 144 | 3-$C_6H_5$-N-Methylpyrazolyl-4 |
| 145 | 3-(4'-$CH_3$-$C_6H_4$)-N-Methylpyrazolyl-4 |
| 146 | 3-(3'-$CH_3$-$C_6H_4$)-N-Methylpyrazolyl-4 |
| 147 | 3-(2'-$CH_3$-$C_6H_4$)-N-Methylpyrazolyl-4 |
| 148 | 3-(4'-$CH_3O$-$C_6H_4$)-N-Methylpyrazolyl-4 |
| 149 | 3-(3'-$CH_3O$-$C_6H_4$)-N-Methylpyrazolyl-4 |
| 150 | 3-(2'-$CH_3O$-$C_6H_4$)-N-Methylpyrazolyl-4 |
| 151 | 3-(4'-$NO_2$-$C_6H_4$)-N-Methylpyrazolyl-4 |
| 152 | 3-(3'-$NO_2$-$C_6H_4$)-N-Methylpyrazolyl-4 |
| 153 | 3-(2'-$NO_2$-$C_6H_4$)-N-Methylpyrazolyl-4 |
| 154 | 3-(4'-CN-$C_6H_4$)-N-Methylpyrazolyl-4 |
| 155 | 3-(3'-CN-$C_6H_4$)-N-Methylpyrazolyl-4 |
| 156 | 3-(2'-CN-$C_6H_4$)-N-Methylpyrazolyl-4 |
| 157 | 3-(4'-Cl-$C_6H_4$)-N-Methylpyrazolyl-4 |
| 158 | 3-(3'-Cl-$C_6H_4$)-N-Methylpyrazolyl-4 |
| 159 | 3-(2'-Cl-$C_6H_4$)-N-Methylpyrazolyl-4 |
| 160 | Isoxazolyl-5 |
| 161 | 3-$CH_3$-Isoxazolyl-5 |
| 162 | 3-$C_6H_5$-Isoxazolyl-5 |
| 163 | 3-(4'-$CH_3$-$C_6H_4$)-Isoxazolyl-5 |
| 164 | 3-(3'-$CH_3$-$C_6H_4$)-Isoxazolyl-5 |
| 165 | 3-(2'-$CH_3$-$C_6H_4$)-Isoxazolyl-5 |
| 166 | 3-(4'-$CH_3O$-$C_6H_4$)-Isoxazolyl-5 |
| 167 | 3-(3'-$CH_3O$-$C_6H_4$)-Isoxazolyl-5 |
| 168 | 3-(2'-$CH_3O$-$C_6H_4$)-Isoxazolyl-5 |
| 169 | 3-(4'-$NO_2$-$C_6H_4$)-Isoxazolyl-5 |

| Nummer | A |
|---|---|
| 170 | 3-(3'-$NO_2$-$C_6H_4$)-Isoxazolyl-5 |
| 171 | 3-(2'-$NO_2$-$C_6H_4$)-Isoxazolyl-5 |
| 172 | 3-(4'-CN-$C_6H_4$)-Isoxazolyl-5 |
| 173 | 3-(3'-CN-$C_6H_4$)-Isoxazolyl-5 |
| 174 | 3-(2'-CN-$C_6H_4$)-Isoxazolyl-5 |
| 175 | 3-(4'-Cl-$C_6H_4$)-Isoxazolyl-5 |
| 176 | 3-(3'-Cl-$C_6H_4$)-Isoxazolyl-5 |
| 177 | 3-(2'-Cl-$C_6H_4$)-Isoxazolyl-5 |
| 178 | 4-Chlorisoxazolyl-5 |
| 179 | 3-$CH_3$-4-Chlorisoxazolyl-5 |
| 180 | 3-$C_6H_5$-4-Chlorisoxazolyl-5 |
| 181 | 3-(4'-$CH_3$-$C_6H_4$)-4-Chlorisoxazolyl-5 |
| 182 | 3-(3'-$CH_3$-$C_6H_4$)-4-Chlorisoxazolyl-5 |
| 183 | 3-(2'-$CH_3$-$C_6H_4$)-4-Chlorisoxazolyl-5 |
| 184 | 3-(4'-$CH_3O$-$C_6H_4$)-4-Chlorisoxazolyl-5 |
| 185 | 3-(3'-$CH_3O$-$C_6H_4$)-4-Chlorisoxazolyl-5 |
| 186 | 3-(2'-$CH_3O$-$C_6H_4$)-4-Chlorisoxazolyl-5 |
| 187 | 3-(4'-$NO_2$-$C_6H_4$)-4-Chlorisoxazolyl-5 |
| 188 | 3-(3'-$NO_2$-$C_6H_4$)-4-Chlorisoxazolyl-5 |
| 189 | 3-(2'-$NO_2$-$C_6H_4$)-4-Chlorisoxazolyl-5 |
| 190 | 3-(4'-CN-$C_6H_4$)-4-Chlorisoxazolyl-5 |
| 191 | 3-(3'-CN-$C_6H_4$)-4-Chlorisoxazolyl-5 |
| 192 | 3-(2'-CN-$C_6H_4$)-4-Chlorisoxazolyl-5 |
| 193 | 3-(4'-Cl-$C_6H_4$)-4-Chlorisoxazolyl-5 |
| 194 | 3-(3'-Cl-$C_6H_4$)-4-Chlorisoxazolyl-5 |
| 195 | 3-(2'-Cl-$C_6H_4$)-4-Chlorisoxazolyl-5 |
| 196 | Isoxazolyl-3 |
| 197 | 5-$CH_3$-Isoxazolyl-3 |
| 198 | 5-$C_6H_5$-Isoxazolyl-3 |
| 199 | 5-(4'-$CH_3$-$C_6H_4$)-Isoxazolyl-3 |
| 200 | 5-(3'-$CH_3$-$C_6H_4$)-Isoxazolyl-3 |
| 201 | 5-(2'-$CH_3$-$C_6H_4$)-Isoxazolyl-3 |
| 202 | 5-(4'-$CH_3O$-$C_6H_4$)-Isoxazolyl-3 |
| 203 | 5-(3'-$CH_3O$-$C_6H_4$)-Isoxazolyl-3 |
| 204 | 5-(2'-$CH_3O$-$C_6H_4$)-Isoxazolyl-3 |
| 205 | 5-(4'-$NO_2$-$C_6H_4$)-Isoxazolyl-3 |

| Nummer | A |
|---|---|
| 206 | 5-(3'-$NO_2$-$C_6H_4$)-Isoxazolyl-3 |
| 207 | 5-(2'-$NO_2$-$C_6H_4$)-Isoxazolyl-3 |
| 208 | 5-(4'-CN-$C_6H_4$)-Isoxazolyl-3 |
| 209 | 5-(3'-CN-$C_6H_4$)-Isoxazolyl-3 |
| 210 | 5-(2'-CN-$C_6H_4$)-Isoxazolyl-3 |
| 211 | 5-(4'-Cl-$C_6H_4$)-Isoxazolyl-3 |
| 212 | 5-(3'-Cl-$C_6H_4$)-Isoxazolyl-3 |
| 213 | 5-(2'-Cl-$C_6H_4$)-Isoxazolyl-3 |
| 214 | Isothiazolyl-5 |
| 215 | 3-$CH_3$-Isothiazolyl-5 |
| 216 | 3-$C_6H_5$-Isothiazolyl-5 |
| 217 | 3-(4'-$CH_3$-$C_6H_4$)-Isothiazolyl-5 |
| 218 | 3-(3'-$CH_3$-$C_6H_4$)-Isothiazolyl-5 |
| 219 | 3-(2'-$CH_3$-$C_6H_4$)-Isothiazolyl-5 |
| 220 | 3-(4'-$CH_3O$-$C_6H_4$)-Isothiazolyl-5 |
| 221 | 3-(3'-$CH_3O$-$C_6H_4$)-Isothiazolyl-5 |
| 222 | 3-(2'-$CH_3O$-$C_6H_4$)-Isothiazolyl-5 |
| 223 | 3-(4'-$NO_2$-$C_6H_4$)-Isothiazolyl-5 |
| 224 | 3-(3'-$NO_2$-$C_6H_4$)-Isothiazolyl-5 |
| 225 | 3-(2'-$NO_2$-$C_6H_4$)-Isothiazolyl-5 |
| 226 | 3-(4'-CN-$C_6H_4$)-Isothiazolyl-5 |
| 227 | 3-(3'-CN-$C_6H_4$)-Isothiazolyl-5 |
| 228 | 3-(2'-CN-$C_6H_4$)-Isothiazolyl-5 |
| 229 | 3-(4'-Cl-$C_6H_4$)-Isothiazolyl-5 |
| 230 | 3-(3'-Cl-$C_6H_4$)-Isothiazolyl-5 |
| 231 | 3-(2'-Cl-$C_6H_4$)-Isothiazolyl-5 |
| 232 | Oxazolyl-4 |
| 233 | 2-$CH_3$-Oxazolyl-4 |
| 234 | 2-$C_6H_5$-Oxazolyl-4 |
| 235 | 2-(4'-$CH_3$-$C_6H_4$)-Oxazolyl-4 |
| 236 | 2-(3'-$CH_3$-$C_6H_4$)-Oxazolyl-4 |
| 237 | 2-(2'-$CH_3$-$C_6H_4$)-Oxazolyl-4 |
| 238 | 2-(4'-$CH_3O$-$C_6H_4$)-Oxazolyl-4 |
| 239 | 2-(3'-$CH_3O$-$C_6H_4$)-Oxazolyl-4 |
| 240 | 2-(2'-$CH_3O$-$C_6H_4$)-Oxazolyl-4 |
| 241 | 2-(4'-$NO_2$-$C_6H_4$)-Oxazolyl-4 |

| Nummer | A |
|---|---|
| 242 | 2-(3'-$NO_2$-$C_6H_4$)-Oxazolyl-4 |
| 243 | 2-(2'-$NO_2$-$C_6H_4$)-Oxazolyl-4 |
| 244 | 2-(4'-CN-$C_6H_4$)-Oxazolyl-4 |
| 245 | 2-(3'-CN-$C_6H_4$)-Oxazolyl-4 |
| 246 | 2-(2'-CN-$C_6H_4$)-Oxazolyl-4 |
| 247 | 2-(4'-Cl-$C_6H_4$)-Oxazolyl-4 |
| 248 | 2-(3'-Cl-$C_6H_4$)-Oxazolyl-4 |
| 249 | 2-(2'-Cl-$C_6H_4$)-Oxazolyl-4 |
| 250 | Thiazolyl-4 |
| 251 | 2-$CH_3$-Thiazolyl-4 |
| 252 | 2-$C_6H_5$-Thiazolyl-4 |
| 253 | 2-(4'-$CH_3$-$C_6H_4$)-Thiazolyl-4 |
| 254 | 2-(3'-$CH_3$-$C_6H_4$)-Thiazolyl-4 |
| 255 | 2-(2'-$CH_3$-$C_6H_4$)-Thiazolyl-4 |
| 256 | 2-(4'-$CH_3O$-$C_6H_4$)-Thiazolyl-4 |
| 267 | 2-(3'-$CH_3O$-$C_6H_4$)-Thiazolyl-4 |
| 258 | 2-(2'-$CH_3O$-$C_6H_4$)-Thiazolyl-4 |
| 259 | 2-(4'-$NO_2$-$C_6H_4$)-Thiazolyl-4 |
| 260 | 2-(3'-$NO_2$-$C_6H_4$)-Thiazolyl-4 |
| 261 | 2-(2'-$NO_2$-$C_6H_4$)-Thiazolyl-4 |
| 262 | 2-(4'-CN-$C_6H_4$)-Thiazolyl-4 |
| 263 | 2-(3'-CN-$C_6H_4$)-Thiazolyl-4 |
| 264 | 2-(2'-CN-$C_6H_4$)-Thiazolyl-4 |
| 265 | 2-(4'-Cl-$C_6H_4$)-Thiazolyl-4 |
| 266 | 2-(3'-Cl-$C_6H_4$)-Thiazolyl-4 |
| 267 | 2-(2'-Cl-$C_6H_4$)-Thiazolyl-4 |
| 268 | N-$CH_3$-1,2,4-Triazolyl-5 |
| 269 | 3-$CH_3$-N-$CH_3$-1,2,4-Triazolyl-5 |
| 270 | 3-$C_6H_5$-N-$CH_3$-1,2,4-Triazolyl-5 |
| 271 | 3-(4'-$CH_3$-$C_6H_4$)-N-$CH_3$-1,2,4-Triazolyl-5 |
| 272 | 3-(3'-$CH_3$-$C_6H_4$)-N-$CH_3$-1,2,4-Triazolyl-5 |
| 273 | 3-(2'-$CH_3$-$C_6H_4$)-N-$CH_3$-1,2,4-Triazolyl-5 |
| 274 | 3-(4'-$CH_3O$-$C_6H_4$)-N-$CH_3$-1,2,4-Triazolyl-5 |
| 275 | 3-(3'-$CH_3O$-$C_6H_4$)-N-$CH_3$-1,2,4-Triazolyl-5 |
| 276 | 3-(2'-$CH_3O$-$C_6H_4$)-N-$CH_3$-1,2,4-Triazolyl-5 |
| 277 | 3-(4'-$NO_2$-$C_6H_4$)-N-$CH_3$-1,2,4-Triazolyl-5 |

| Nummer | A |
|---|---|
| 278 | 3-(3'-$NO_2$-$C_6H_4$)-N-$CH_3$-1,2,4-Triazolyl-5 |
| 279 | 3-(2'-$NO_2$-$C_6H_4$)-N-$CH_3$-1,2,4-Triazolyl-5 |
| 280 | 3-(4'-CN-$C_6H_4$)-N-$CH_3$-1,2,4-Triazolyl-5 |
| 281 | 3-(3'-CN-$C_6H_4$)-N-$CH_3$-1,2,4-Triazolyl-5 |
| 282 | 3-(2'-CN-$C_6H_4$)-N-$CH_3$-1,2,4-Triazolyl-5 |
| 283 | 3-(4'-Cl-$C_6H_4$)-N-$CH_3$-1,2,4-Triazolyl-5 |
| 284 | 3-(3'-Cl-$C_6H_4$)-N-$CH_3$-1,2,4-Triazolyl-5 |
| 285 | 3-(2'-Cl-$C_6H_4$)-N-$CH_3$-1,2,4-Triazolyl-5 |
| 286 | 1,3,4-Oxadiazolyl-2 |
| 287 | 5-$CH_3$-1,3,4-Oxadiazolyl-2 |
| 288 | 5-$C_6H_5$-1,3,4-Oxadiazolyl-2 |
| 289 | 5-(4'-$CH_3$-$C_6H_4$)-1,3,4-Oxadiazolyl-2 |
| 290 | 5-(3'-$CH_3$-$C_6H_4$)-1,3,4-Oxadiazolyl-2 |
| 291 | 5-(2'-$CH_3$-$C_6H_4$)-1,3,4-Oxadiazolyl-2 |
| 292 | 5-(4'-$CH_3O$-$C_6H_4$)-1,3,4-Oxadiazolyl-2 |
| 293 | 5-(3'-$CH_3O$-$C_6H_4$)-1,3,4-Oxadiazolyl-2 |
| 294 | 5-(2'-$CH_3O$-$C_6H_4$)-1,3,4-Oxadiazolyl-2 |
| 295 | 5-(4'-$NO_2$-$C_6H_4$)-1,3,4-Oxadiazolyl-2 |
| 296 | 5-(3'-$NO_2$-$C_6H_4$)-1,3,4-Oxadiazolyl-2 |
| 297 | 5-(2'-$NO_2$-$C_6H_4$)-1,3,4-Oxadiazolyl-2 |
| 298 | 5-(4'-CN-$C_6H_4$)-1,3,4-Oxadiazolyl-2 |
| 299 | 5-(3'-CN-$C_6H_4$)-1,3,4-Oxadiazolyl-2 |
| 300 | 5-(2'-CN-$C_6H_4$)-1,3,4-Oxadiazolyl-2 |
| 301 | 5-(4'-Cl-$C_6H_4$)-1,3,4-Oxadiazolyl-2 |
| 302 | 5-(3'-Cl-$C_6H_4$)-1,3,4-Oxadiazolyl-2 |
| 303 | 5-(2'-Cl-$C_6H_4$)-1,3,4-Oxadiazolyl-2 |
| 304 | 1,2,4-Oxadiazolyl-3 |
| 305 | 5-$CH_3$-1,2,4-Oxadiazolyl-3 |
| 306 | 5-$C_6H_5$-1,2,4-Oxadiazolyl-3 |
| 307 | 5-(4'-$CH_3$-$C_6H_4$)-1,2,4-Oxadiazolyl-3 |
| 308 | 5-(3'-$CH_3$-$C_6H_4$)-1,2,4-Oxadiazolyl-3 |
| 309 | 5-(2'-$CH_3$-$C_6H_4$)-1,2,4-Oxadiazolyl-3 |
| 310 | 5-(4'-$CH_3O$-$C_6H_4$)-1,2,4-Oxadiazolyl-3 |
| 311 | 5-(3'-$CH_3O$-$C_6H_4$)-1,2,4-Oxadiazolyl-3 |
| 312 | 5-(2'-$CH_3O$-$C_6H_4$)-1,2,4-Oxadiazolyl-3 |
| 313 | 5-(4'-$NO_2$-$C_6H_4$)-1,2,4-Oxadiazolyl-3 |

| Nummer | A |
|---|---|
| 314 | 5-(3'-$NO_2$-$C_6H_4$)-1,2,4-Oxadiazolyl-3 |
| 315 | 5-(2'-$NO_2$-$C_6H_4$)-1,2,4-Oxadiazolyl-3 |
| 316 | 5-(4'-CN-$C_6H_4$)-1,2,4-Oxadiazolyl-3 |
| 317 | 5-(3'-CN-$C_6H_4$)-1,2,4-Oxadiazolyl-3 |
| 318 | 5-(2'-CN-$C_6H_4$)-1,2,4-Oxadiazolyl-3 |
| 319 | 5-(4'-Cl-$C_6H_4$)-1,2,4-Oxadiazolyl-3 |
| 320 | 5-(3'-Cl-$C_6H_4$)-1,2,4-Oxadiazolyl-3 |
| 321 | 5-(2'-Cl-$C_6H_4$)-1,2,4-Oxadiazolyl-3 |
| 322 | 1,2,4-Oxadiazolyl-5 |
| 323 | 3-$CH_3$-1,2,4-Oxadiazolyl-5 |
| 324 | 3-$C_6H_5$-1,2,4-Oxadiazolyl-5 |
| 325 | 3-(4'-$CH_3$-$C_6H_4$)-1,2,4-Oxadiazolyl-5 |
| 326 | 3-(3'-$CH_3$-$C_6H_4$)-1,2,4-Oxadiazolyl-5 |
| 327 | 3-(2'-$CH_3$-$C_6H_4$)-1,2,4-Oxadiazolyl-5 |
| 328 | 3-(4'-$CH_3O$-$C_6H_4$)-1,2,4-Oxadiazolyl-5 |
| 329 | 3-(3'-$CH_3O$-$C_6H_4$)-1,2,4-Oxadiazolyl-5 |
| 330 | 3-(2'-$CH_3O$-$C_6H_4$)-1,2,4-Oxadiazolyl-5 |
| 331 | 3-(4'-$NO_2$-$C_6H_4$)-1,2,4-Oxadiazolyl-5 |
| 332 | 3-(3'-$NO_2$-$C_6H_4$)-1,2,4-Oxadiazolyl-5 |
| 333 | 3-(2'-$NO_2$-$C_6H_4$)-1,2,4-Oxadiazolyl-5 |
| 334 | 3-(4'-CN-$C_6H_4$)-1,2,4-Oxadiazolyl-5 |
| 335 | 3-(3'-CN-$C_6H_4$)-1,2,4-Oxadiazolyl-5 |
| 336 | 3-(2'-CN-$C_6H_4$)-1,2,4-Oxadiazolyl-5 |
| 337 | 3-(4'-Cl-$C_6H_4$)-1,2,4-Oxadiazolyl-5 |
| 338 | 3-(3'-Cl-$C_6H_4$)-1,2,4-Oxadiazolyl-5 |
| 339 | 3-(2'-Cl-$C_6H_4$)-1,2,4-Oxadiazolyl-5 |
| 340 | 1,2,4-Thiadiazolyl-3 |
| 341 | 5-$CH_3$-1,2,4-Thiadiazolyl-3 |
| 342 | 5-$C_6H_5$-1,2,4-Thiadiazolyl-3 |
| 343 | 5-(4'-$CH_3$-$C_6H_4$)-1,2,4-Thiadiazolyl-3 |
| 344 | 5-(3'-$CH_3$-$C_6H_4$)-1,2,4-Thiadiazolyl-3 |
| 345 | 5-(2'-$CH_3$-$C_6H_4$)-1,2,4-Thiadiazolyl-3 |
| 346 | 5-(4'-$CH_3O$-$C_6H_4$)-1,2,4-Thiadiazolyl-3 |
| 347 | 5-(3'-$CH_3O$-$C_6H_4$)-1,2,4-Thiadiazolyl-3 |
| 348 | 5-(2'-$CH_3O$-$C_6H_4$)-1,2,4-Thiadiazolyl-3 |
| 349 | 5-(4'-$NO_2$-$C_6H_4$)-1,2,4-Thiadiazolyl-3 |

| Nummer | A |
|---|---|
| 350 | 5-(3'-$NO_2$-$C_6H_4$)-1,2,4-Thiadiazolyl-3 |
| 351 | 5-(2'-$NO_2$-$C_6H_4$)-1,2,4-Thiadiazolyl-3 |
| 352 | 5-(4'-CN-$C_6H_4$)-1,2,4-Thiadiazolyl-3 |
| 353 | 5-(3'-CN-$C_6H_4$)-1,2,4-Thiadiazolyl-3 |
| 354 | 5-(2'-CN-$C_6H_4$)-1,2,4-Thiadiazolyl-3 |
| 355 | 5-(4'-Cl-$C_6H_4$)-1,2,4-Thiadiazolyl-3 |
| 356 | 5-(3'-Cl-$C_6H_4$)-1,2,4-Thiadiazolyl-3 |
| 357 | 5-(2'-Cl-$C_6H_4$)-1,2,4-Thiadiazolyl-3 |
| 358 | 1,3,4-Thiadiazolyl-2 |
| 359 | 5-$CH_3$-1,3,4-Thiadiazolyl-2 |
| 360 | 5-$C_6H_5$-1,3,4-Thiadiazolyl-2 |
| 361 | 5-(4'-$CH_3$-$C_6H_4$)-1,3,4-Thiadiazolyl-2 |
| 362 | 5-(3'-$CH_3$-$C_6H_4$)-1,3,4-Thiadiazolyl-2 |
| 363 | 5-(2'-$CH_3$-$C_6H_4$)-1,3,4-Thiadiazolyl-2 |
| 364 | 5-(4'-$CH_3O$-$C_6H_4$)-1,3,4-Thiadiazolyl-2 |
| 365 | 5-(3'-$CH_3O$-$C_6H_4$)-1,3,4-Thiadiazolyl-2 |
| 366 | 5-(2'-$CH_3O$-$C_6H_4$)-1,3,4-Thiadiazolyl-2 |
| 367 | 5-(4'-$NO_2$-$C_6H_4$)-1,3,4-Thiadiazolyl-2 |
| 368 | 5-(3'-$NO_2$-$C_6H_4$)-1,3,4-Thiadiazolyl-2 |
| 369 | 5-(2'-$NO_2$-$C_6H_4$)-1,3,4-Thiadiazolyl-2 |
| 370 | 5-(4'-CN-$C_6H_4$)-1,3,4-Thiadiazolyl-2 |
| 371 | 5-(3'-CN-$C_6H_4$)-1,3,4-Thiadiazolyl-2 |
| 372 | 5-(2'-CN-$C_6H_4$)-1,3,4-Thiadiazolyl-2 |
| 373 | 5-(4'-Cl-$C_6H_4$)-1,3,4-Thiadiazolyl-2 |
| 374 | 5-(3'-Cl-$C_6H_4$)-1,3,4-Thiadiazolyl-2 |
| 375 | 5-(2'-Cl-$C_6H_4$)-1,3,4-Thiadiazolyl-2 |
| 376 | Pyridinyl-2 |
| 377 | Pyridinyl-4 |
| 378 | Pyridazinyl-3 |
| 379 | Pyridazinyl-4 |
| 380 | Pyridazinyl-2 |
| 381 | Pyrimidinyl-4 |
| 382 | Pyrimidinyl-5 |
| 383 | Pyrimidinyl-2 |
| 384 | Pyridinyl-3 |

Tabelle 3

| Nr. | Verbindung | IR ( $cm^{-1}$ ) oder $^1H$-NMR ( ppm ) | F p |
|---|---|---|---|
| 1 | 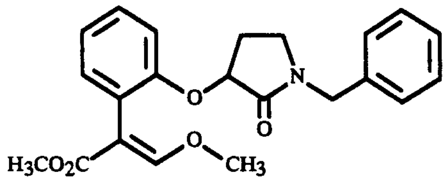 | 7,5 (s, 1 H); 3,8 (s, 3 H); 3,65 (s, 3 H) | |
| 2 | 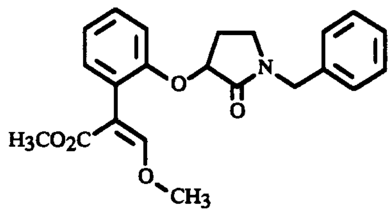 | 6,6 (s, 1 H); 3,9 (s, 3 H); 3,65 (s, 3 H) | |
| 3 | 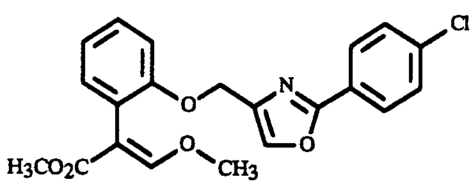 | | 104 |
| 4 | 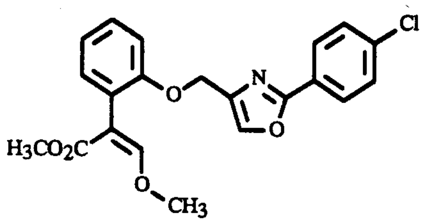 | 6,6 (s, 1 H); 3,9 (s, 3 H); 3,7 (s, 3 H) | |
| 5 | 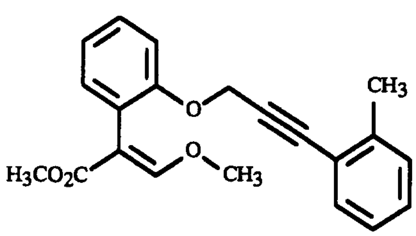 | | 80 |

| Nr. | Verbindung | IR ( $cm^{-1}$ ) oder $^{1}H$-NMR ( ppm ) | Fp |
|---|---|---|---|
| 6 | 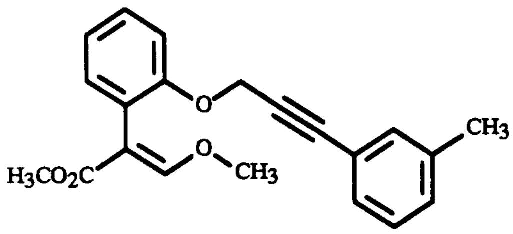 | | 76 |
| 7 | 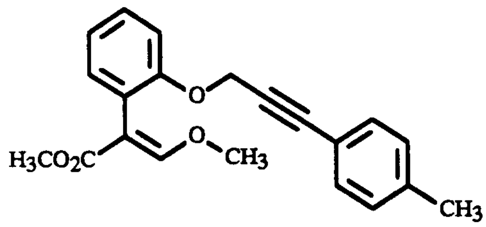 | | 85 |
| 8 | 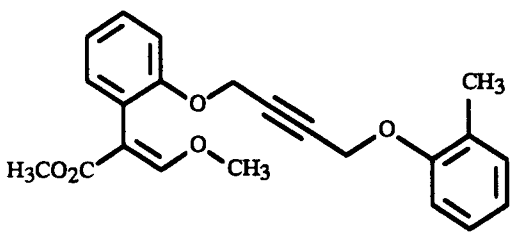 | 7,5 (s, 1 H); 3,8 (s, 3 H); 3,7 (s, 3 H) | |
| 9 | 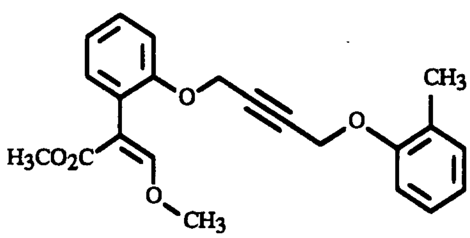 | 6,55 (s, 1 H); 3,9 (s, 3 H); 3,7 (s, 3 H) | |
| 10 | 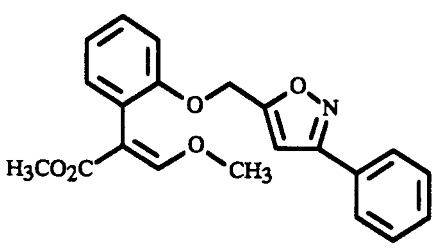 | 7,5 (s, 1 H); 3,8 (s, 3 H); 3,7 (s, 3 H) | |

| Nr. | Verbindung | IR ( $cm^{-1}$ ) oder $^1H$-NMR ( ppm ) | Fp |
|---|---|---|---|
| 11 | 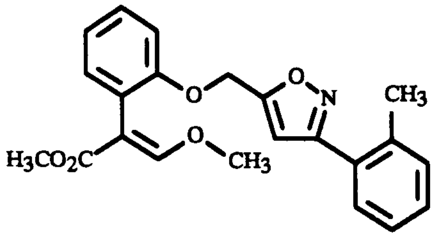 | | 67 |
| 12 | 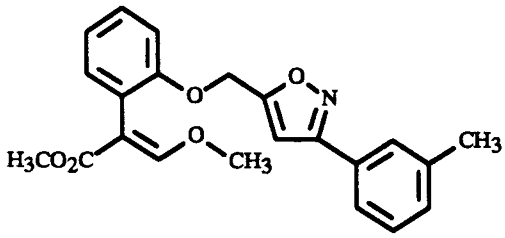 | | 99 |
| 13 | 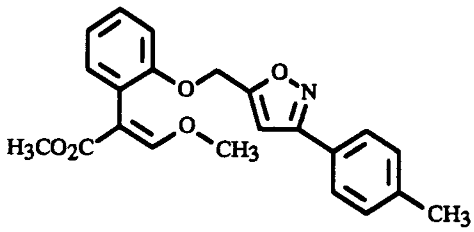 | | 82 |
| 14 | 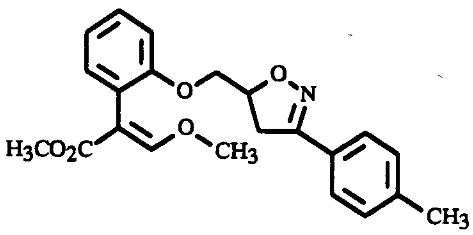 | | 125 |
| 15 | 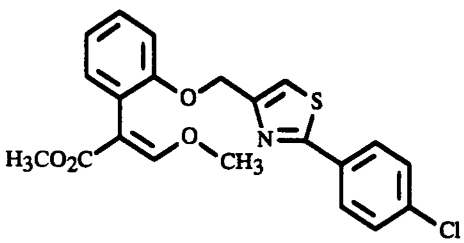 | | 127 |

| Nr. | Verbindung | IR ( $cm^{-1}$ ) oder $^1H$-NMR ( ppm ) | Fp |
|---|---|---|---|
| 16 | 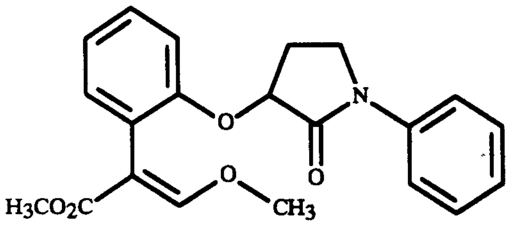 | 7,5 (s, 1 H); 3,8 (s, 3 H); 3,7 (s, 3 H) | |
| 17 | 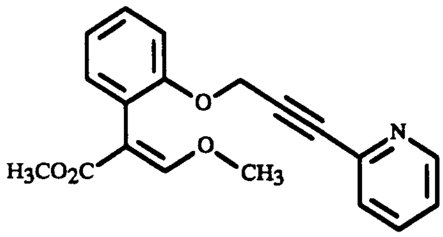 | 7,5 (s, 1 H); 3,8 (s, 3 H); 3,7 (s, 3 H) | |
| 18 | 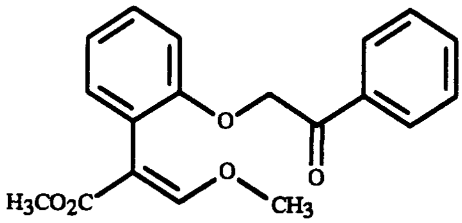 | | 76 |
| 19 | 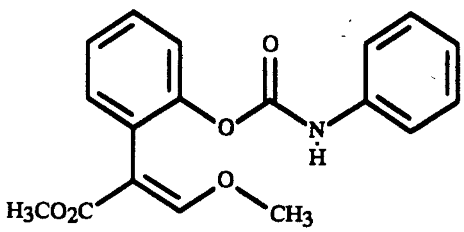 | | 179 |
| 20 | 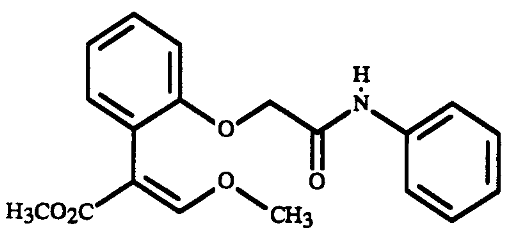 | | 116 |

| Nr. | Verbindung | IR ( $cm^{-1}$ ) oder $^1H$-NMR ( ppm ) | F p |
|---|---|---|---|
| 21 | 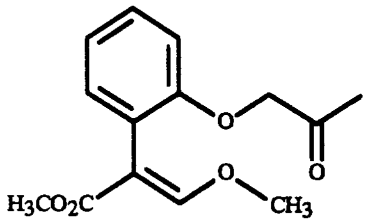 | | 91 |
| 22 | 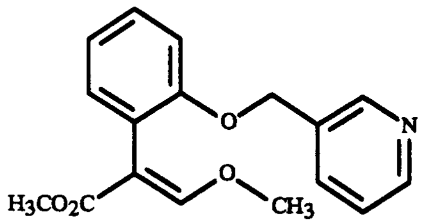 | 7,5 (s, 1 H); 3,75 (s, 3 H); 3,65 (s, 3 H) | |
| 23 | 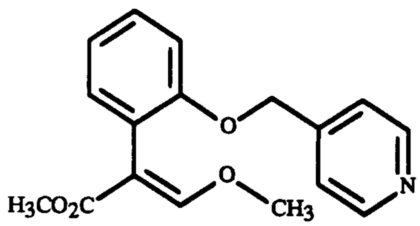 | | 98 |
| 24 | 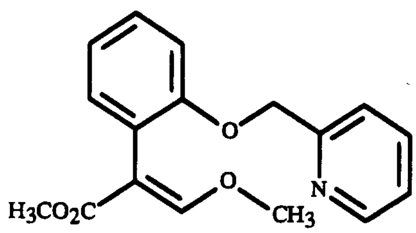 | 7,55 (s, 1 H); 3,8 (s, 3 H); 3,65 (s, 3 H) | |
| 25 | 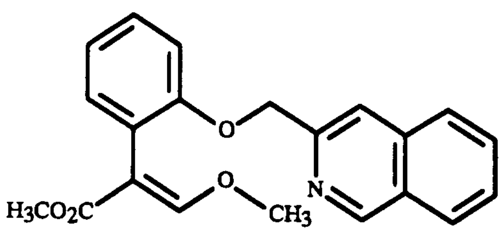 | | 125 |

| Nr. | Verbindung | IR ( $cm^{-1}$ ) oder $^1H$-NMR ( ppm ) | F p |
|---|---|---|---|
| 26 | 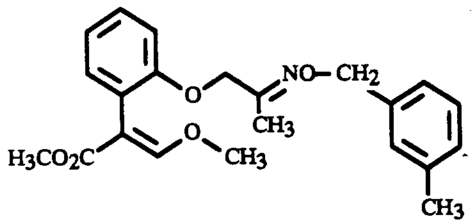 | 2 Isomere (ca. 3 : 1): 7,5 (2 s, 1 H); 3,8 (2 s, 3 H); 3,65 (2 s, 3 H) | |
| 27 | 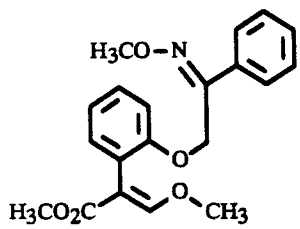 | | 74 |
| 28 | 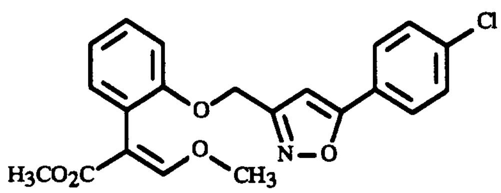 | | 111 |
| 29 | 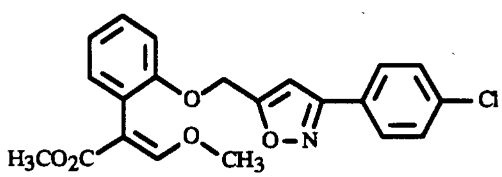 | 7,55 (s, 1 H); 3,8 (s, 3 H); 3,7 (s, 3 H) | |
| 30 | 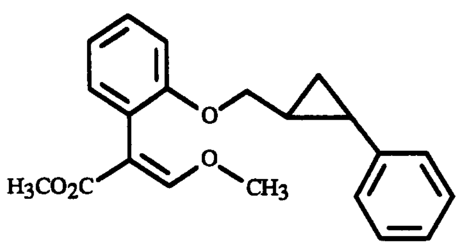 | 7,5 (s, 1 H); 3,75 (s, 3 H); 3,65 (s, 3 H) | |

| Nr. | Verbindung | IR ( $cm^{-1}$ ) oder $^{1}H$-NMR ( ppm ) | F p |
|---|---|---|---|
| 31 | 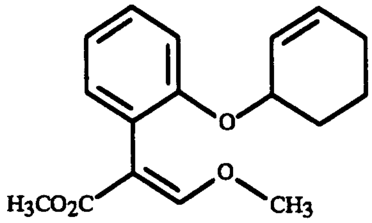 | 7,45 (s, 1 H); 3,8 (s, 3 H); 3,65 (s, 3 H) | |
| 32 | 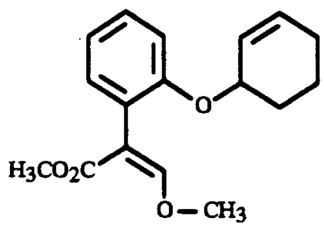 | 6,5 (s, 1 H); 3,85 (s, 3H); 3,65 (s, 3 H) | |
| 33 | 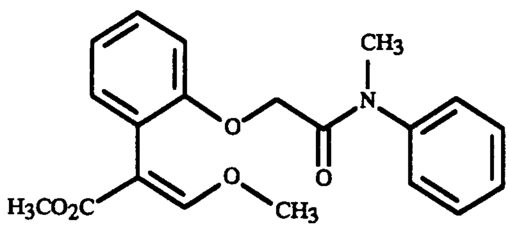 | | 123 |
| 34 | 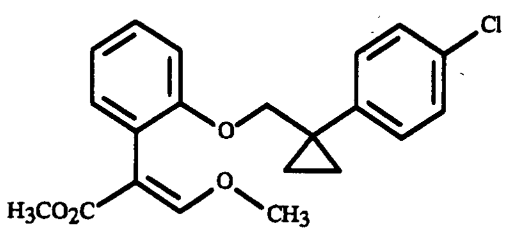 | 7,45 (s, 1 H); 3,75 (s, 3 H); 3,6 (s, 3 H) | |
| 35 | 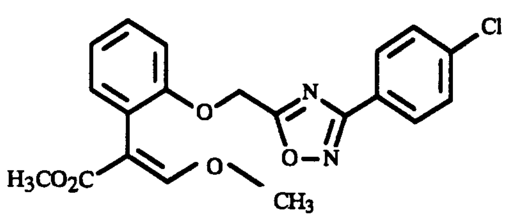 | 7,55 (s, 1 H); 3,85 (s, 3 H); 3,7 (s, 3 H) | 96 |

| Nr. | Verbindung | IR ( $cm^{-1}$ ) oder $^1H$-NMR ( ppm ) | F p |
|---|---|---|---|
| 36 | 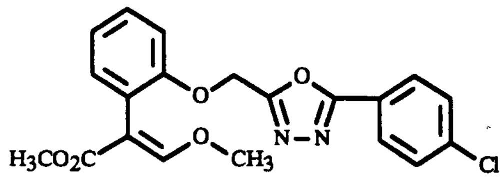 | | 129 |
| 37 | 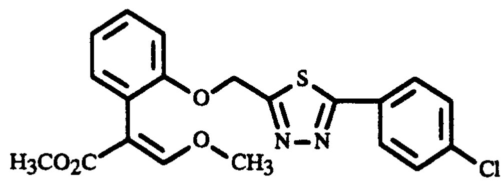 | | 129 |
| 38 | 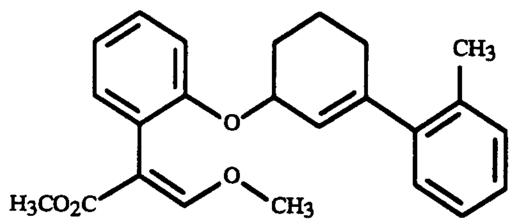 | 7,45 (s, 1 H); 3,75 (s, 3 H); 3,65 (s, 3 H) | |
| 39 | 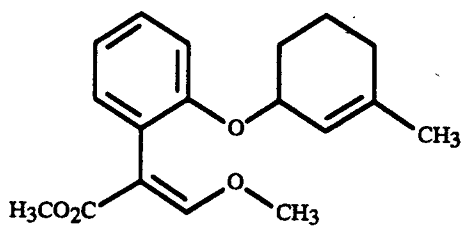 | 7,45 (s, 1 H); 3,8 (s, 3 H); 3,65 (s, 3 H) | |
| 40 | 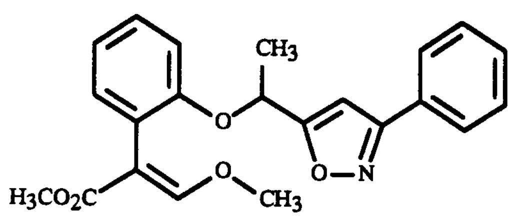 | 7,5 (s, 1 H); 3,8 (s, 3 H); 3,7 (s, 3 H) | |

| Nr. | Verbindung | IR ( $cm^{-1}$ ) oder $^{1}H$-NMR ( ppm ) | F p |
|---|---|---|---|
| 41 | 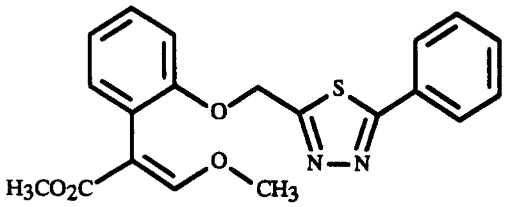 | | 106 |
| 42 | 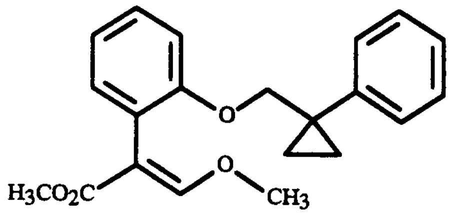 | | 78 |
| 43 | 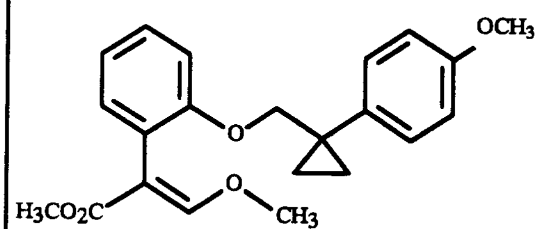 | 7,45 (s, 1H); 3,75 (s, 3H); 3,7 (s, 3H); 3,6 (s, 3H) | |
| 44 | 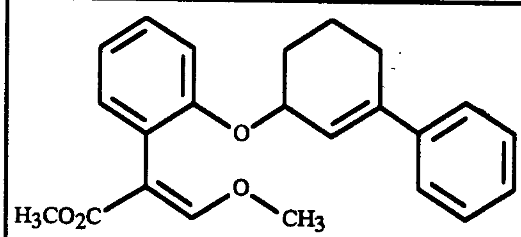 | 7,46 (s, 1H); 3,75 (s, 3H); 3,6 (s, 3H) | |
| 45 | 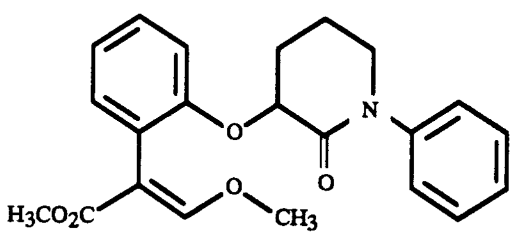 | | 126 |

| Nr. | Verbindung | IR ( $cm^{-1}$ ) oder $^{1}H$-NMR ( ppm ) | F p |
|---|---|---|---|
| 46 | 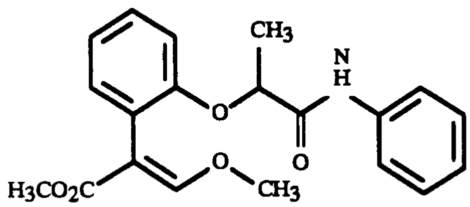 | 7,6 (s, 1H); 3,9 (s, 3H); 3,8 (s, 3H) | |
| 47 | 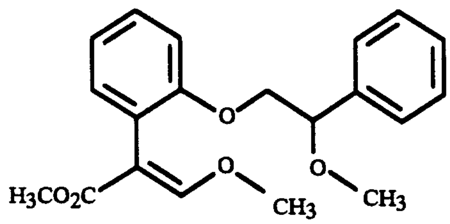 | 7,45 (s, 1H); 3,75 (s, 3H); 3,65 (s, 3H); 3,35 (s, 3H) | |
| 48 | 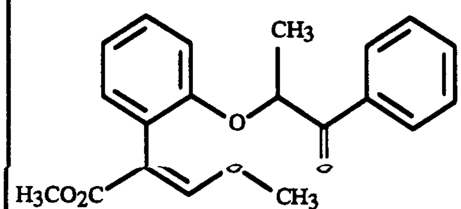 | | 98 |
| 49 | 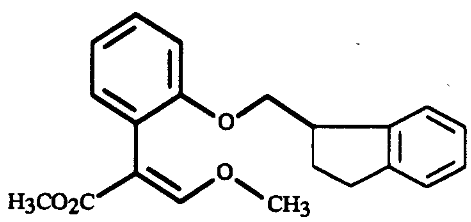 | 7,46 (s, 1H); 3,75 (s, 3H); 3,6 (s, 3H) | |
| 50 | 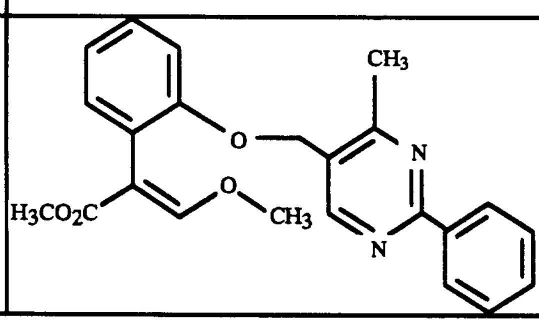 | | 162 |

| Nr. | Verbindung | IR ( $cm^{-1}$ ) oder $^1H$-NMR ( ppm ) | F p |
|---|---|---|---|
| 51 | 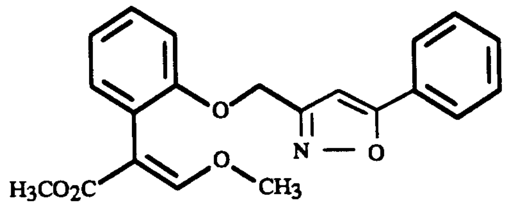 | | 81 |
| 52 | 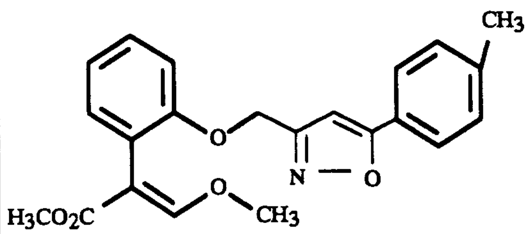 | | 110 |
| 53 | 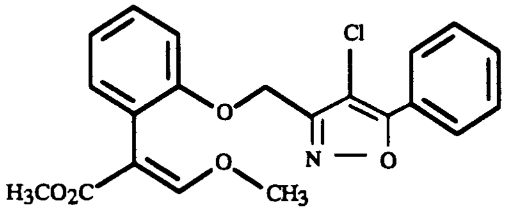 | | 134 |
| 54 | 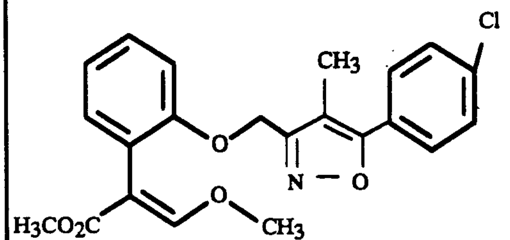 | | 138 |
| 55 | 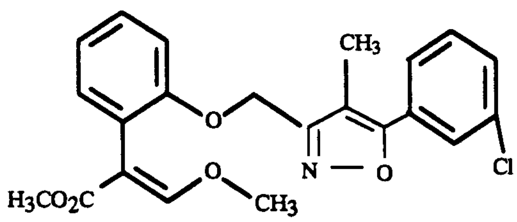 | | 119 |

| Nr. | Verbindung | IR ( $cm^{-1}$ ) oder $^1H$-NMR ( ppm ) | Fp |
|---|---|---|---|
| 56 | 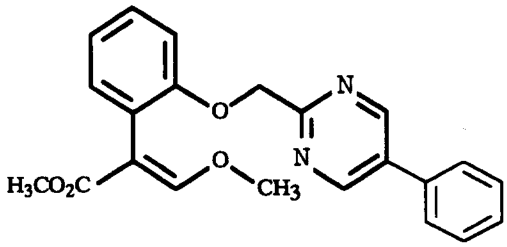 | | 117 |
| 57 | 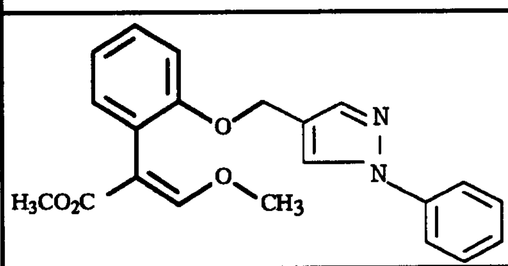 | | 97 |
| 58 | 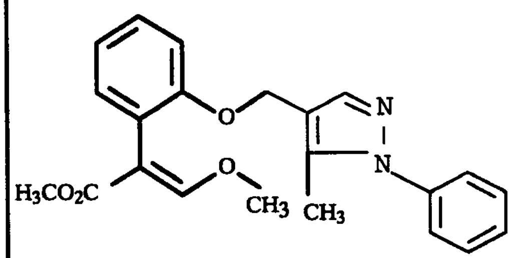 | 7,6 (s, 1H); 3,8 (s, 3H); 3,65 (s, 3H) | |
| 59 | 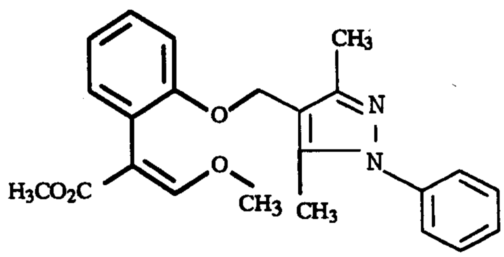 | | 128 |
| 60 | 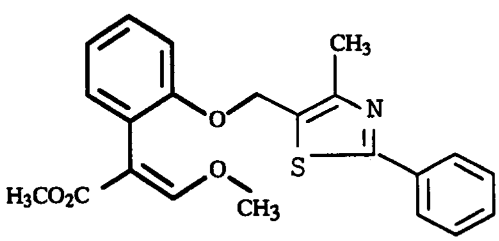 | | 136 |

| Nr. | Verbindung | IR ( $cm^{-1}$ ) oder $^1H$-NMR ( ppm ) | F p |
|---|---|---|---|
| 61 | 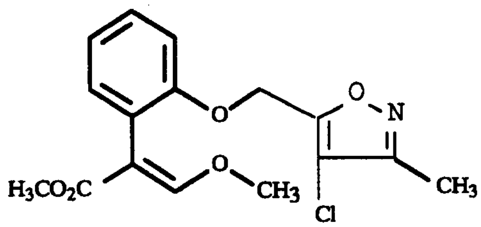 | | 82 |
| 62 | 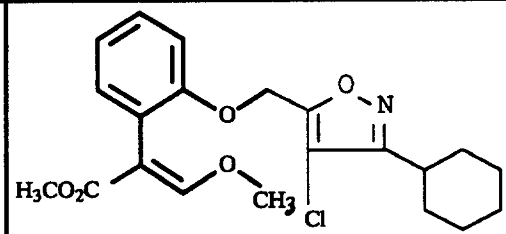 | | 115 |
| 63 | 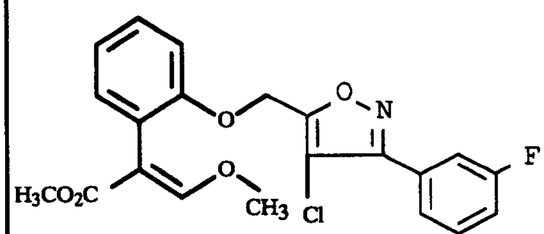 | | 93 |
| 64 | 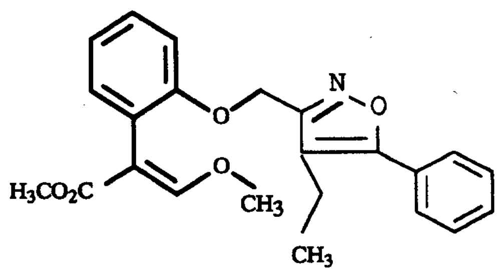 | | 103 |
| 65 | 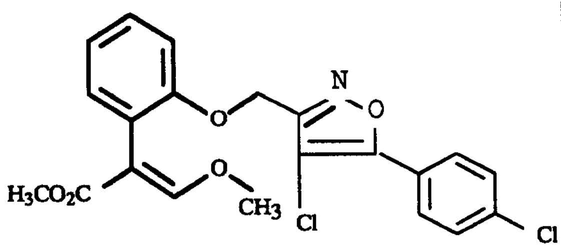 | | 144 |

| Nr. | Verbindung | IR ( $cm^{-1}$ ) oder $^1$H-NMR ( ppm ) | F p |
|---|---|---|---|
| 66 | 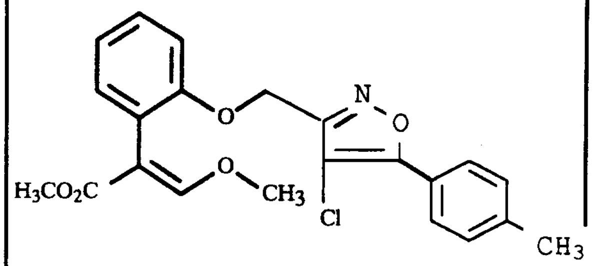 | | 135 |
| 67 | 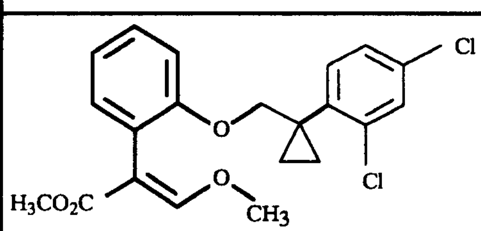 | 7,4 (s,1H); 3,7 (s,3H); 3,6 (s, 3H) | |
| 68 | 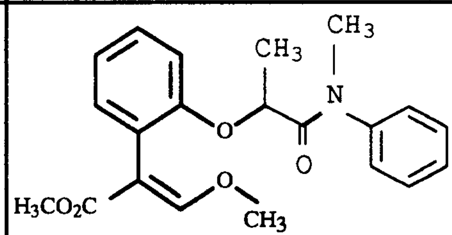 | 7,4 (s,3H); 3,8 (s,3H); 3,7(s,3H); | |
| 69 | 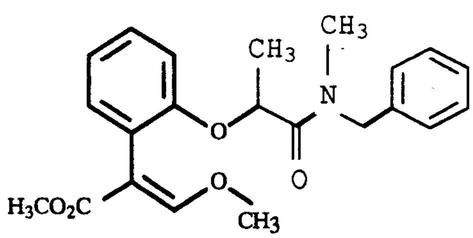 | | 108 |
| 70 | 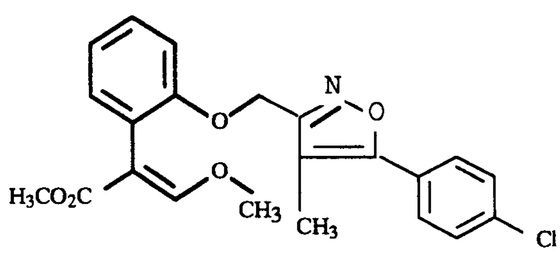 | | 119 |

| Nr. | Verbindung | IR ( $cm^{-1}$ ) oder $^1H$-NMR ( ppm ) | F p |
|---|---|---|---|
| 71 | 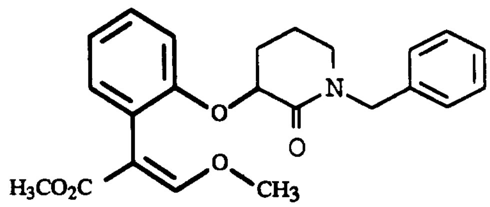 | 7,5 (s,1H); 3,8 (s,3H); 3,7 (s,3H)) | |
| 72 | 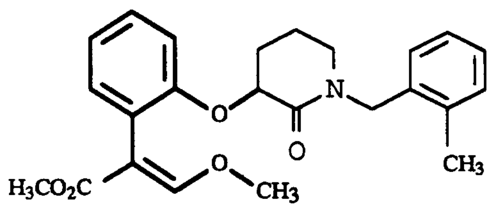 | 7,45 (s,1H); 3,8 (s,3H); 3,7 (s, 3H) | |
| 73 | 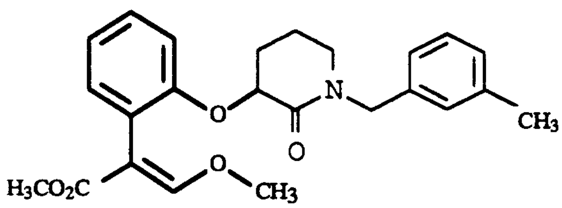 | 7,45 (s,1H); 3,75 (s,3H); 3,65 (s,3H); | |
| 74 | 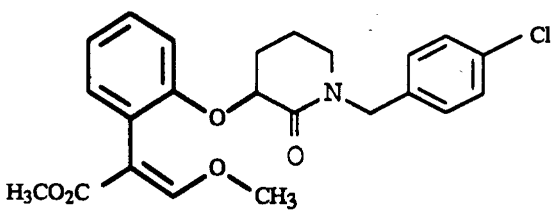 | 7,5 (s,1H); 3,8 (s,3H); 3,65 (s,3H); | |
| 75 | 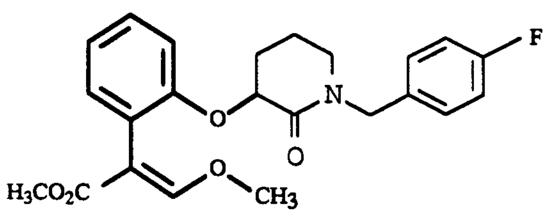 | | 116 |

| Nr. | Verbindung | IR ( $cm^{-1}$ ) oder $^1H$-NMR ( ppm ) | Fp |
|---|---|---|---|
| 76 | 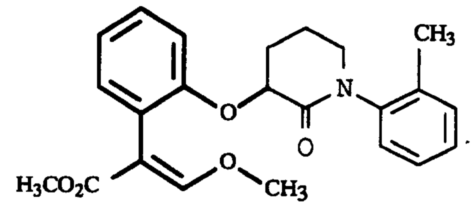 | | 118 |
| 77 | 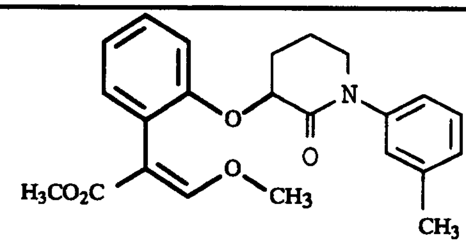 | 7,5 (s,1H); 3,8 (s,3H); 3,7 (s, 3H) | |
| 78 | 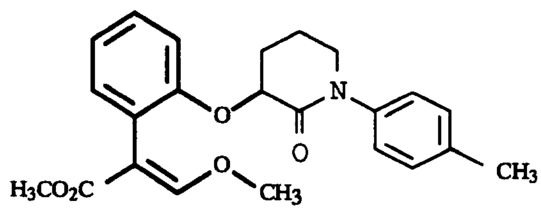 | | 105 |
| 79 | 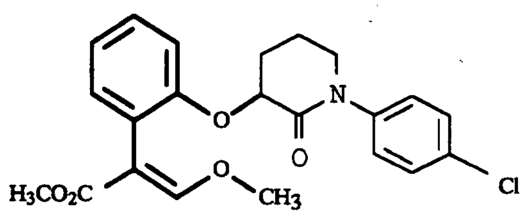 | 7,5 (s,1H); 3,8 (s,3H); 3,7 (s,3H); | |

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide)

und synthetische Gesteinsmehle (z.B. hochdisperse Kieselsäure, Silikate); Emulgiermittel wie nichtionogene und anionische Emulgatoren (z.B. Polyoxethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyde, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Tragerstoffe.

Beispiele für solche Zubereitungen sind:

- I. eine Lösung aus 90 Gew.-Teilen der Verbindung Nr. 1 aus Tabelle 3 und 10 Gew.-Teilen N-Methyl-a-pyrrolidon, die zur Anwendung in Form kleinster Tropfen geeignet ist;
- II. eine Mischung aus 20 Gew.-Teilen der Verbindung Nr. 2 aus Tabelle 3, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
- III. eine wäßrige Dispersion aus 20 Gew.-Teilen der Verbindung Nr. 3 aus Tabelle 3, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
- IV. eine wäßrige Dispersion aus 20 Gew.-Teilen der Verbindung Nr. 4 aus Tabelle 3, 25 Gew.-Teilen Cyclohexanol, 65 Gew.-Teilen einer Mineralölfraktion vom Siedepunkt 210 bis 280'C und 10 Gew.-Teilen des Anlagerungsproduktes von 40 mol Ethylenoxid an 1 mol Ricinusöl;
- V. eine in einer Hammermühle vermahlene Mischung aus 80 Gew.-Teilen der Verbindung Nr. 5 aus Tabelle 3, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
- VI. eine innige Mischung aus 3 Gew.-Teilen der Verbindung Nr. 6 aus Tabelle 3 und 97 Gew.-Teilen feinteiligem Kaolin; dieses Stäubemittel enthält 3 Gew.-% Wirkstoff;
- VII. eine innige Mischung aus 30 Gew.-Teilen der Verbindung Nr. 7 aus Tabelle 3, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
- VIII. eine stabile wäßrige Dispersion aus 40 Gew.-Teilen der Verbindung Nr. 8 aus Tabelle 3, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
- IX. eine stabile ölige Dispersion aus 20 Gew.-Teilen der Verbindung Nr. 9 aus Tabelle 3, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Anwendungsbeispiele

Für die folgenden Anwendungsbeispiele wurde als Vergleichssubstanz die Verbindung 2-(2-Hydroxyphenyl)-3-methoxy-acrylsäuremethylester (A) - bekannt aus EP 251082 - verwendet.

Anwendungsbeispiel 1
Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola 8Rebensperonospora) infiziert. Danach wurden die Reben zunächst für 48 Stunden in einer wasserdampfgesättigten kammer bei 24°C und anschließend für 5 Tage in einem Gewächshaus bei Temperaturen zwischen 20 und 30°C aufgestellt. nach dieser Zeit wurden die Pflanzen zur Beschleunigung des Sporangienträgerausbruchs abermals für 16 Stunden in der feuchten Kammer aufgestellt. Dann erfolgte die

Beurteilung des Ausmaßes des Pilzausbruchs auf den Blattunterseiten.

Das Ergebnis des Versuchs zeigt, daß die Verbindungen aus Tabelle 3, Nr. 3, 4, 5, 6, 7, 8, 10, 11, 12, 13, 15, 18, 26, 28 und 29 bei der Anwendung als 250 ppm Wirkstoff enthaltende Spritzbrühe eine bessere fungizide Wirkung (90 %) zeigen als der bekannte Vergleichwirkstoff A (40 %).

Anwendungsbeispiel 2
Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22 °C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22 °C und 65 bis 70 % relativer Luftfeuchte aufgestellt. Nach 8 Tagen wurde das Ausmaß der Rostpilzentwicklung auf den Blättern ermittelt.

Das Ergebnis des Versuchs zeigt, daß die Wirkstoffe aus Tabelle 3, Nr. 7, 8, 10 und 31 bei der Anwendung als 250 ppm Wirkstoff enthaltende Spritzbrühe eine bessere fungizide Wirkung zeigten (95 %) als der bekannte Vergleichswirkstoff A (0 %).

**Patentansprüche**

**1.** Substituierte Acrylsäureester der Formel I

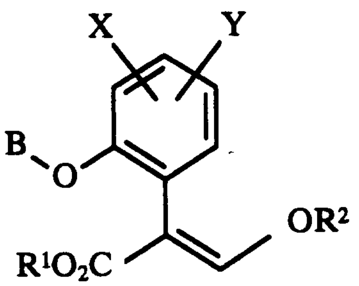

I

in der die Substituenten die folgende Bedeutung besitzen:

B bedeutet

Alkyl, das mit 1 - 4 gleichen oder verschiedenen Substituenten $R^3$ substituiert ist, Alkenyl, das mit 1 - 4 gleichen oder verschiedenen Substituenten $R^4$ substituiert ist, Alkinyl, das mit 1 - 4 gleichen oder verschiedenen Substituenten $R^5$ substituiert ist, Cycloalkyl, das mit 1 -4 gleichen oder verschiedenen Substituenten $R^6$ substituiert ist, Cycloalkenyl, das mit 1 - 4 gleichen oder verschiedenen Substituenten $R^7$ substituiert ist, Cycloalkinyl, das mit 1 - 4 gleichen oder verschiedenen Substituenten $R^8$ substituiert ist oder Heterocyclyl, das mit 1 - 4 gleichen oder verschiedenen Substituenten $R^9$ substituiert ist,

X und Y bedeuten unabhängig voneinander

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyl oximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy, oder Heterocyclylalkyloxy,

Stehen die Substituenten X und Y an benachbarten Kohlenstoffatomen, dann können sie zu einem ggf. substituierten aromatischen oder heteroaromatischen, alicyclischen oder heterocyclischen, partiell oder vollständig hydrierten Ring kondensiert sein,

$R^1$ und $R^2$ können ggf. substituiert sein, und bedeuten unabhängig voneinander Alkyl, Alkenyl, Alkinyl, Cycloalkyl oder Cycloalkenyl, $R^3$ und $R^4$ können ggf. substituiert sein mit 1 - 4 gleichen oder verschiedenen Substituenten $R^{10}$, und $R^3$ und $R^4$ bedeuten Nitro, Haloalkoxy, Alkoxy, Alkinyl, Cycloalkyl, Hetaryl, Heterocyclyl, Cycloalkenyl, Cycloalkinyl, Alkenyloxy, Alkinyloxy, Cycloalkyloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Hetarylalkyloxy, Heterocyclylalkyloxy, Alkinylalkenyl, Cycloalkylalkenyl, Cycloalkenylalkenyl, Cycloalkinylalkenyl, Hetarylalkenyl, Heterocyclylalkenyl, Alkoximinoalkyl, Alkenyloximinoalkyl, Alkinyloximinoalkyl, Cycloalkyloximinoalkyl, Cycloalkenyloximinoalkyl, Cycloalkinyloximinoalkyl, Aryloximinoalkyl, Hetaryloximinoalkyl, Heterocyclyloximinoalkyl, Alkoximinoalkenyl, Alkenyloximinoalkenyl, Alkinyloximinoalkenyl, Cycloalkyloximinoalkenyl, Cycloalkenyloximinoalkenyl, Cycloalkinyloximinoalkenyl, Aryloximinoalkenyl, Hetaryloximinoalkenyl oder Heterocyclyloximinoalkenyl,

$R^5$, $R^6$, $R^7$ und $R^8$ können ggf. substituiert sein mit 1 -4 gleichen oder verschiedenen Substituenten $R^{10}$, und $R^5$, $R^6$, $R^7$ und $R^8$ bedeuten

Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino oder Heterocyclylcarbonylamino,

$R^9$ kann ggf. substituiert sein, mit 1 - 4 gleichen oder verschiedenen Substituenten $R^{10}$, und $R^9$ bedeutet

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,

$R^{10}$ kann ggf. substituiert sein, mit 1 - 4 gleichen oder verschiedenen Substituenten $R^{11}$, und $R^{10}$ bedeutet

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,

$R^{11}$ kann ggf. substituiert sein und bedeutet

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy und die Saureadditionsprodukte und die Basenadditionsprodukte dieser Verbindungen.

**2.** Substituierte Acrylsäureester der Formel II

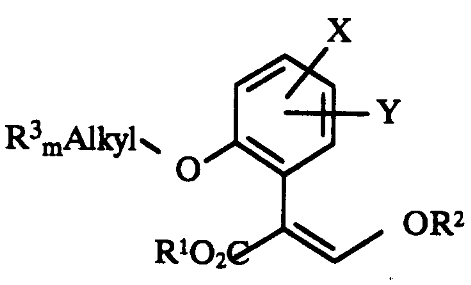

II

in der die Substituenten $R^1$, $R^2$, $R^3$, X, Y die in Anspruch 1 genannte Bedeutung besitzen und m 1, 2, 3 oder 4 ist.

**3.** Substituierte Acrylsäureester der Formel III

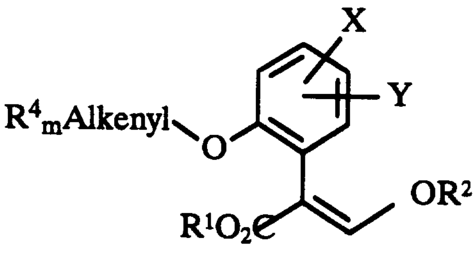

III

in der die Substituenten $R^1$, $R^2$, $R^4$, X, Y die in AnSpruch 1 genannte Bedeutung besitzen und m 1, 2, 3 oder 4 ist.

**4.** Substituierte Acrylsäureester der Formel IV

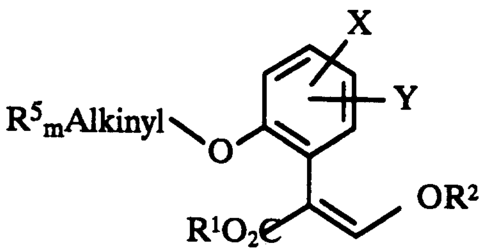

IV

in der die Substituenten $R^1$, $R^2$, $R^5$, X, Y die in Anspruch 1 genannte Bedeutung besitzen und m 1, 2, 3 oder 4 ist.

**5.** Substituierte Acrylsäureester der Formel V

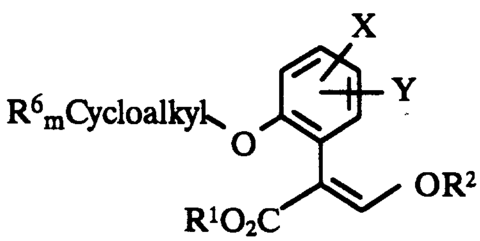

V

in der die Substituenten $R^1$, $R^2$, $R^6$, X, Y die in Anspruch 1 genannte Bedeutung besitzen und m 1, 2, 3 oder 4 ist.

**6.** Substituierte Acrylsäureester der Formel VI

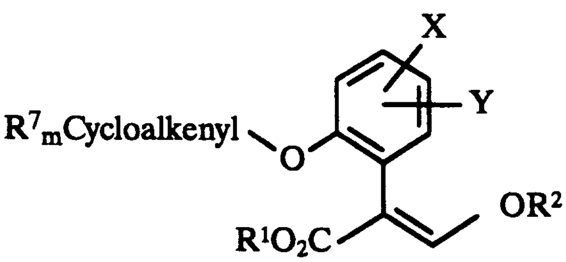

VI

in der die Substituenten $R^1$, $R^2$, $R^6$, X, Y die in Anspruch 1 genannte Bedeutung besitzen und m 1, 2, 3 oder 4 ist.

**7.** Substituierte Acrylsäureester der Formel VII

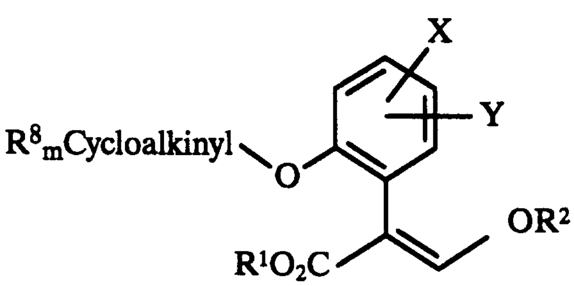

VII

in der die Substituenten $R^1$, $R^2$, $R^8$, X, Y die in Anspruch 1 genannte Bedeutung besitzen und m 1, 2, 3 oder 4 ist.

**8.** Substituierte Acrylsäureester der Formel VIII

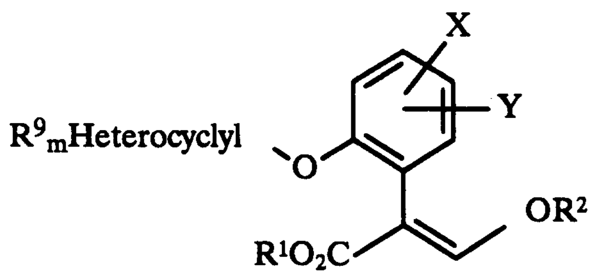

VIII

in der die Substituenten $R^1$, $R^2$, $R^9$, X, Y die in Anspruch 1 genannte Bedeutung besitzen und m 1, 2, 3 oder 4 ist.

**9.** Verbindung der Formel IX

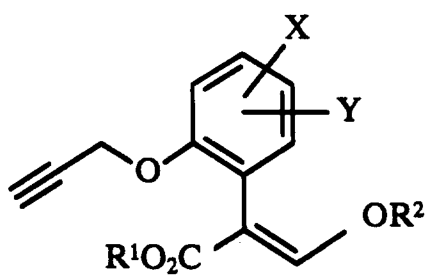

IX

in der $R^1$, $R^2$, X, Y die in Anspruch 1 genannte Bedeutung besitzen.

**10.** Verbindung der Formel X

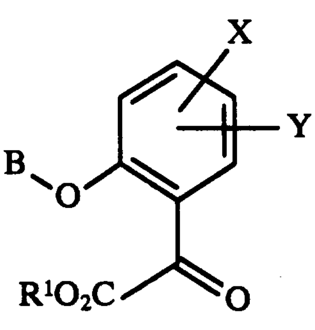

X

in der $R^1$, X, Y und B die in Anspruch 1 genannte Bedeutung besitzen.

**11.** Verbindung der Formel XI

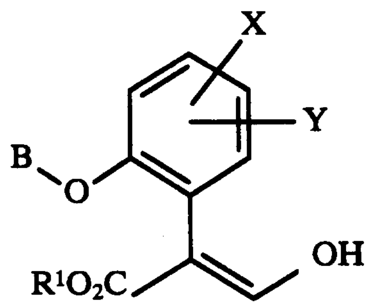

XI

in der $R^1$, X, Y und B die in Anspruch 1 genannte Bedeutung besitzen.

**12.** Verbindung der Formel XII

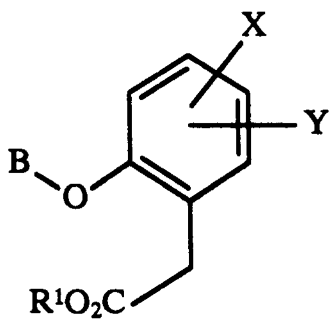

XII

in der $R^1$, X, Y und B die in Anspruch 1 genannte Bedeutung besitzen.

**13.** Verbindung der Formel XIII

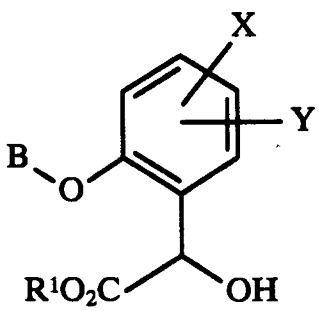

XIII

in der $R^1$, X, Y und B die in Anspruch 1 genannte Bedeutung besitzen.

**14.** Verbindung der Formel XIV

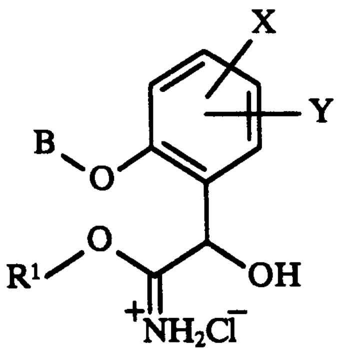

XIV

in der $R^1$, X, Y und B die in Anspruch 1 genannte Bedeutung besitzen.

**15.** Verbindung der Formel XV

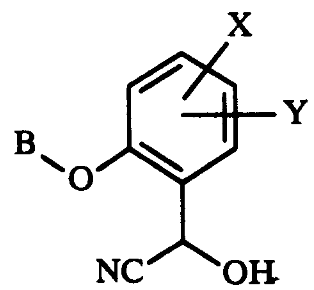

XV

in der $R^1$, X, Y und B die in Anspruch 1 genannte Bedeutung besitzen.

**16.** Verbindung der Formel XVI

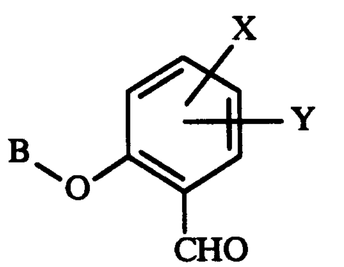

XVI

in der $R^1$, X, Y und B die in Anspruch 1 genannte Bedeutung besitzen.

**17.** Verfahren zur Herstellung von substituierten Acrylsäureestern der Formel I, gemäß Anspruch 1, dadurch gekennzeichnet, daß man einen substituierten Acrylsäureester der Formel XVII

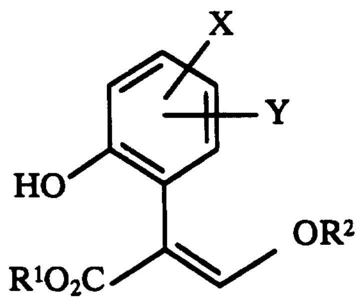

XVII

in der $R^1$, $R^2$, X und Y die in Anspruch 1 genannte Bedeutung besitzen, mit einem Alkylierungsmittel in einem komplexierenden Lösungsmittel, in Gegenwart einer Base, ggf. in Gegenwart eines Katalysators, oder in Gegenwart eines Molsiebs umsetzt.

**18.** Verfahren zur Herstellung von substituierten Acrylsäureestern der Formel XVIII

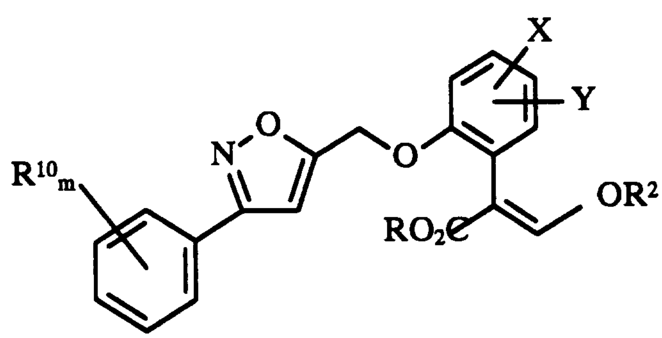

XVIII

in der $R^1$, $R^2$, $R^{10}$, X und Y die in Anspruch 1 genannte Bedeutung besitzen und m 1, 2, 3 oder 4 bedeutet, gekennzeichnet dadurch, daß man einen Propargylether der Formel IX

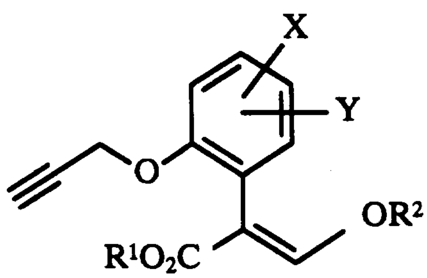

IX

in Gegenwart eines Aldoxims der Formel XIX

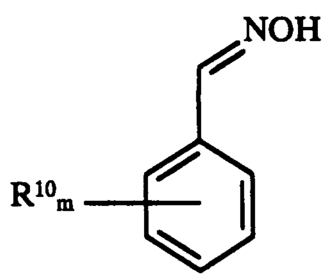

XIX

in der $R^{10}$ die in Anspruch 1 genannte Bedeutung besitzt und m 1, 2, 3 oder 4 bedeutet, mit NaOCl-Lösung umsetzt.

**19.** Verfahren zur Herstellung von substituierten Acrylsäureestern der Formel XX

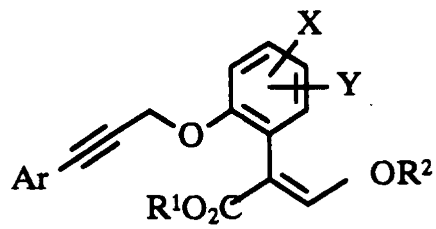

XX

in der $R^1$, $R^2$, X und Y die in Anspruch 1 genannte Bedeutung besitzen und Ar eine mit 1 - 4 Substituenten $R^{10}$ substituierte aromatische oder heteroaromatische Gruppe bedeutet, gekennzeichnet dadurch, daß man einen Propargylether der Formel IX in Gegenwart von Pd, Pd-II-acetat oder $PdCl_2$ , und Triphenylphosphosphin, einer organ. Base, und Kupfersalzen, mit Verbindungen der Formel Ar-Hal (Hal = J, Br) umsetzt.

**20.** Verfahren zur Herstellung von substituierten Acrylsäureestern der Formel I gemäß Anspruch 1 gekennzeichnet dadurch, daß man einen Ketoester der Formel X

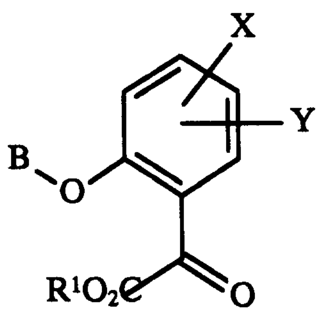

X

in einer Wittig-Reaktion mit $(C_6H_5)_3P^+\text{-}CH_2\text{-}O\text{-}CH_3$ Cl- umsetzt.

**21.** Verfahren zur Herstellung von substituierten Acrylsäureestern der Formel I gemäß Anspruch 1 gekennzeichnet dadurch, daß man einen substituierten Acrylsäureester der Formel XI

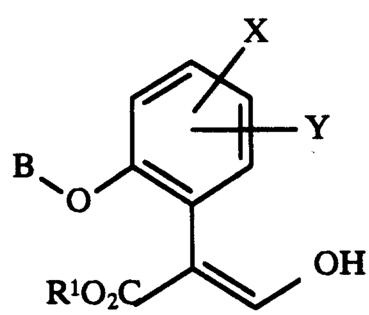

XI

in der $R^1$, X, Y und B die in Anspruch 1 genannte Bedeutung besitzen, unter alkalischen Bedingungen alkyliert.

**22.** Verfahren zur Herstellung von substituierten Acrylsäureestern der Formel XI gemäß Anspruch 11 gekennzeichnet dadurch, daß man einen Phenylessigester der Formel XII

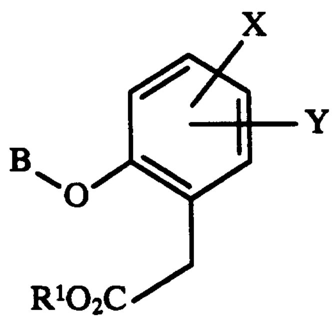

XII

in der $R^1$, X, Y und B die in Anspruch 1 genannte Bedeutung besitzen, unter alkalischen Bedingungen mit einem Ameisensäureester $HCO_2R^2$ umsetzt.

**23.** Verfahren zur Herstellung von Ketoestern der Formel X, gekennzeichnet dadurch, daß man Hydroxyester der Formel XII

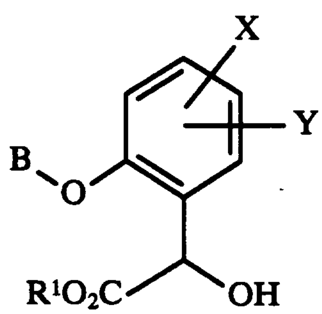

XIII

in der $R^1$, X, Y und B die in Anspruch 1 genannte Bedeutung besitzen, oxidiert.

**24.** Verfahren zur Herstellung von Hydroxyestern der Formel XIII, gekennzeichnet dadurch, daß man Imidoester-hydrochloride der Formel XIV

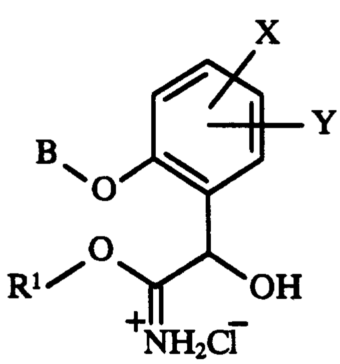

XIV

in der $R^1$, X, Y und B die in Anspruch 1 genannte Bedeutung besitzen, mit Wasser umsetzt.

**25.** Verfahren zur Herstellung von Imidoester-hydrochloriden der Formel XIV, gekennzeichnet dadurch, daß man Cyanhydrine der Formel XV

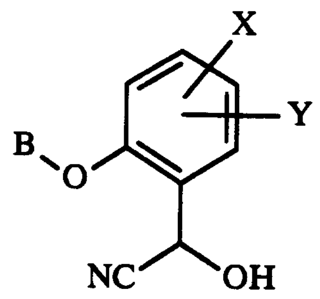

XV

in der X, Y und B die in Anspruch 1 genannte Bedeutung besitzen, in Gegenwart von Salzsäure mit Alkoholen $R^1$-OH umsetzt.

**26.** Verfahren zur Herstellung von Cyanhydrinen der Formel XV, gekennzeichnet dadurch, daß man Aldehyde der Formel XVI

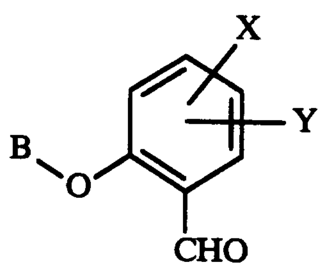

XVI

in der X, Y und B die in Anspruch 1 genannte Bedeutung besitzen, mit Blausäure umsetzt.

**27.** Verfahren zur Herstellung von substituierten Acrylsäureestern gemäß Formel I Anspruch I dadurch gekennzeichnet, daß man ein Phenol der Formel XVII

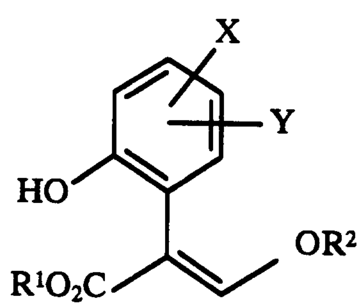

XVII

in der $R^1$, $R^2$, X und Y die in Anspruch angegebene Bedeutung besitzen, in einem Lösungsmittel, und in Gegenwart von Azodicarbonsäurediethylester und Triphenylphosphin, mit einem Alkohol der Formel B-OH, in der B die in Anspruch 1 genannte Bedeutung besitzt, umsetzt.

**28.** Fungizid, enthaltend einen inerten Trägerstoff und eine fungizid wirksame Menge eines substituierten Acrylsäureesters der Formel I

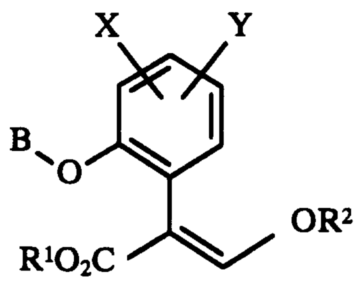

I

in der die Substituenten die folgende Bedeutung besitzen:

B bedeutet

Alkyl, das mit 1 - 4 gleichen oder verschiedenen Substituenten $R^3$ substituiert ist, Alkenyl, das mit 1 - 4 gleichen oder verschiedenen Substituenten $R^4$ substituiert ist, Alkinyl, das mit 1 - 4 gleichen oder verschiedenen Substituenten $R^5$ substituiert ist, Cycloalkyl, das mit 1 -4 gleichen oder verschiedenen Substituenten $R^6$ substituiert ist, Cycloalkenyl, das mit 1 - 4 gleichen oder verschiedenen Substituenten $R^7$ substituiert ist, Cycloalkinyl, das mit 1 - 4 gleichen oder verschiedenen Substituenten $R^8$ substituiert ist oder Heterocyclyl, das mit 1 - 4 gleichen oder verschiedenen Substituenten $R^9$ substituiert ist,

X und Y bedeuten unabhängig voneinander

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy, Stehen die Substituenten X und Y an benachbarten Kohlenstoffatomen, dann können sie zu einem ggf. substituierten aromatischen oder heteroaromatischen, alicyclischen oder heterocyclischen, partiell oder vollständig hydrierten Ring kondensiert sein,

$R^1$ und $R^2$ können ggf. substituiert sein, und bedeuten unabhängig voneinander Alkyl, Alkenyl, Alkinyl, Cycloalkyl oder Cycloalkenyl, $R^3$ und $R^4$ können ggf. substituiert sein mit 1 - 4 gleichen oder verschiedenen Substituenten $R^{10}$, und $R^3$ und $R^4$ bedeutenNitro, Haloalkoxy, Alkoxy, Alkinyl, Cycloalkyl, Hetaryl, Heterocyclyl, Cycloalkenyl, Cycloalkinyl, Alkenyloxy, Alkinyloxy, Cycloalkyloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Hetarylalkyloxy, Heterocyclylalkyloxy, Alkinylalkenyl, Cycloalkylalkenyl, Cycloalkenylalkenyl, Cycloalkinylalkenyl, Hetarylalkenyl, Heterocyclylalkenyl, Alkoximinoalkyl, Alkenyloximinoalkyl, Alkinyloximinoalkyl, Cycloalkyloximinoalkyl, Cycloalkenyloximinoalkyl, Cycloalkinyloximinoalkyl, Aryloximinoalkyl, Hetaryloximinoalkyl, Heterocyclyloximinoalkyl, Alkoximinoalkenyl, Alkenyloximinoalkenyl, Alkinyloximinoalkenyl, Cycloalkyloximinoalkenyl, Cycloalkenyloximinoalkenyl, Cycloalkinyloximinoalkenyl, Aryloximinoalkenyl, Hetaryloximinoalkenyl oder Heterocyclyloximinoalkenyl,

$R^5$, $R^6$, $R^7$ und $R^8$ können ggf. substituiert sein mit 1 -4 gleichen oder verschiedenen Substituenten $R^{10}$, und $R^5$, $R^6$, $R^7$ und $R^8$ bedeuten

Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino oder Heterocyclylcarbonylamino,

$R^9$ kann ggf. substituiert sein, mit 1 - 4 gleichen oder verschiedenen Substituenten $R^{10}$, und $R^9$ bedeutet

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,

$R^{10}$ kann ggf. substituiert sein, mit 1 - 4 gleichen oder verschiedenen Substituenten $R^{11}$, und $R^{10}$ bedeutet

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,

$R^{11}$ kann ggf. substituiert sein und bedeutet

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy oder

eines Säureadditionsproduktes oder Basenadditionsprodukte dieser Verbindung.

**29.** Verfahren zur Bekämpfung von Pilzen, dadurch gekennzeichnet, daß man die Pilze oder die vom Pilzbefall bdrohten Pflanzen, Saatgut, Materialien oder den Erdboden behandelt mit einer fungizid wirksamen Menge einer Verbindung der Formel I

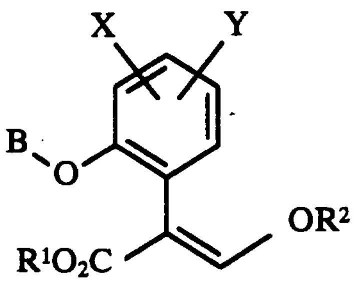

I

in der die Substituenten die folgende Bedeutung besitzen:
B bedeutet
Alkyl, das mit 1 - 4 gleichen oder verschiedenen Substituenten $R^3$ substituiert ist, Alkenyl, das mit 1 - 4 gleichen oder verschiedenen Substituenten $R^4$ substituiert ist, Alkinyl, das mit 1 - 4 gleichen oder verschiedenen Substituenten $R^5$ substituiert ist, Cycloalkyl, das mit 1 -4 gleichen oder verschiedenen Substituenten $R^6$ substituiert ist, Cycloalkenyl, das mit 1 - 4 gleichen oder verschiedenen Substituenten $R^7$ substituiert ist, Cycloalkinyl, das mit 1 - 4 gleichen oder verschiedenen Substituenten $R^8$ substituiert ist oder Heterocyclyl, das mit 1 - 4 gleichen oder verschiedenen Substituenten $R^9$ substituiert ist,
X und Y bedeuten unabhängig voneinander
Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,
Stehen die Substituenten X und Y an benachbarten Kohlenstoffatomen, dann können sie zu einem ggf. substituierten aromatischen oder heteroaromatischen, alicyclischen oder heterocyclischen, partiell oder vollständig hydrierten Ring kondensiert sein,
$R^1$ und $R^2$ können ggf. substituiert sein, und bedeuten unabhängig voneinander Alkyl, Alkenyl, Alkinyl, Cycloalkyl oder Cycloalkenyl, $R^3$ und $R^4$ können ggf. substituiert sein mit 1 - 4 gleichen oder verschiedenen Substituenten $R^{10}$, und $R^3$ und $R^4$ bedeutenNitro, Haloalkoxy, Alkoxy, Alkinyl, Cycloalkyl, Hetaryl, Heterocyclyl, Cycloalkenyl, Cycloalkinyl, Alkenyloxy, Alkinyloxy, Cycloalkyloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Hetarylalkyloxy, Heterocyclylalkyloxy, Alkinylalkenyl, Cycloalkylalkenyl, Cycloalkenylalkenyl, Cycloalkinylalkenyl, Hetarylalkenyl, Heterocyclylalkenyl, Alkoximinoalkyl, Alkenyloximinoalkyl, Alkinyloximinoalkyl, Cycloalkyloximinoalkyl, Cycloalkenyloximinoalkyl, Cycloalkinyloximinoalkyl, Aryloximinoalkyl, Hetaryloximinoalkyl, Heterocyclyloximinoalkyl, Alkoximinoalkenyl, Alkenyloximinoalkenyl, Alkinyloximinoalkenyl, Cycloalkyloximinoalkenyl, Cycloalkenyloximinoalkenyl, Cycloalkinyloximinoalkenyl, Aryloximinoalkenyl, Hetaryloximinoalkenyl oder Heterocyclyloximinoalkenyl,

$R^5$, $R^6$, $R^7$ und $R^8$ können ggf. substituiert sein mit 1 -4 gleichen oder verschiedenen Substituenten $R^{10}$, und $R^5$, $R^6$, $R^7$ und $R^8$ bedeuten

Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino oder Heterocyclylcarbonylamino,

$R^9$ kann ggf. substituiert sein, mit 1 - 4 gleichen oder verschiedenen Substituenten $R^{10}$, und $R^9$ bedeutet

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,

$R^{10}$ kann ggf. substituiert sein, mit 1 - 4 gleichen oder verschiedenen Substituenten $R^{11}$, und $R^{10}$ bedeutet

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl, Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy oder Heterocyclylalkyloxy,

$R^{11}$ kann ggf. substituiert sein und bedeutet

Wasserstoff, Halogen, Cyano, Nitro, Haloalkyl, Alkyl, Haloalkoxy, Alkenyl, Alkinyl, Cycloalkyl, Aryl, Hetaryl, Heterocyclyl, Cycloalkenyl, Cycloalkinyl, Alkoxy, Alkenyloxy, Alkinyloxy, Cycloalkyloxy, Aryloxy, Hetaryloxy, Heterocyclyloxy, Cycloalkenyloxy, Cycloalkinyloxy, Alkoximino, Alkenyloximino, Alkinyloximino, Cycloalkyloximino, Cycloalkenyloximino, Cycloalkinyloximino, Aryloximino, Hetaryloximino, Heterocyclyloximino, Alkoxycarbonyl, Alkenyloxycarbonyl, Alkinyloxycarbonyl, Cycloalkyloxycarbonyl, Aryloxycarbonyl, Hetaryloxycarbonyl, Heterocyclyloxycarbonyl, Cycloalkenyloxycarbonyl, Cycloalkinyloxycarbonyl, Alkylaminocarbonyl, Dialkylaminocarbonyl, Alkenylaminocarbonyl, Dialkenylaminocarbonyl, Alkinylaminocarbonyl, Dialkinylaminocarbonyl, Cycloalkylaminocarbonyl, Cycloalkenylaminocarbonyl, Cycloalkinylaminocarbonyl, Arylaminocarbonyl, Hetarylaminocarbonyl, Heterocyclylaminocarbonyl, Alkylthio, Alkenylthio, Alkinylthio, Cycloalkylthio, Arylthio, Hetarylthio, Heterocyclylthio, Cycloalkenylthio, Cycloalkinylthio, Alkylamino, Alkenylamino, Alkinylamino, Cycloalkylamino, Arylamino, Hetarylamino, Heterocyclylamino, Cycloalkenylamino, Cycloalkinylamino, Alkylcarbonyl, Alkenylcarbonyl, Alkinylcarbonyl, Cycloalkylcarbonyl, Arylcarbonyl, Hetarylcarbonyl, Heterocyclylcarbonyl, Cycloalkenylcarbonyl, Cycloalkinylcarbonyl, Alkylsulfoxyl, Alkenylsulfoxyl, Alkinylsulfoxyl, Cycloalkylsulfoxyl, Arylsulfoxyl, Hetarylsulfoxyl, Heterocyclylsulfoxyl, Cycloalkinylsulfoxyl, Cycloalkenylsulfoxyl, Alkylsulfonyl, Alkenylsulfonyl, Alkinylsulfonyl, Cycloalkylsulfonyl, Arylsulfonyl, Hetarylsulfonyl, Heterocyclylsulfonyl, Cycloalkenylsulfonyl, Cycloalkinylsulfonyl, Alkylsulfinyl, Alkenylsulfinyl, Alkinylsulfinyl, Cycloalkylsulfinyl, Arylsulfinyl,

Hetarylsulfinyl, Heterocyclylsulfinyl, Cycloalkenylsulfinyl, Cycloalkinylsulfinyl, Alkylcarbonyloxy, Alkenylcarbonyloxy, Alkinylcarbonyloxy, Cycloalkylcarbonyloxy, Cycloalkenylcarbonyloxy, Cycloalkinylcarbonyloxy, Arylcarbonyloxy, Hetarylcarbonyloxy, Heterocyclylcarbonyloxy, Alkylcarbonylamino, Alkenylcarbonylamino, Alkinylcarbonylamino, Cycloalkylcarbonylamino, Cycloalkenylcarbonylamino, Cycloalkinylcarbonylamino, Arylcarbonylamino, Hetarylcarbonylamino, Heterocyclylcarbonylamino, Cycloalkylalkyloxy, Cycloalkenylalkyloxy, Cycloalkinylalkyloxy, Arylalkyloxy, Hetarylalkyloxy, oder Heterocyclylalkyloxy oder eines Säureadditionsproduktes oder Basenadditionsprodukte dieser Verbindungen.